# EUROPÄISCHE PATENTSCHRIFT

(11) **EP 1 056 717 B1**
(45) Veröffentlichungstag und Bekanntmachung des Hinweises auf die Patenterteilung: **20.07.2005**
(21) Anmeldenummer: 99904881.2
(22) Anmeldetag: 17.02.1999
(51) Int. Cl.: C07D 207/38, A01N 43/90, A01N 43/48, A01N 43/08, A01N 43/10, A01N 43/36, A01N 43/38, A01N 43/86, A01N 43/16, A01N 45/02, A01N 35/06, C07D 491/10, C07D 209/96, C07D 401/12, C07D 307/94

(54) **ARYLPHENYLSUBSTITUIERTE CYCLISCHE KETOENOLE**
ARYLPHENYL-SUBSTITUTED CYCLIC KETO-ENOLS
CETO-ENOLS CYCLIQUES A SUBSTITUTION ARYLPHENYLE

(30) Priorität: 27.02.1998 DE 19808261
(43) Veröffentlichungstag der Anmeldung: 06.12.2000
(73) Patentinhaber: Bayer CropScience AG, 40789 Monheim (DE)
(72) Erfinder: LIEB, Folker, D-51375 Leverkusen (DE); FISCHER, Reiner, D-40789 Monheim (DE); GRAFF, Alan, D-51061 Köln (DE); SCHNEIDER, Udo, D-51373 Leverkusen (DE); BRETSCHNEIDER, Thomas, D-53797 Lohmar (DE); ERDELEN, Christoph, D-42799 Leichlingen (DE); ANDERSCH, Wolfram, D-51469 Bergisch Gladbach (DE); DREWES, Mark-Wilhelm, D-40764 Langenfeld (DE); DOLLINGER, Markus, Overland Park, KS 66213 (US); WETCHOLOWSKY, Ingo, CEP-13280-000 Vinhedo, SP (BR); MYERS, Randy, Allen, D-40489 Düsseldorf (DE)
(86) Internationale Anmeldenummer: PCT/EP1999/001029
(87) Internationale Veröffentlichungsnummer: WO 1999/043649

(56) Entgegenhaltungen:
- EP-A- 0 442 077
- EP-A- 0 521 334
- WO-A-96/20196
- WO-A-97/14667
- DE-A- 4 431 730
- DE-A- 19 543 864
- DE-A- 19 649 665
- US-A- 4 613 617
- US-A- 5 719 310

## Beschreibung

Die vorliegende Erfindung betrifft neue arylphenylsubstituierte cyclische Ketoenole, mehrere Verfahren zu ihrer Herstellung und ihre Verwendung als Schädlingsbekämpfungsmittel und Herbizide.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-415 211) sowie substituierte monocyclische 3-Aryl-pyrrolidin-2,4-dion-Derivate (EP-A-377 893 und EP-442 077).

Weiterhin bekannt sind polycyclische 3-Arylpyrrolidin-2,4-dion-Derivate (EP-442 073) sowie 1H-Arylpyrrolidin-dion-Derivate (EP-456 063, EP-521 334, EP-596 298, EP-613 884, EP-613 885, WO 94/01 997, WO 95/26 954, WO 95/20 572, EP-A-0 668 267, WO 96/25 395, WO 96/35 664, WO 97/01 535, WO 97/02 243 und WO 97/36 868, WO 98/05 638).

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528156, EP-A-0 647 637, WO 95/26 345, WO 96/20 196, WO 96/25 395, WO 96/35 664, WO 97/01 535, WO 97/02 243 und WO 97/36 868, WO 98/05 638 bekannt. Auch 3-Aryl-Δ³-dihydrothiphen-on-Derivate sind bekannt (WO 95/26 345, 96/25 395, WO 97/01 535, WO 97/02 243, WO 97/36 868, WO 98/05 638).

Aus der Literatur sind ferner bestimmte 3H-Pyrazol-3-on-Derivate, wie beispielsweise 1,2-Diethyl-1,2-dihydro-5-hydroxy-4-phenyl-3H-pyrazol-3-on oder {[5-Oxo-1,2-diphenyl-4-(p-sulfophenyl)-3-pyrazolin-3-yl]-oxy}-dinatriumsalz oder p-(3-Hydroxy-5-oxo-1,2-diphenyl-3-pyrazolin-4-yl)-benzolsulfonsäure bekannt (vgl. J. Heterocycl. Chem., 25(5), 1301-1305, 1988 oder J. Heterocycl. Chem., 25(5), 1307-1310, 1988 oder Zh. Obshch. Khim., 34(7), 2397-2402, 1964). Eine biologische Wirkung dieser Verbindungen wird aber nicht beschrieben.

Weiterhin ist bekannt, daß das Trinatriumsalz der 4,4',4"-(5-Hydroxy-3-oxo-1H-pyrazol-1,2,4(3H)-triyl)-tris-benzolsulfonsäure pharmakologische Eigenschaften besitzt (vgl. Farmakol. Toksikol. (Moscow), 38(2), 180-186, 1976). Seine Verwendung im Pflanzenschutz ist aber nicht bekannt.

Außerdem sind in EP-508 126 und in WO 92/16 510, WO 96/21 652 4-Arylpyrazolidin-3,5-dion-Derivate mit herbiziden, akariziden und insektiziden Eigenschaften beschrieben. Zudem wurden 4-Arylpyrazolidine bekannt, von denen fungizide Eigenschaften beschrieben wurden (WO 96/36 229, WO 96/36 615, WO 96/36 616, WO 96/36 633).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941 und WO 97/36 868, WO 98/05 638 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868 beschrieben.

Es ist bekannt, daß bestimmte substituierte 2-Arylcyclopentandione herbizide und akarizide Eigenschaften besitzen (vgl. z.B. US-4 283 348; 4 338 122; 4 436 666; 4 526 723; 4 551 547; 4 632 698; WO 96/01 798; WO 96/03 366 sowie WO 97/14 667). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-2 361 084 bekannt, mit Angabe von herbiziden und akariziden Wirkungen.

Es ist bekannt, daß bestimmte substituierte 2-Arylcyclohexandione herbizide und akarizide Eigenschaften besitzen (US-4 175 135, 4 209 432, 4 256 657, 4 256 658, 4 256 659, 4 257 858, 4 283 348, 4 303 669, 4351666, 4 409 153, 4 436 666, 4 526 723, 4 613 617, 4 659 372, DE-2 813 341, sowie Wheeler, T.N., J. Org. Chem. 44, 4906 (1979)).

Die Wirksamkeit und Wirkungsbreite dieser Verbindungen ist jedoch insbesondere bei niedrigen Aufwandmengen und Konzentrationen nicht immer voll znfriedenstellend. Weiterhin ist die Pflanzenverträglichkeit dieser Verbindungen nicht immer ausreichend.

Es wurden nun neue Verbindungen der Formel (I) 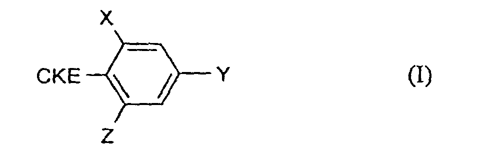 in welcher
- X: für Fluor, Chlor, Methyl, Ethyl, Propyl, iso-Propyl,
- Y: für einen der Reste 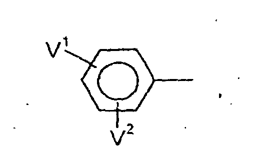 steht,
- V¹: für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder Phenyl steht,
- V²: für Wasserstoff, Fluor, Chlor, Methyl, Ethyl, n-Propyl, iso-Propyl, Methoxy, Ethoxy, Trifluormethyl oder Trifluormethoxy stehen,
- Z: für Wasserstoff, Fluor, Chlor, Methyl, Ethyl, n-Propyl, steht,
- CKE: für eine der Gruppen 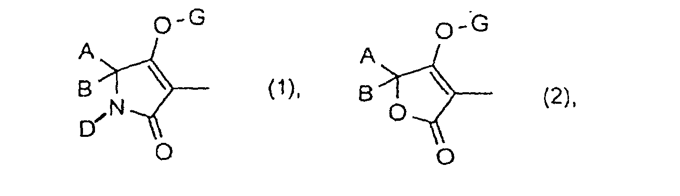 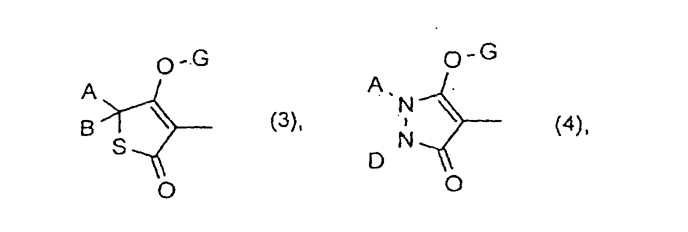 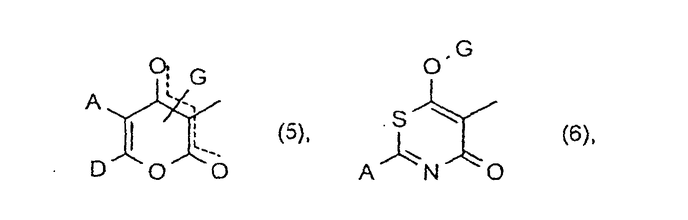 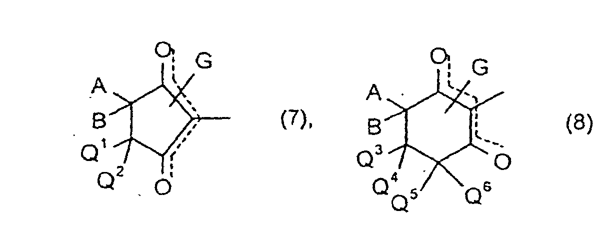 steht,
- A: für Wasserstoff, jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Formeln (I-5), (I-7) und (I-8)) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl steht,
- B: für C₁-C₄-Alkyl steht, oder
- A, B: und das Kohlenstoffatom an das sie gebunden sind für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, n-Propyl, Isopropyl, Butyl, iso-Butyl, sec.-Butyl, tert.-Butyl, Trifluormethyl, Methoxy, Ethoxy, n-Propoxy, iso-Propoxy, n-Butoxy, iso-Butoxy, sek.-Butoxy, tert.-Butoxy, Fluor oder Chlor substituiert ist oder
- A, B: und das Kohlenstoffatom, an das sie gebunden sind, für C₅-C₆-Cycloalkyl stehen, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
- D: für Wasserstoff, für jeweils gegebenenfalls durch Fluor oder Chlor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl, Furanyl, Pyridyl, Thienyl oder Benzyl steht,
oder
- A und D: gemeinsam für gegebenenfalls substituiertes C₃-C₄-Alkandiyl stehen, worin gegebenenfalls ein Kohlenstoffatom durch Schwefel ersetzt ist und welches gegebenenfalls durch Hydroxy, Methyl, Ethyl, Methoxy oder Ethoxy substituiert ist oder
- A und D: (im Fall der Verbindungen der Formel (I-1)) gemeinsam mit den Atomen, an die sie gebunden sind, für eine der folgenden Gruppen AD stehen: 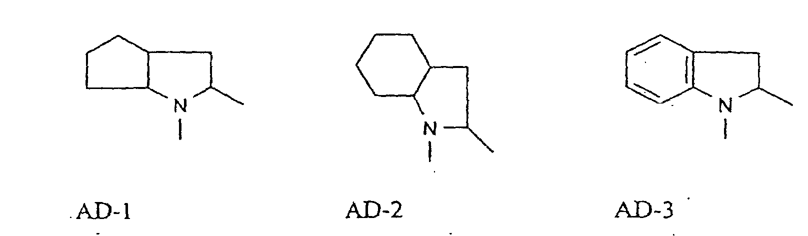 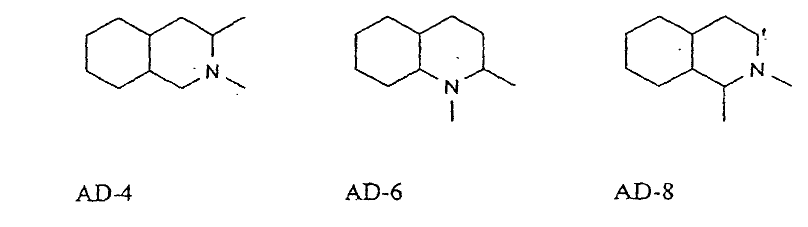 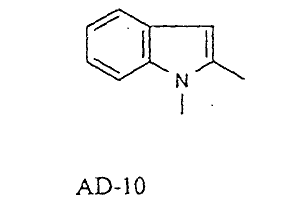
- A und Q¹: gemeinsam für gegebenenfalls einfach oder zweifach durch Fluor, Hydroxy, Methyl oder Methoxy substituiertes C₃-C₄-Alkandiyl oder Butendiyl stehen, oder
- Q¹: für Wasserstoff steht,
- Q²: für Wasserstoff steht,
- Q⁴, Q⁵ und Q⁶: unabhängig voneinander für Wasserstoff, Methyl oder Ethyl stehen,
- Q³: für Wasserstoff, Methyl, Ethyl oder C₃-C₆-Cycloalkyl stehen, worin gegebenenfalls eine Methylengruppe durch Sauerstoff oder Schwefel ersetzt ist oder
- Q³ und Q⁴: gemeinsam mit dem Kohlenstoff, an den sie gebunden sind, für gegebenenfalls durch Methyl oder Methoxy substituiertes gesättigtes C₅-C₆-Cycloalkyl stehen, in welchem gegebenenfalls ein Ringglied durch Sauerstoff oder Schwefel ersetzt ist,
- G: für Wasserstoff (a) oder für eine der Gruppen 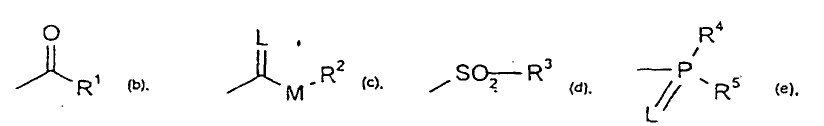 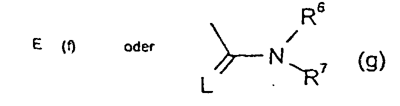 steht, in welchen
E für ein Metallion oder ein Ammoniumion steht,
L für Sauerstoff oder Schwefel steht und
M für Sauerstoff oder Schwefel steht,
- R¹: für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl oder Ethyl substituiertes Phenoxy-C₁-C₂-alkyl oder
für jeweils gegebenenfalls durch Fluor, Chlor, Amino, Methyl oder Ethyl substituiertes Pyridyloxy-C₁-C₂-alkyl, Pyrimidyloxy-C₁-C₂-alkyl oder Thiazolyloxy-C₁-C₂-alkyl steht,
- R²: für jeweils gegebenenfalls durch Fluor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
- R³: für gegebenenfalls durch Fluor substituiertes Methyl, Ethyl, n-Propyl, Isopropyl oder jeweils gegebenenfalls durch Fluor, Chlor, Brom, Methyl, tert.-Butyl, Methoxy, Trifluormethyl, Trifluormethoxy, Cyano oder Nitro substituiertes Phenyl oder Benzyl steht,
- R⁴ und R⁵: unabhängig voneinander für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio stehen, und
- R⁶ und R⁷: unabhängig voneinander für Wasserstoff, für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, Trifluorme-thyl, Methyl oder Methoxy substituiertes Phenyl, für gegebenenfalls durch Fluor, Chlor, Brom, Methyl, Trifluormethyl oder Methoxy substituiertes Benzyl, oder zusammen für einen C₅-C₆-Alkylenrest stehen, in welchem gegebenenfalls eine Methylengruppe durch Sauerstoff oder Schwefel ersetzt ist.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sein können.

Unter Einbeziehung der Bedeutungen (1) bis (8) der Gruppe CKE ergeben sich folgende hauptsächliche Strukturen (I-1) bis (I-8): 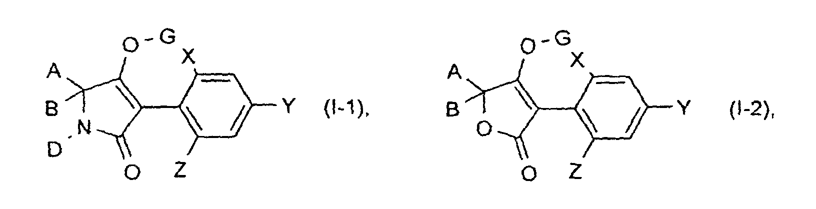 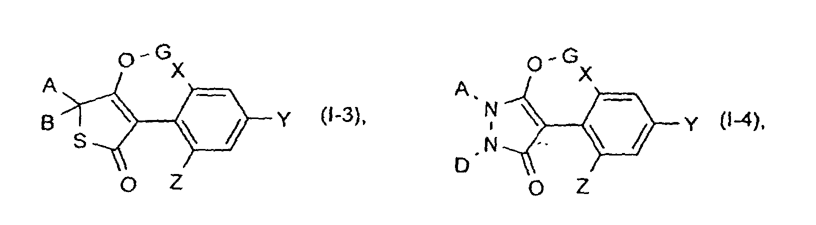 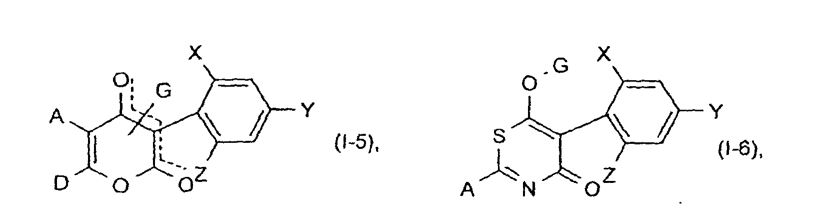 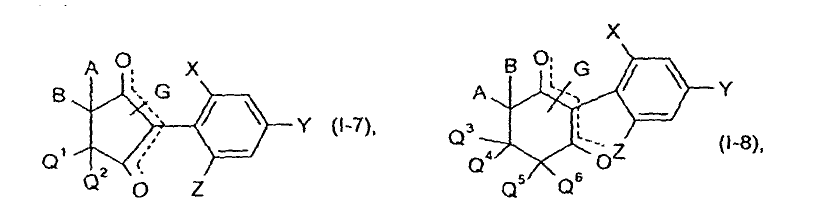 worin
A, B, D, G, Q¹, Q², Q³, Q⁴, Q⁵, Q⁶, X, Y und Z die oben angegebenen Bedeutungen haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn CKE für die Gruppe (1) steht, 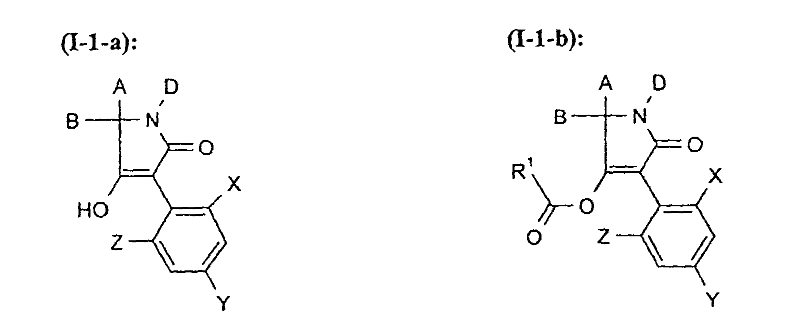 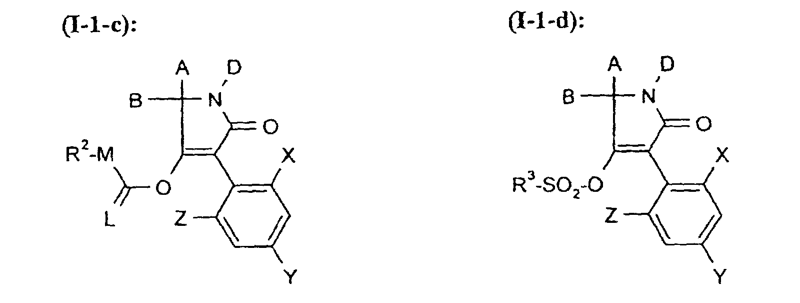 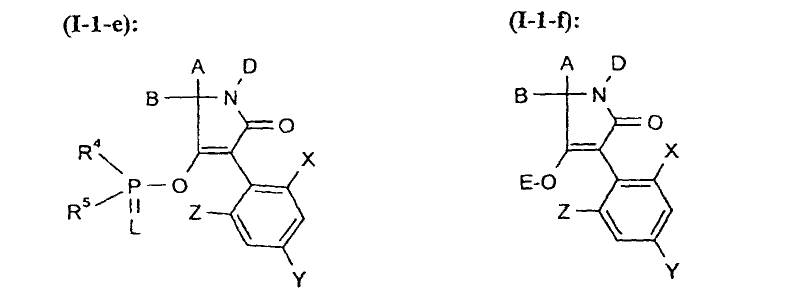 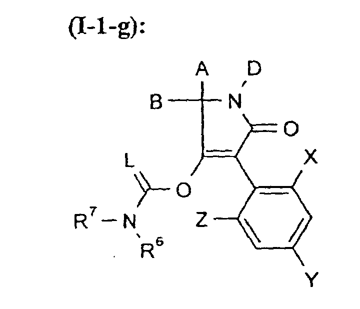 worin
A, B, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn CKE für die Gruppe (2) steht, 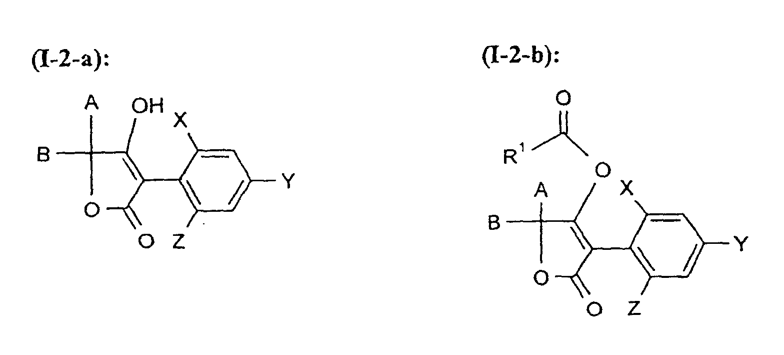 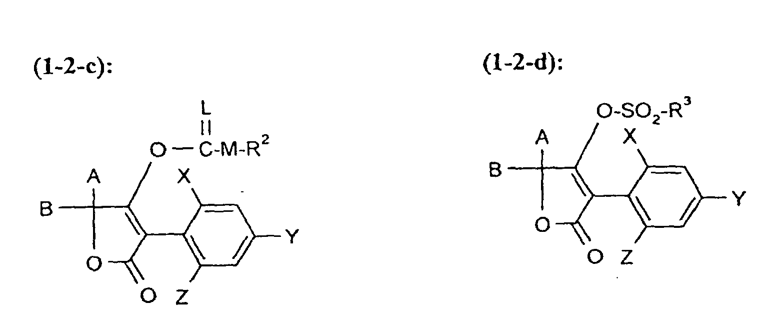 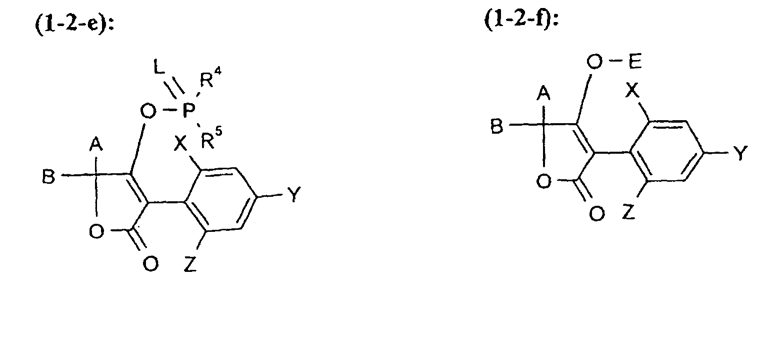 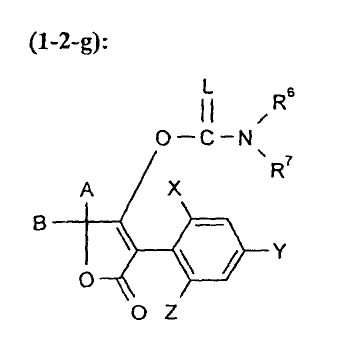 worin
A, B, E, L, M, X, Y, Z, R¹, R², R³, R⁴, R⁵, R⁶ und R⁷ die oben angegebenen Bedeutungen haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn CKE für die Gruppe (3) steht, 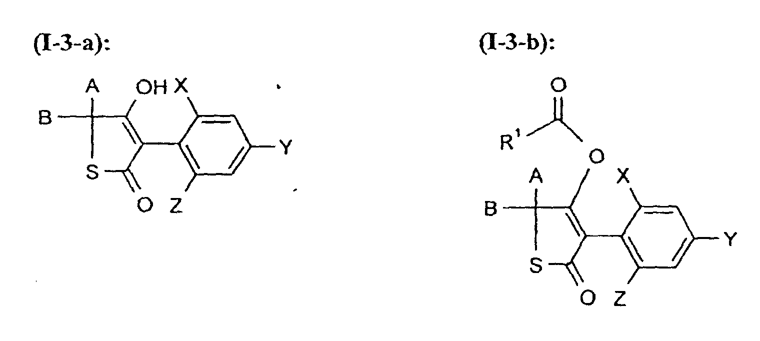 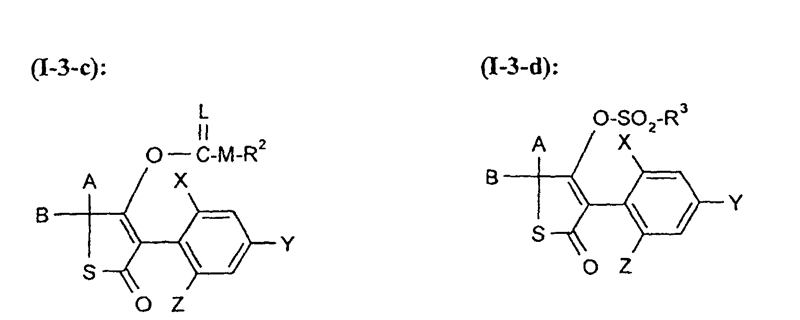 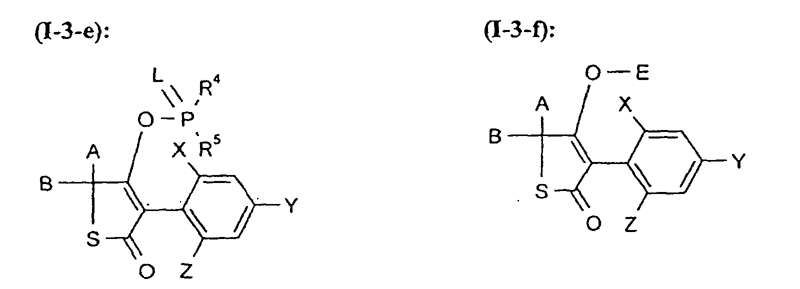 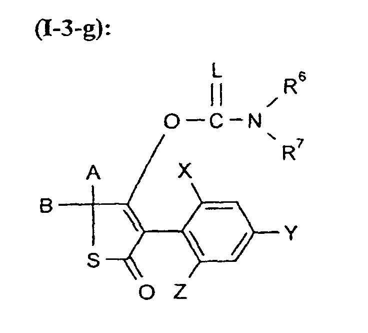 worin
A, B, E, L, M, X, Y, Z, R¹, R², R³, R⁴, R⁵, R⁶ und R⁷ die oben angegebenen Bedeutung besitzen.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn CKE für die Gruppe (4) steht, 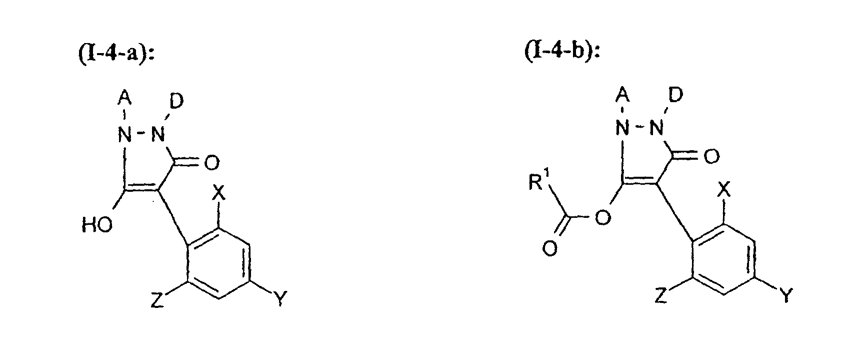 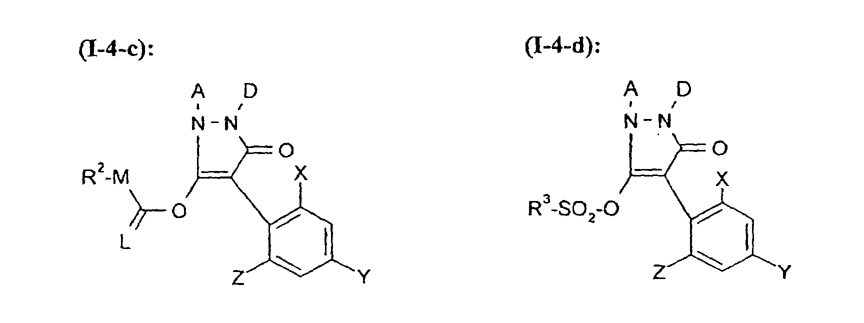 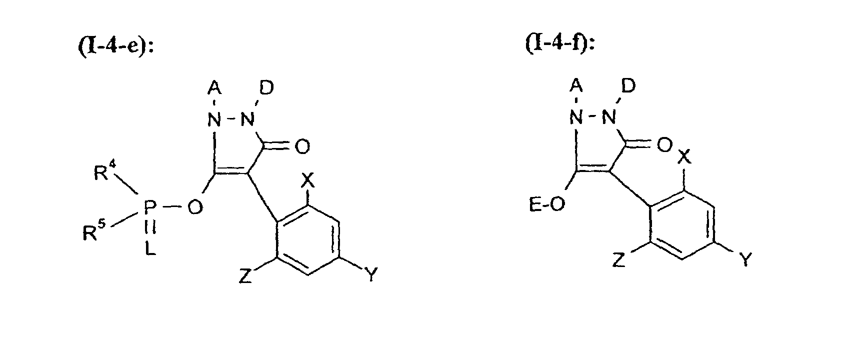 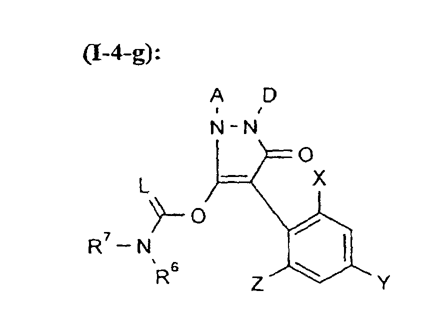 worin
A, D, E, L, M, X, Y, Z, R¹, R², R³, R⁴, R⁵, R⁶ und R⁷ die oben angegebenen Bedeutungen besitzen.

Die Verbindungen der Formel (I-5) können in Abhängigkeit von der Stellung des Substituenten G in den zwei isomeren Formen der Formeln (I-5-A) und (I-5-B) vorliegen, 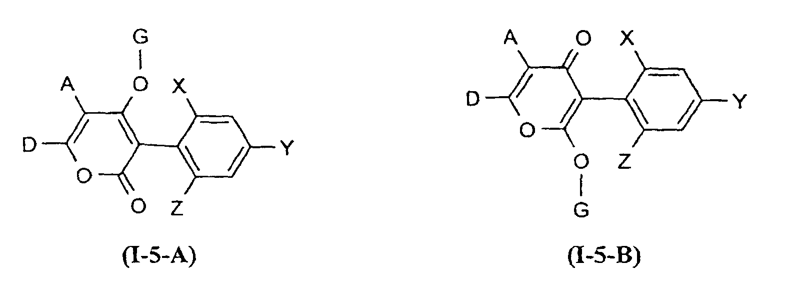 was durch die gestrichelte Linie in der Formel (I-5) zum Ausdruck gebracht werden soll.

Die Verbindungen der Formeln (I-5-A) und (I-5-B) können sowohl als Gemische als auch in Form ihrer reinen Isomeren vorliegen. Gemische der Verbindungen der Formeln (I-5-A) und (I-5-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn CKE für die Gruppe (5) steht, 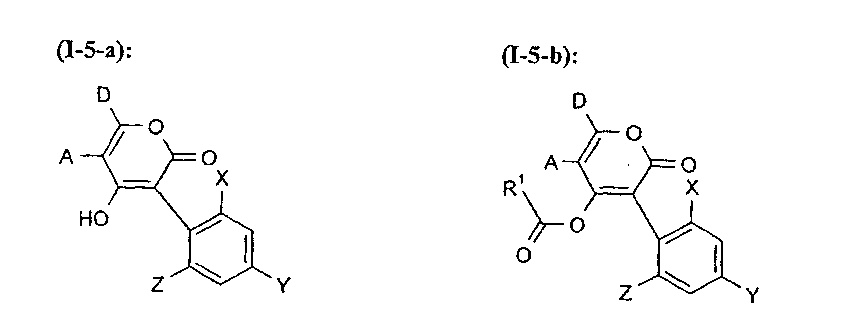 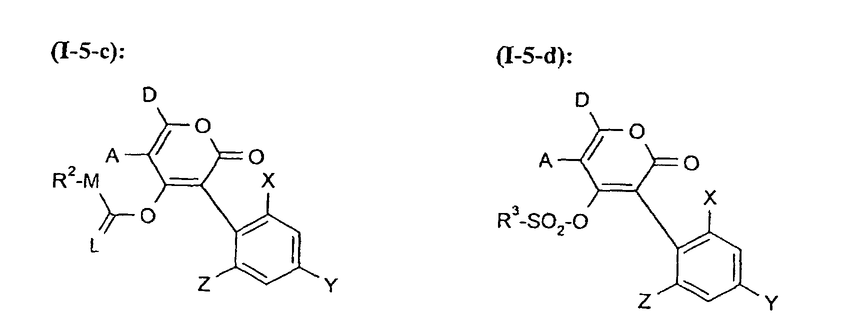 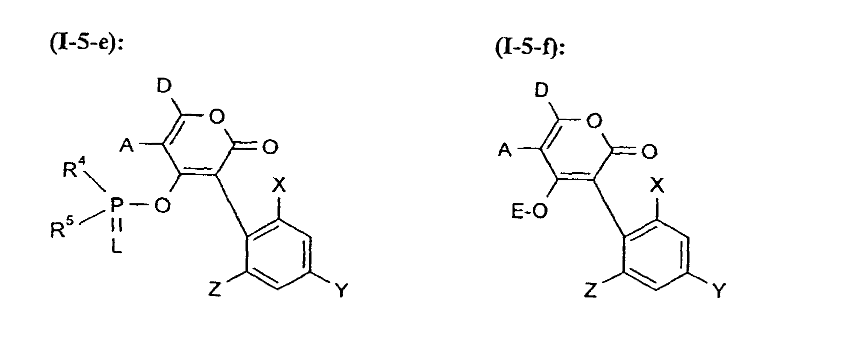 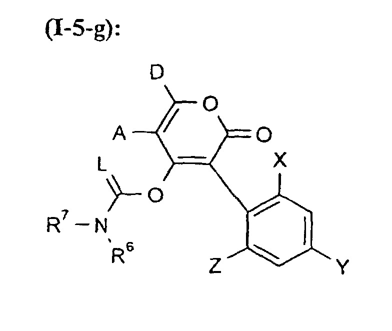 worin
A, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-6-a) bis (I-6-g), wenn CKE für die Gruppe (6) steht, 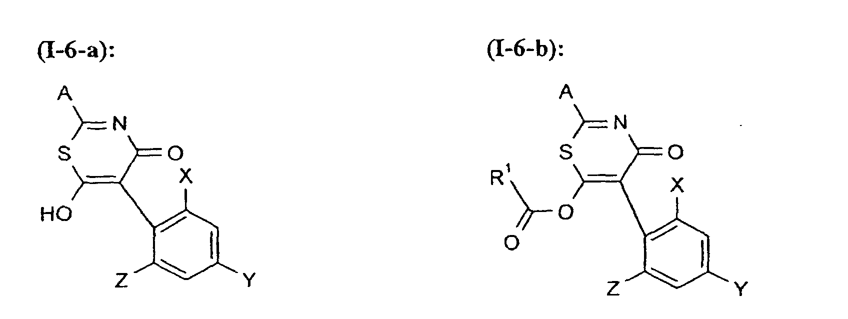 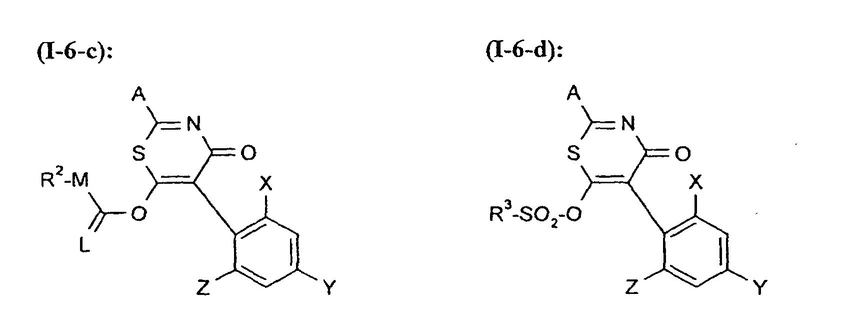 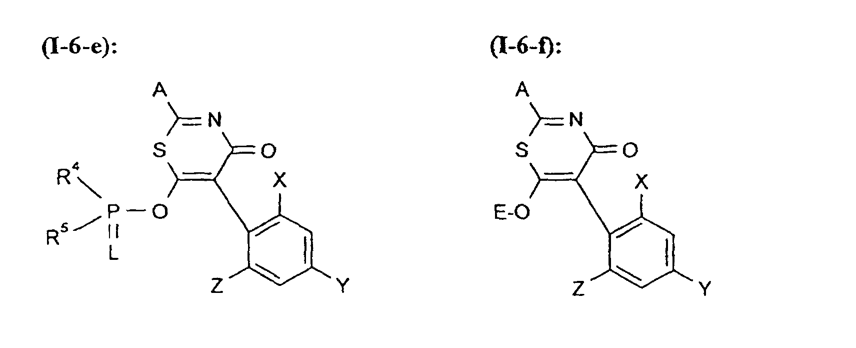 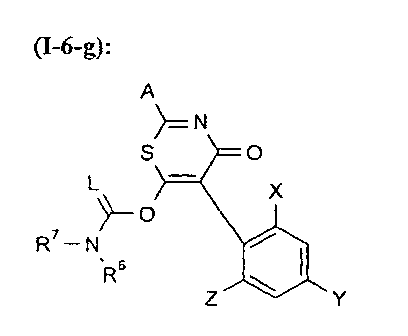 worin
A, E, L, M, X, Y, Z, R¹, R², R³, R⁴, R⁵, R⁶ und R⁷ die oben angegebenen Bedeutungen besitzen.

Die Verbindungen der Formel (1-7) können in Abhängigkeit von der Stellung des Substituenten G in den zwei isomeren Formen der Formeln (I-7-A) und (I-7-B) vorliegen, 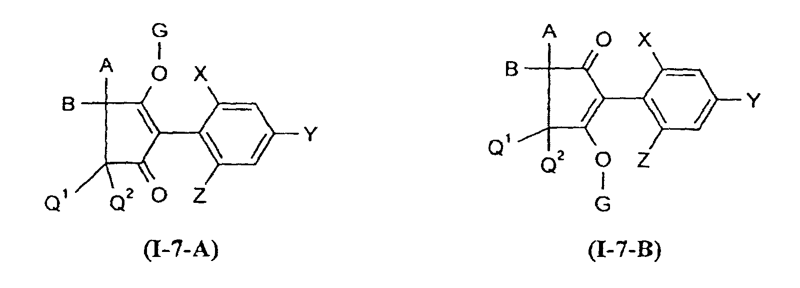 was durch die gestrichelte Linie in der Formel (I) zum Ausdruck gebracht werden soll.

Die Verbindungen der Formeln (I-7-A) und (I-7-B) können sowohl als Gemische als auch in Form ihrer reinen Isomeren vorliegen. Gemische der Verbindungen der Formeln (I-7-A) und (I-7-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (I-7-a) bis (I-7-g): 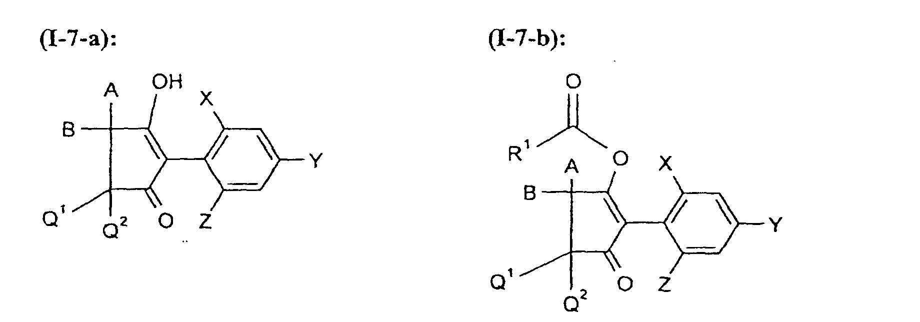 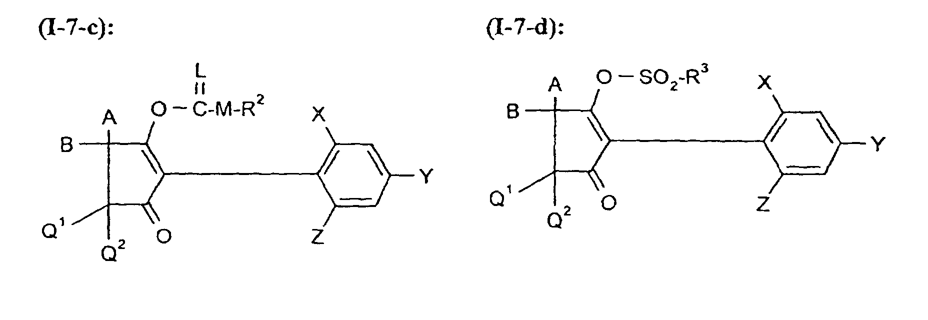 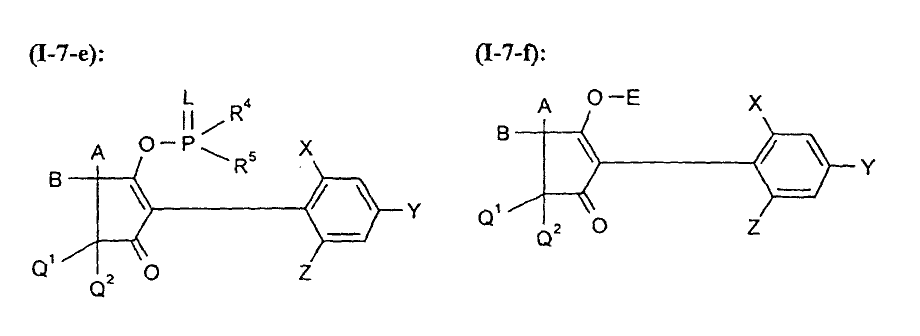 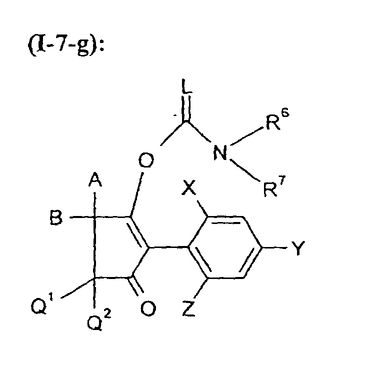 worin
A, B, Q¹, Q², E, L, M, X, Y, Z, R¹, R², R³, R⁴, R⁵, R⁶ und R⁷ die oben angegebenen Bedeutungen besitzen.

Die Verbindungen der Formel (1-8) können in Abhängigkeit von der Stellung des Substituenten G in den zwei isomeren Formen der Formeln (I-8-A) bzw. (I-8-B) vorliegen, was durch die gestrichelte Linie in der Formel (1-8) zum Ausdruck gebracht werden soll:

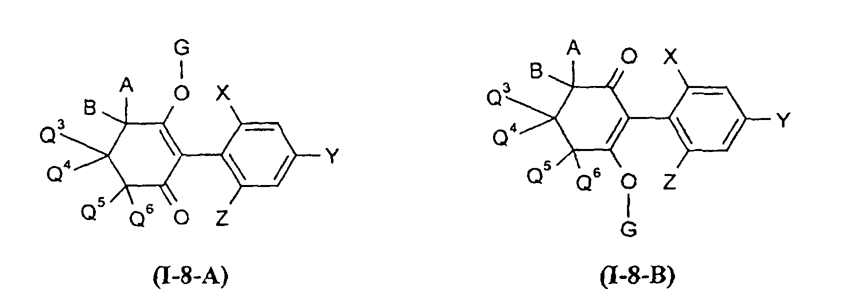

Die Verbindungen der Formeln (I-8-A) bzw. (I-8-B) können sowohl als Gemische als auch in Form ihrer reinen Isomeren vorliegen. Gemische der Verbindungen der Formeln (I-8-A) und (I-8-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, daß die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (I-8-a) bis (I-8-g): 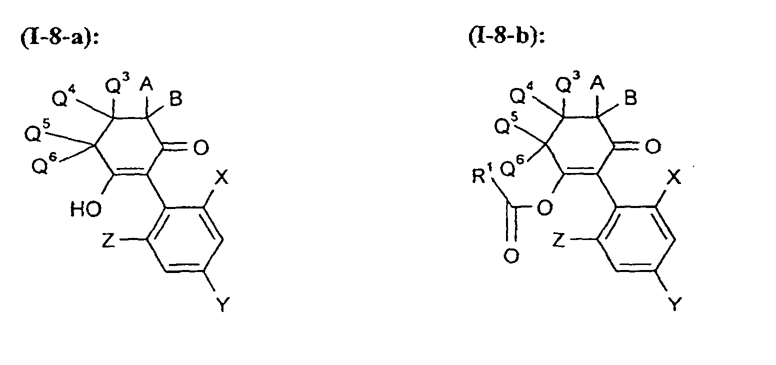 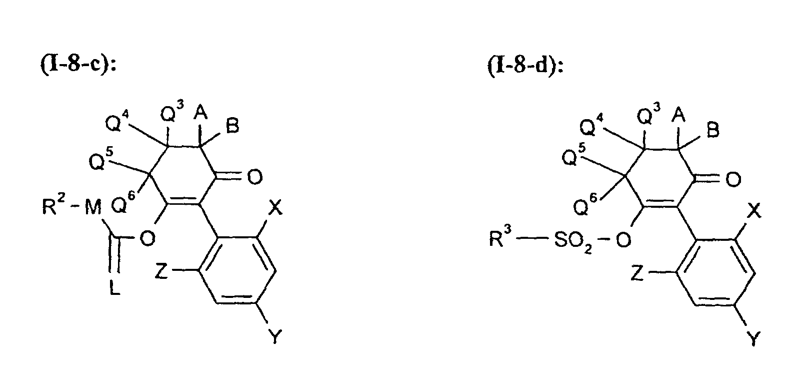 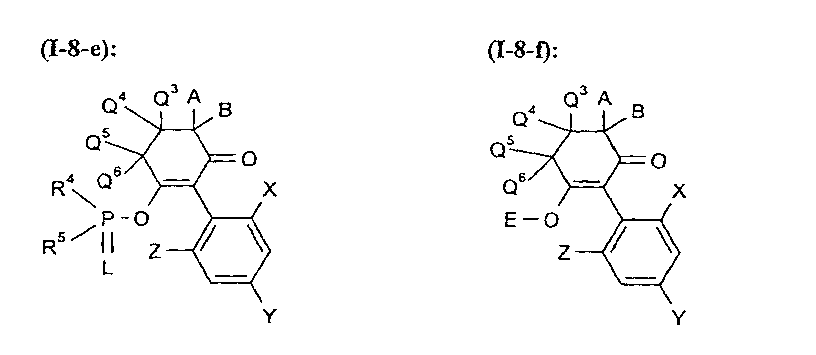 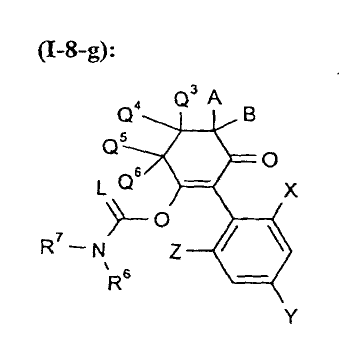 worin
A, B, E, L, M, Q³, Q⁴, Q⁵, Q⁶, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 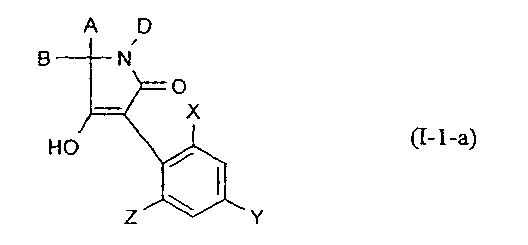 in welcher
   - A, B, D, X, Y und Z: die oben angegebenen Bedeutungen haben,
   wenn man
   N-Acylaminosäureester der Formel (II) 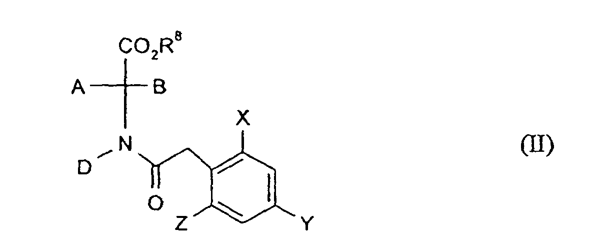 in welcher
   - A, B, D, X, Y und Z: die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man substituierte 3-Phenyl-4-hydroxy-Δ³-dihydrofuranon-Derivate der Formel (I-2-a) 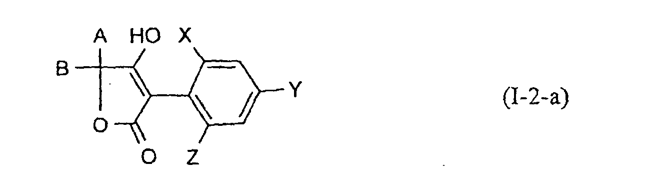 in welcher
   - A, B, X, Y und Z: die oben angegebenen Bedeutungen haben,
   erhält, wenn man
   Carbonsäureester der Formel (III) 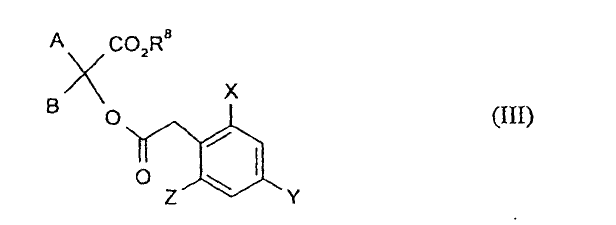 in welcher
   - A, B, X, Y, Z und R⁸: die oben angegebenen Bedeutungen haben,
   in Gegenwart eines Verdünnungsmittels und in Gegenwart einer Base intramolekular kondensiert.
(C) Weiterhin wurde gefunden, daß man substituierte 3-Phenyl-4-hydroxy-Δ³-dihydrothiophenon-Derivate der Formel (I-3-a) 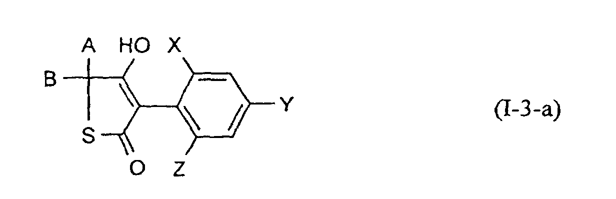 in welcher
   - A, B, X, Y und Z: die oben angegebenen Bedeutungen haben,
   erhält, wenn man
   β-Ketocarbonsäureester der Formel (IV) 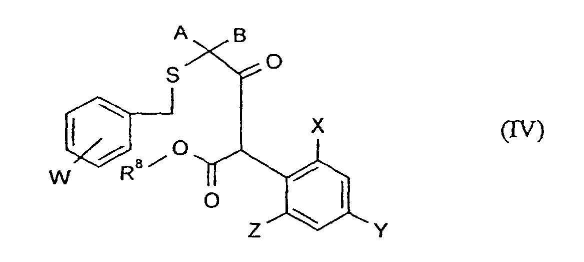 in welcher
   - A, B, X, Y, Z und R⁸: die oben angegebenen Bedeutungen haben und
   - W: für Wasserstoff, Halogen (bevorzugt Fluor, Chlor, Brom), Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin erhält man substituierte 3-Hydroxy-4-phenyl-5-oxo-pyrazoline der Formel (I-4-a) 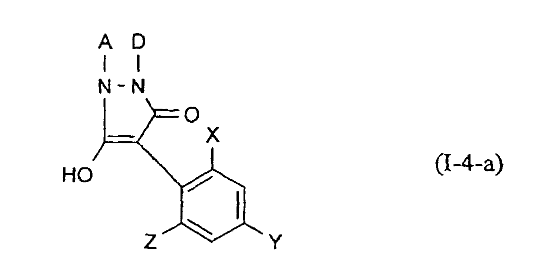 in welcher
   - A, D, X, Y und Z: die oben angegebenen Bedeutungen haben,
   wenn man
   (α) Halogencarbonylketene der Formel (V) 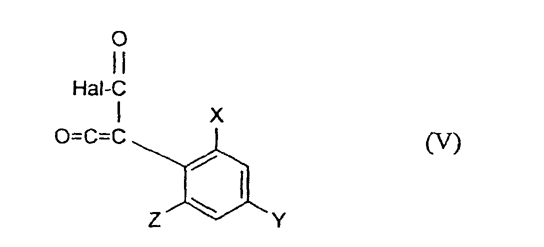 in welcher
      - X, Y und Z: die oben angegebenen Bedeutungen haben
      und
      - Hal: für Halogen (insbesondere Chlor oder Brom) steht,
      oder
   (β) Malonsäurederivate der Formel (VI) 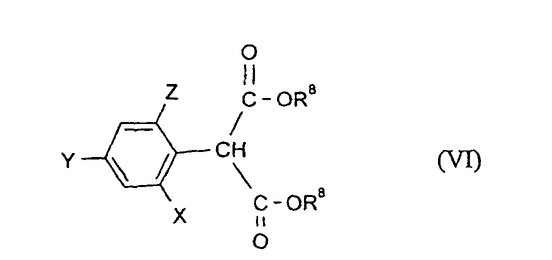 in welcher
      - R⁸, X, Y und Z: die oben angegebenen Bedeutungen haben,
      mit Hydrazinen der Formel (VII)

      A-NH-NH-D (VII)

      in welcher
      - A und D: die oben angegebenen Bedeutungen haben,
      gegebenenfalls in Gegenwart eines Verdünnungsmittels und gegebenenfalls in Gegenwart einer Base umsetzt.
(E) Weiterhin wurde gefunden, daß man die neuen substituierten 3-Phenylpyron-Derivate der Formel (I-5-a) 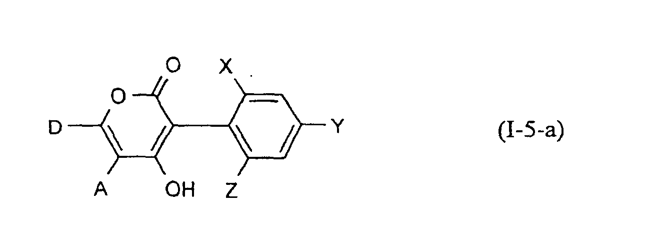 in welcher
   - A, D, X, Y und Z: die oben angegebenen Bedeutungen haben,
   erhält, wenn man
   Carbonylverbindungen der Formel (VIII) 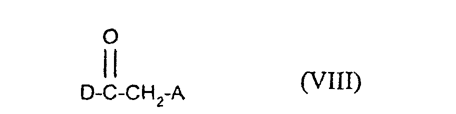 in welcher
   - A und D: die oben angegebenen Bedeutungen haben,
   oder deren Silyienolether der Formel (VIIIa) 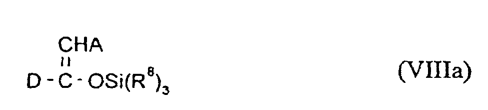 in welcher
   - A, D und R⁸: die oben angegebenen Bedeutungen haben,
   mit Ketensäurehalogeniden der Formel (V) 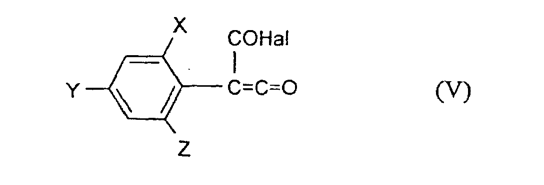 in welcher
   - X, Y und Z: die oben angegebenen Bedeutungen haben und
   - Hal: für Halogen (vorzugsweise für Chlor oder Brom) steht,
   gegebenenfalls in Gegenwart eines Verdünnungsmittels und gegebenenfalls in Gegenwart eines Säureakzeptors umsetzt.
   Weiterhin wurde gefunden,
(F) daß man die neuen substituierten Phenyl-1,3-thiazin-Derivate der Formel (I-6-a) 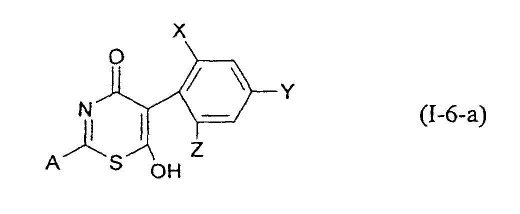 in welcher
   - A, X, Y und Z: die oben angegebenen Bedeutungen haben,
   erhält, wenn man Thioamide der Formel (IX) 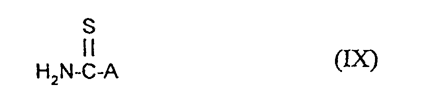 in welcher
   - A: die oben angegebene Bedeutung hat,
   mit Ketensäurehalogeniden der Formel (V) 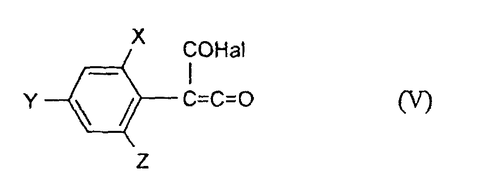 in welcher
   - Hal, X, Y und Z: die oben angegebenen Bedeutungen haben,
   gegebenenfalls in Gegenwart eines Verdünnungsmittels und gegebenenfalls in Gegenwart eines Säureakzeptors umsetzt.
   Weiterhin wurde gefunden,
(G) daß man Verbindungen der Formel (I-7-a) 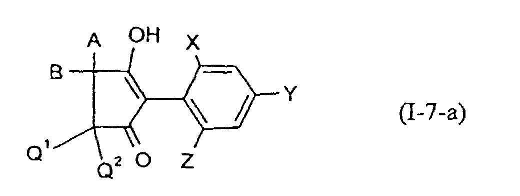 in welcher
   - A, B, Q¹, Q², X, Y und Z: die oben angegebenen Bedeutungen haben,
   erhält, wenn man
   Ketocarbonsäureester der Formel (X) 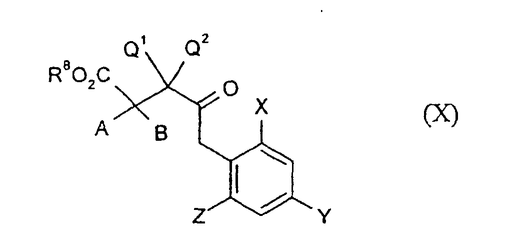 in welcher
   - A, B, Q¹, Q², X, Y und Z: die oben angegebenen Bedeutungen haben, und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden,
(H) daß man Verbindungen der Formel (I-8-a) 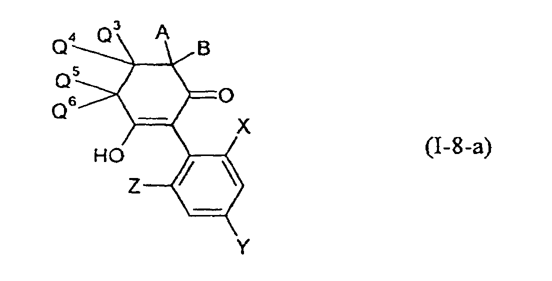 in welcher
   - A, B, Q³, Q⁴, Q⁵, Q⁶, X, Y und Z: die oben angegebenen Bedeutungen haben,
   erhält, wenn man
   6-Aryl-5-keto-hexansäureester der Formel (XI) 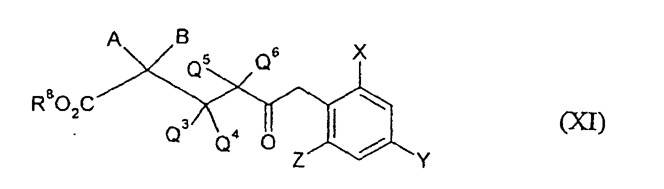 in welcher
   - A, B, Q³, Q⁴, Q⁵, Q⁶, X, Y und Z: die oben angegebenen Bedeutungen haben
   und
   - R⁸: für Alkyl (bevorzugt C₁-C₆-Alkyl) steht,
   in Gegenwart eines Verdünnungsmittels und in Gegenwart einer Base intramolekular kondensiert;
   oder
(I) daß man Verbindungen der oben gezeigten Formeln (I-1-a) bis (I-8-a), in welchen A, B, D, Q¹, Q², Q³, Q⁴, Q⁵, Q⁶, X, Y und Z die oben angegebenen Bedeutungen haben, erhält, wenn man Verbindungen der Formel (I-1'-a) bis (I-8'-a), 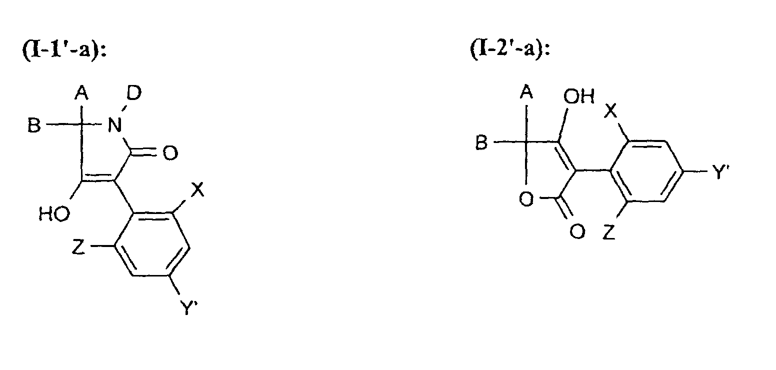 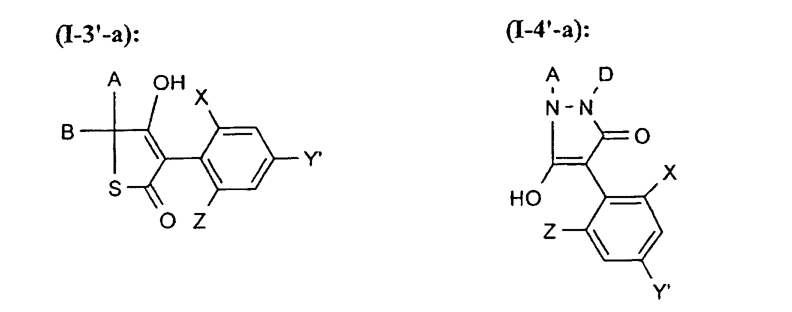 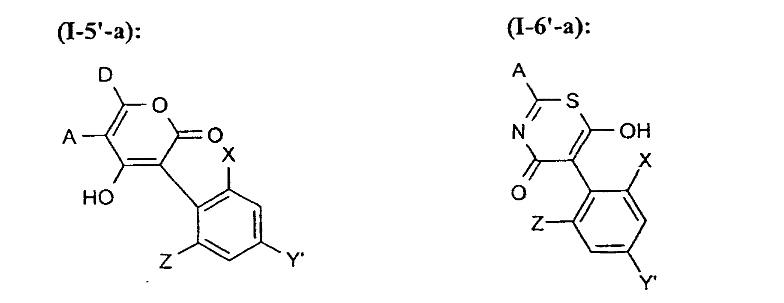 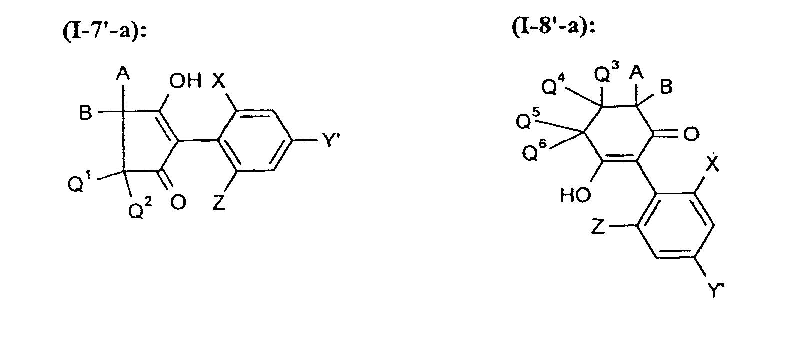 in welchen
   - A, B, D, Q¹, Q², Q³, Q⁴, Q⁵, Q⁶, X und Z: die oben angegebene Bedeutung haben und
   - Y': für Chlor, Brom oder Jod, bevorzugt für Brom steht,
   mit Boronsäuren der Formel (XII) 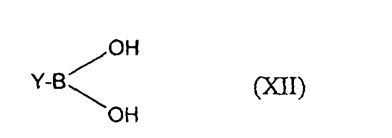 in welcher
   - Y: die oben angegebene Bedeutung hat,
   in Gegenwart eines Lösungsmittels, einer Base und eines Katalysators umsetzt, wobei als Katalysator insbesondere Palladiumkomplexe in Frage kommen.
   Außerdem wurde gefunden
(J) daß man die Verbindungen der oben gezeigten Formeln (I-1-b) bis (I-8-b), in welchen A, B, D, Q¹, Q², Q³, Q⁴, Q⁵, Q⁶, R¹, X, Y und Z die oben angebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, X, Y und Z die oben angegebenen Bedeutungen haben, jeweils
   (α) mit Säurehalogeniden der Formel (XIII) 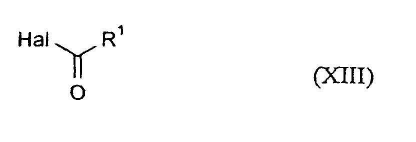 in welcher
      - R¹: die oben angegebene Bedeutung hat und
      - Hal: für Halogen (insbesondere Chlor oder Brom) steht
      oder
   (β) mit Carbonsäureanhydriden der Formel (XIV)

      R¹-CO-O-CO-R¹ (XIV)

      in welcher
      - R¹: die oben angegebene Bedeutung hat,
   gegebenenfalls in Gegenwart eines Verdünnungsmittels und gegebenenfalls in Gegenwart eines Säurebindemittels umsetzt;
(K) daß man die Verbindungen der oben gezeigten Formeln (I-1-c) bis (I-8-c), in welchen A, B, D, Q¹, Q², Q³, Q⁴, Q⁵, Q⁶, R², M, X, Y und Z die oben angegebenen Bedeutungen haben und L für Sauerstoff steht, erhält, wenn man Verbindungen der oben gezeigten Formeln (I-1-a) bis (I-8-a), in welchen A, B, D, Q¹, Q², Q³, Q⁴, Q⁵, Q⁶, X, Y und Z die oben angegebenen Bedeutungen haben, jeweils
   mit Chlorameisensäureestern oder Chlorameisensäurethioestern der Formel (XV)

   R²-M-CO-Cl (XV)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(L) daß man Verbindungen der oben gezeigten Formeln (I-1-c) bis (I-8-c), in welchen A, B, D, Q¹, Q², Q³, Q⁴, Q⁵, Q⁶, R², M, X, Y und Z die oben angegebenen Bedeutungen haben und L für Schwefel steht, erhält, wenn man Verbindungen der oben gezeigten Formeln (I-1-a) bis (I-8-a), in welchen A, B, D, Q¹, Q², Q³, Q⁴, Q⁵, Q⁶, X, Y und Z die oben angegebenen Bedeutungen haben, jeweils
   mit Chlormonothioameisensäureestern oder Chlordithioameisensäureestern der Formel (XVI) 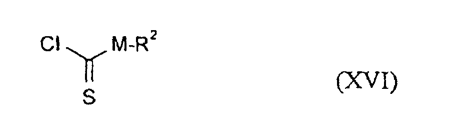 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(M) daß man Verbindungen der oben gezeigten Formeln (I-1-d) bis (I-8-d), in welchen A, B, D, Q¹, Q², Q³, Q⁴, Q⁵, Q⁶, R³, X, Y und Z die oben angegebenen Bedeutungen haben, erhält, wenn man Verbindungen der oben gezeigten Formeln (I-1-a) bis (1-8-a), in welchen A, B, D, Q¹, Q², Q³, Q⁴, Q⁵, Q⁶, X, Y und Z die oben angegebenen Bedeutungen haben, jeweils
   mit Sulfonsäurechloriden der Formel (XVII)

   R³-SO₂-Cl (XVII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(N) daß man Verbindungen der oben gezeigten Formeln (I-1-e) bis (I-8-e), in welchen A, B, D, L, Q¹, Q², Q³, Q⁴, Q⁵, Q⁶, R⁴, R⁵, X, Y und Z die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, X, Y und Z die oben angegebenen Bedeutungen haben, jeweils
   mit Phosphorverbindungen der Formel (XVIII) 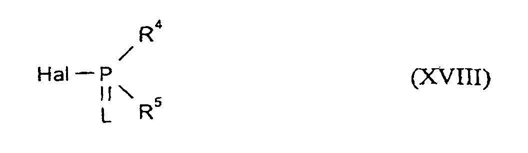 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(O) daß man Verbindungen der oben gezeigten Formeln (I-1-f) bis (I-8-f), in welchen A, B, D, E, Q¹, Q², Q³, Q⁴, Q⁵, Q⁶, X, Y und Z die oben angegebenen Bedeutungen haben, erhält, wenn man Verbindungen der Formeln (I-1-a) bis (I-8-a), in welchen A, B, D, Q¹, Q², Q³, Q⁴, Q⁵, Q⁶, X, Y und Z die oben angegebenen Bedeutungen haben, jeweils
   mit Metallverbindungen oder Aminen der Formeln (XIX) oder (XX)

   Me(OR¹⁰)ₜ (XIX)

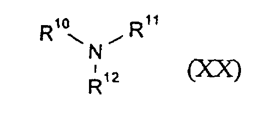

   in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(P) daß man Verbindungen der oben gezeigten Formeln (I-1-g) bis (I-8-g), in welchen A, B, D, L, Q¹, Q², Q³, Q⁴, Q⁵, Q⁶, R⁶, R⁷, X, Y und Z die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, X, Y und Z die oben angegebenen Bedeutungen haben, jeweils
   (α) mit Isocyanaten oder Isothiocyanaten der Formel (XXI)

      R⁶-N=C=L (XXI)

      in welcher
      - R⁶: und L die oben angegebenen Bedeutungen haben,
      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XXII) 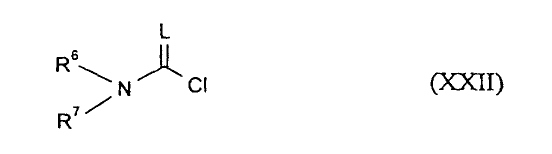 in welcher
      - L, R⁶ und R⁷: die oben angegebenen Bedeutungen haben,
gegebenenfalls in Gegenwart eines Verdünnungsmittels und gegebenenfalls in Gegenwart eines Säurebindemittels, umsetzt.

Weiterhin wurde gefunden, daß die neuen Verbindungen der Formel (I) eine sehr gute Wirksamkeit als Schädlingsbekämpfungsmittel, vorzugsweise als Insektizide, Akarizide und auch als Herbizide aufweisen.

Insbesondere bevorzugt sind Verbindungen der Formel (I), in denen G für Wasserstoff steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

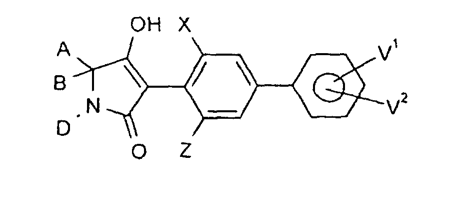

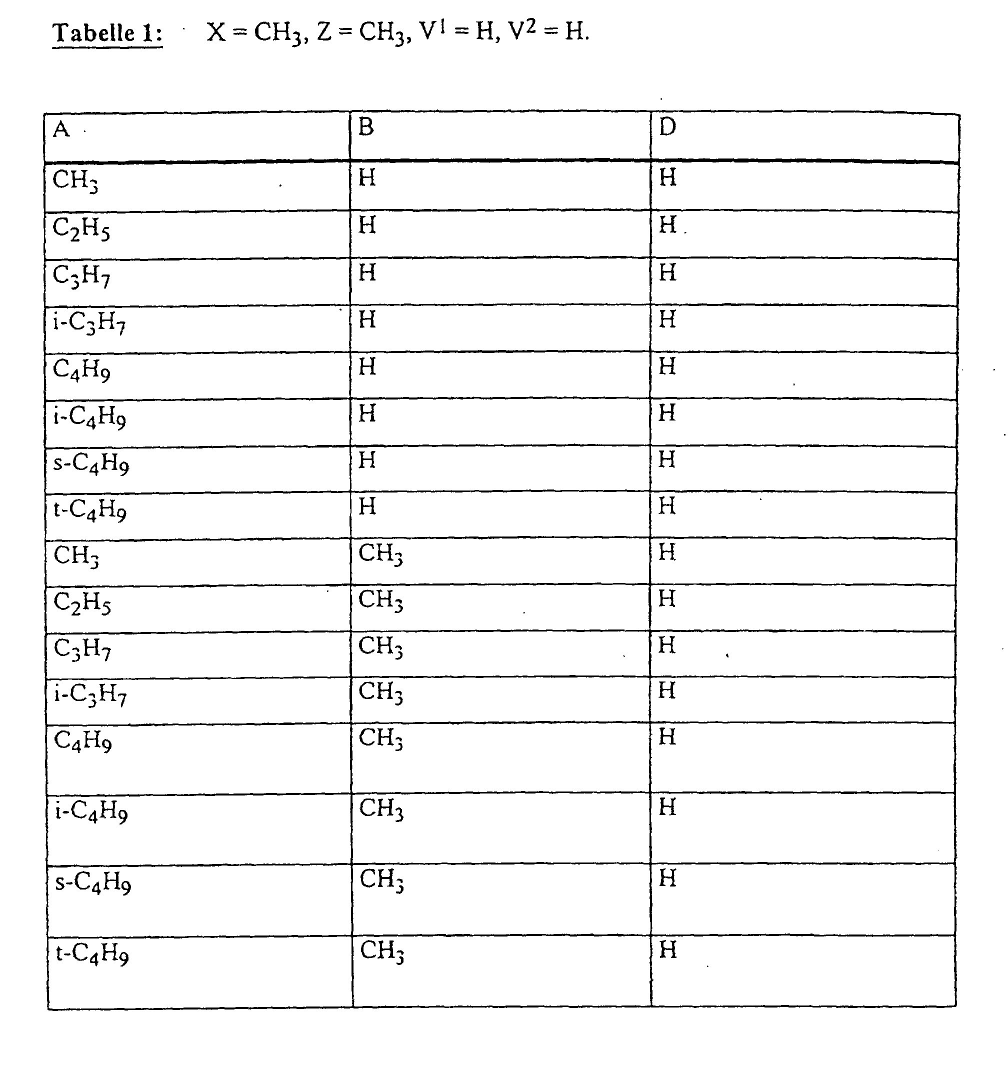

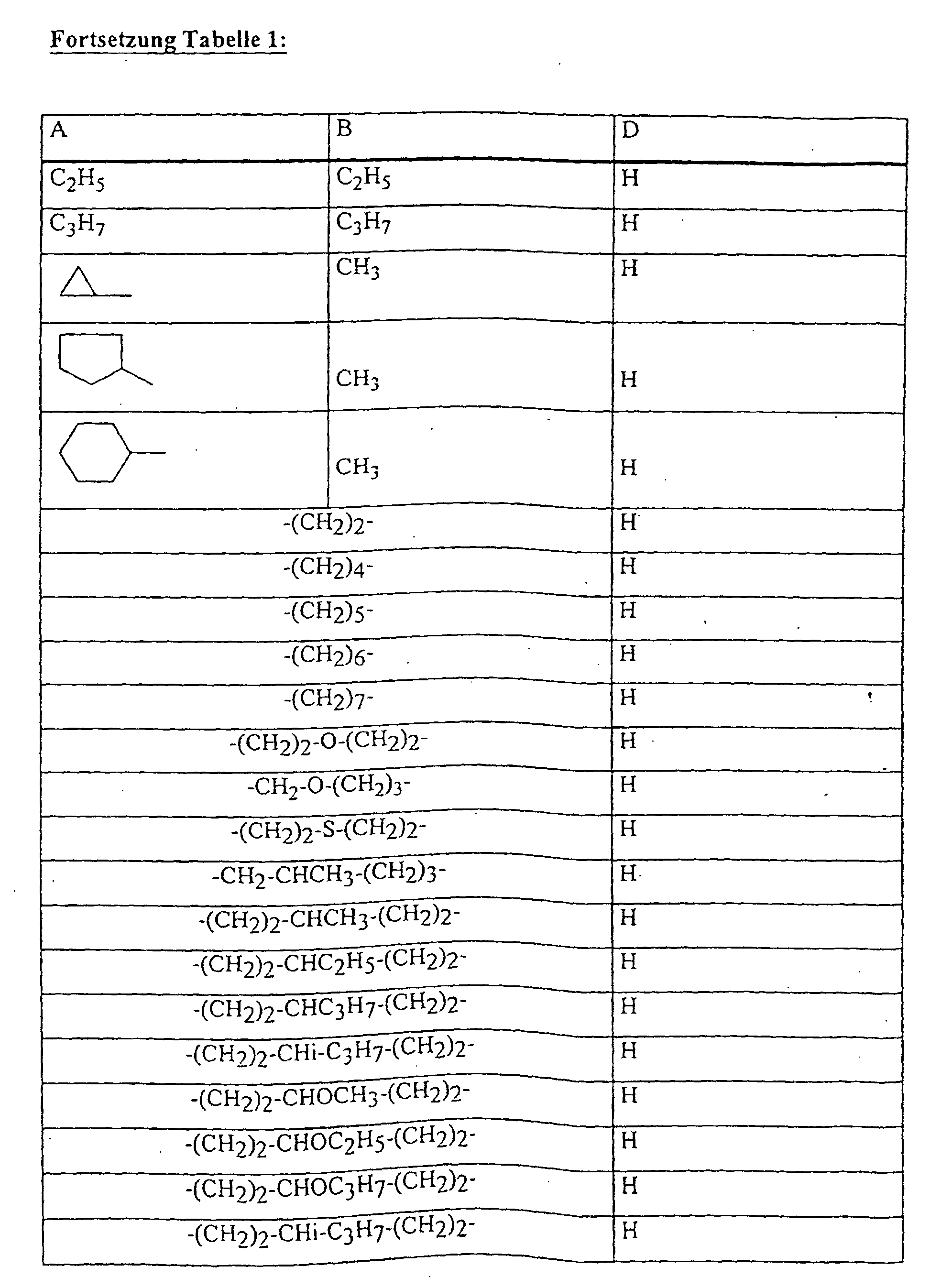

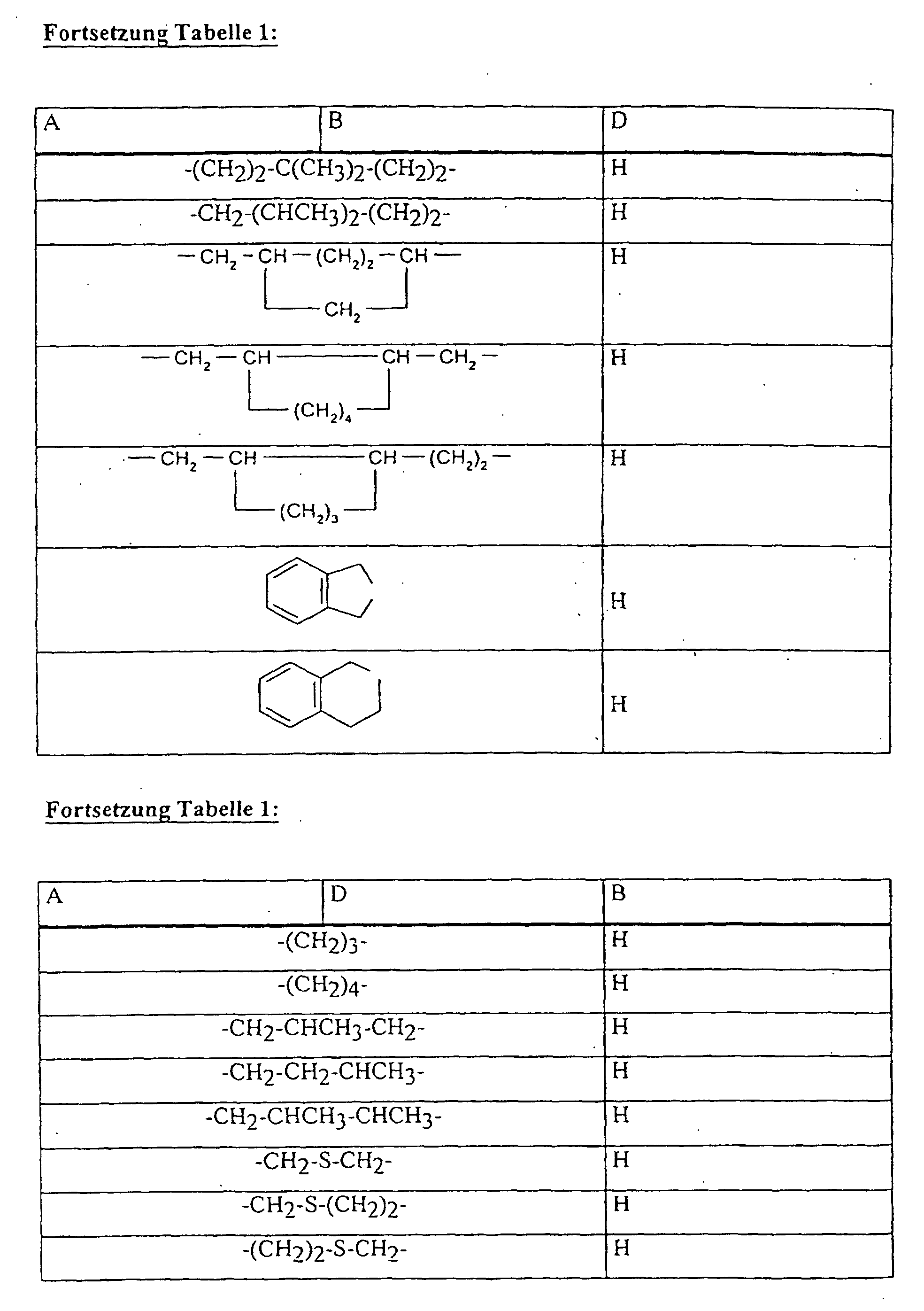

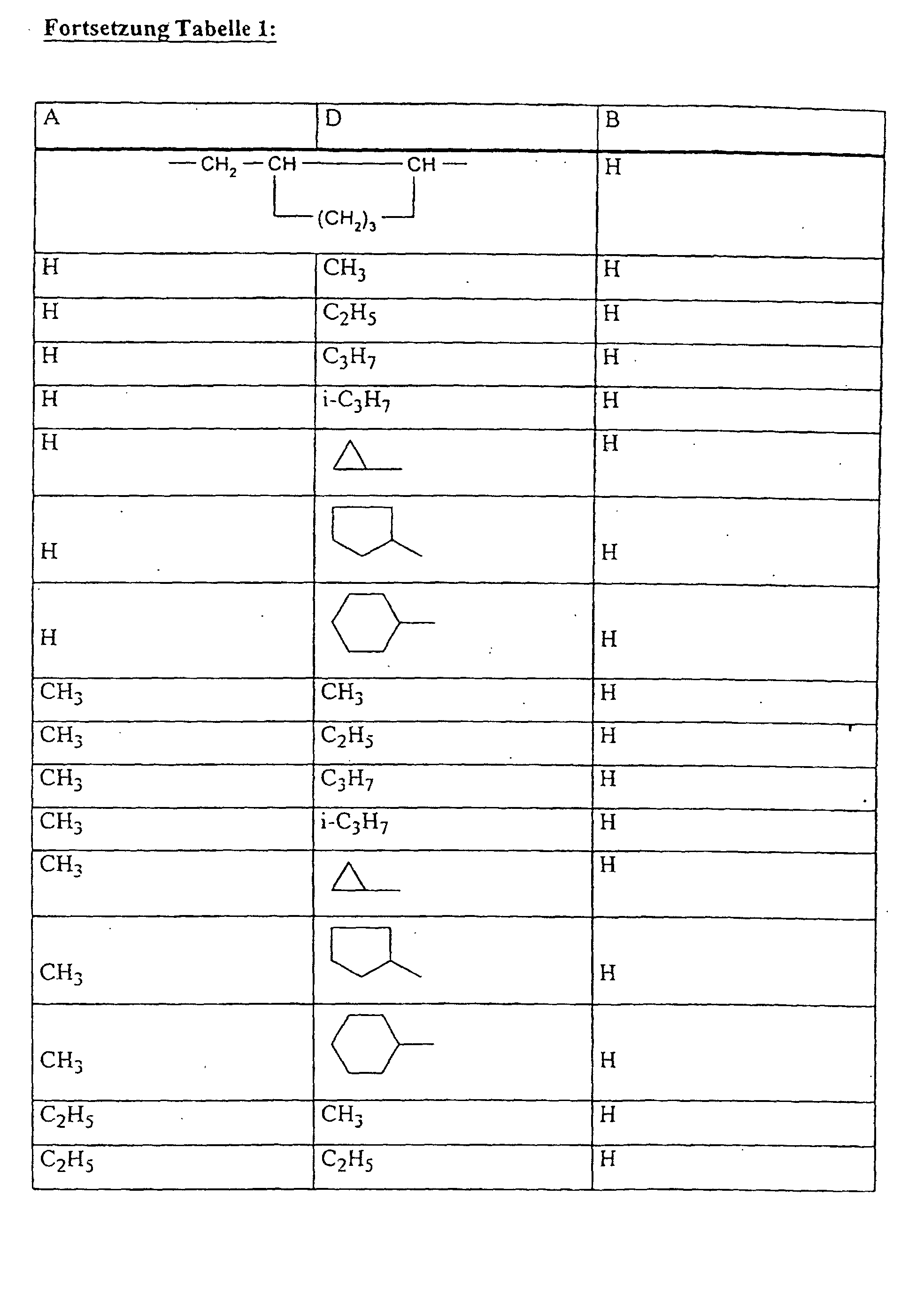

- **Tabelle 2:**: A, B und D wie in Tabelle 1 angegeben
X = C₂H₅; Z = CH₃; V¹ = H; V² = H.
- **Tabelle 3:**: A, B und D wie in Tabelle 1 angegeben
X = C₂H₅; Z = C₂H₅; V¹ = H; V² = H.
- **Tabelle 4:**: A, B und D wie in Tabelle 1 angegeben
X = CH₃; Z = CH₃; V¹ = 4-Cl; V² = H.
- **Tabelle 5:**: A, B und D wie in Tabelle 1 angegeben
X = C₂H₅; Z = CH₃; V¹ = 4-Cl; V² = H.
- **Tabelle 6:**: A, B und D wie in Tabelle 1 angegeben
X = C₂H₅; Z = C₂H₅; V¹ = 4-Cl; V² = H.
- **Tabelle 7:**: A, B und D wie in Tabelle 1 angegeben
X = CH₃; Z = CH₃; V¹ = 3-Cl; V² = H.
- **Tabelle 8:**: A, B und D wie in Tabelle 1 angegeben
X = C₂H₅; Z = CH₃; V¹ = 3-Cl; V² = H.
- **Tabelle 9:**: A, B und D wie in Tabelle 1 angegeben
X = C₂H₅; Z = C₂H₅; V¹ = 3-Cl; V² = H.
- **Tabelle 10:**: A, B und D wie in Tabelle 1 angegeben
X = CH₃; Z = CH₃; V¹ = 2-Cl; V² = 4-Cl.
- **Tabelle 11:**: A, B und D wie in Tabelle 1 angegeben
X = C₂H₅; Z = CH₃; V¹ = 2-Cl; V² = 4-Cl.
- **Tabelle 12:**: A, B und D wie in Tabelle 1 angegeben
X = C₂H₅; Z = C₂H₅; V¹ = 2-Cl; V² = 4-Cl.
- **Tabelle 13:**: A, B und D wie in Tabelle 1 angegeben
X = CH₃; Z = CH₃; V¹ = 4-CF₃; V² = H.
- **Tabelle 14:**: A, B und D wie in Tabelle 1 angegeben
X = C₂H₅; Z = CH₃; V¹ = 4-CF₃; V² = H.
- **Tabelle 15:**: A, B und D wie in Tabelle 1 angegeben
X = C₂H₅; Z = C₂H₅; V¹ = 4-CF₃; V² = H.
- **Tabelle 16:**: A, B und D wie in Tabelle 1 angegeben
X = CH₃; Z = CH₃; V¹ = 4-CH₃; V² = H.
- **Tabelle 17:**: A, B und D wie in Tabelle 1 angegeben
X = C₂H₅; Z = CH₃; V¹ = 4-CH₃; V² = H.
- **Tabelle 18:**: A, B und D wie in Tabelle 1 angegeben
X = C₂H₅; Z = C₂H₅; V¹ = 4-CH₃; V² = H.
- **Tabelle 19:**: A, B und D wie in Tabelle 1 angegeben
X = CH₃; Z = CH₃; V¹ = 4-OCH₃; V² = H.
- **Tabelle 20:**: A, B und D wie in Tabelle 1 angegeben
X = C₂H₅; Z = CH₃; V¹ = 4-OCH₃; V² = H.
- **Tabelle 21:**: A, B und D wie in Tabelle 1 angegeben
X = C₂H₅; Z = C₂H₅; V¹ = 4-OCH₃; V² = H.

Im einzelnen seien außer den bei den Herstellungsbeispielen genannten Verbindungen die folgenden Verbindungen der Formel (I-2-a) genannt:

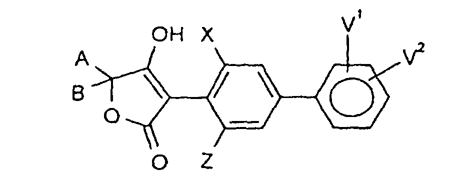

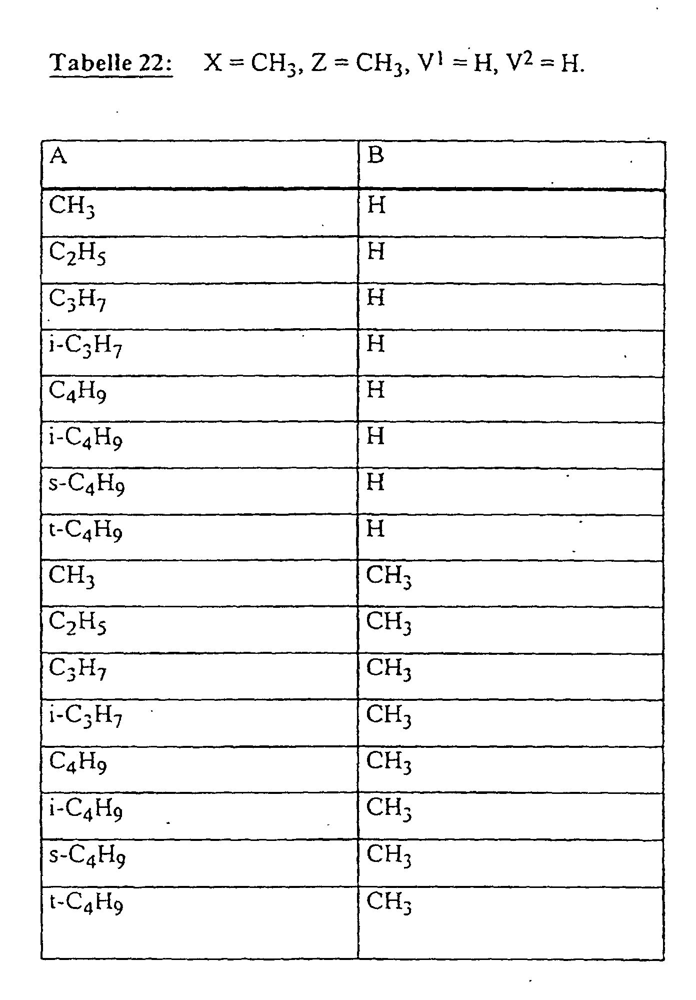

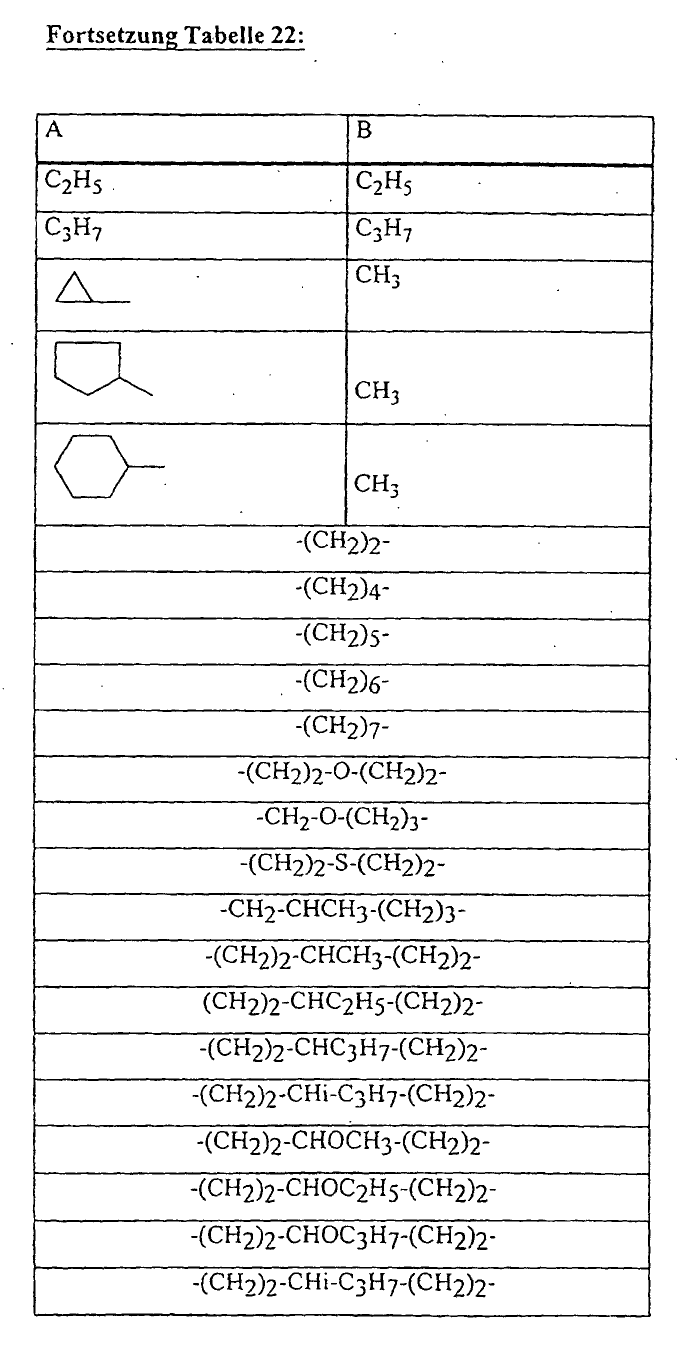

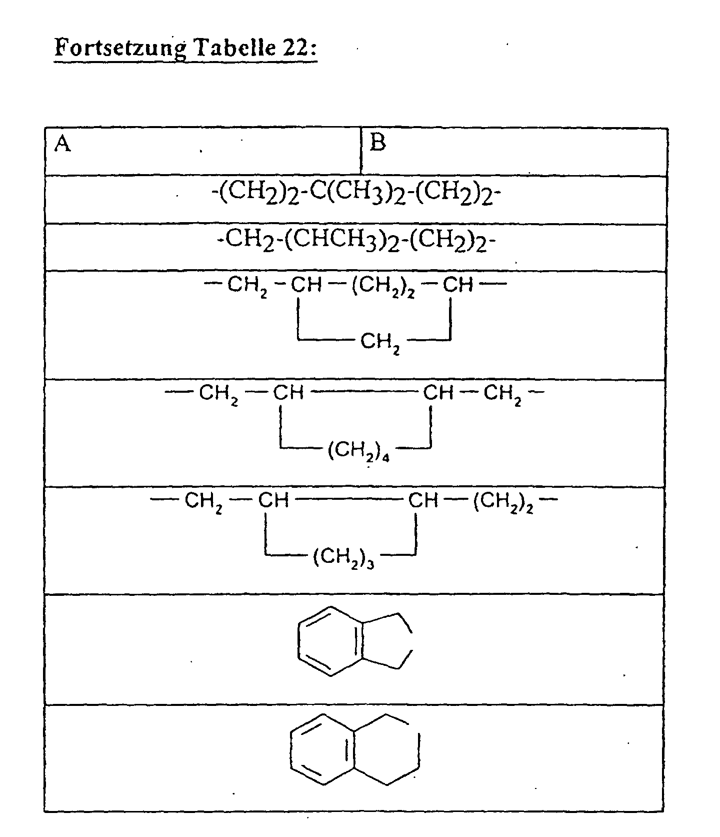

- **Tabelle 23:**: A und B wie in Tabelle 22 angegeben
X = C₂H₅; Z = CH₃; V¹ = H; V² = H.
- **Tabelle 24:**: A und B wie in Tabelle 22 angegeben
X = C₂H₅; Z = C₂H₅; V¹ = H; V² =H.
- **Tabelle 25:**: A und B wie in Tabelle 22 angegeben
X = CH₃; Z = CH₃; V¹ = 4-Cl; V² = H.
- **Tabelle 26:**: A und B wie in Tabelle 22 angegeben
X = C₂H₅; Z = CH₃; V¹ = 4-Cl; V² = H.
- **Tabelle 27:**: A und B wie in Tabelle 22 angegeben
X = C₂H₅; Z = C₂H₅; V¹ = 4-Cl; V² = H.
- **Tabelle 28:**: A und B wie in Tabelle 22 angegeben
X = CH₃; Z = CH₃; V¹ = 3-Cl; V² = H.
- **Tabelle 29:**: A und B wie in Tabelle 22 angegeben
X = C₂H₅; Z = CH₃; V¹ = 3-Cl; V² = H.
- **Tabelle 30:**: A und B wie in Tabelle 22 angegeben
X = C₂H₅; Z = C₂H₅; V¹ = 3-Cl; V² = H.
- **Tabelle 31:**: A und B wie in Tabelle 22 angegeben
X = CH₃; Z = CH₃; V¹ = 4-CF₃; V² = H.
- **Tabelle 32:**: A und B wie in Tabelle 22 angegeben
X = C₂H₅; Z = CH₃; V¹ = 4-CF₃; V² = H.
- **Tabelle 33:**: A und B wie in Tabelle 22 angegeben
X = C₂H₅; Z = C₂H₅; V¹ = 4-CF₃; V² = H.
- **Tabelle 34:**: A und B wie in Tabelle 22 angegeben
X = CH₃; Z = CH₃; V¹ = 2-Cl; V² = 4-Cl.
- **Tabelle 35:**: A und B wie in Tabelle 22 angegeben
X = C₂H₅; Z = CH₃; V¹ = 2-Cl; V² = 4-Cl.
- **Tabelle 36:**: A und B wie in Tabelle 22 angegeben
X = C₂H₅; Z = C₂H₅; V¹ = 2-Cl; V¹ = 4-Cl.
- **Tabelle 37:**: A und B wie in Tabelle 22 angegeben
X = CH₃; Z = CH₃; V¹ = 4-CH₃; V² = H.
- **Tabelle 38:**: A und B wie in Tabelle 22 angegeben
X = C₂H₅; Z = CH₃; V¹ = 4-CH₃; V² = H.
- **Tabelle 39:**: A und B wie in Tabelle 22 angegeben
X = C₂H₅; Z = C₂H₅; V¹ = 4-CH₃; V² = H.
- **Tabelle 40:**: A und B wie in Tabelle 22 angegeben
X = CH₃; Z = CH₃; V¹ = 4-OCH₃; V² = H.
- **Tabelle 41:**: A und B wie in Tabelle 22 angegeben
X = C₂H₅; Z = CH₃; V¹ = 4-OCH₃; V² = H.
- **Tabelle 42:**: A und B wie in Tabelle 22 angegeben
X = C₂H₅; Z = C₂H₅; V¹ = 4-OCH₃; V² = H.

Verwendet man gemäß Verfahren (A) N-[(2-Methyl-4-phenyl)-phenylacetyl]-1-amino-cyclohexan-carbonsäureethylester als Ausgangsstoff, so kann der Verlauf des erfindungsgemäßen Verfahrens durch folgendes Reaktionsschema wiedergegeben werden:

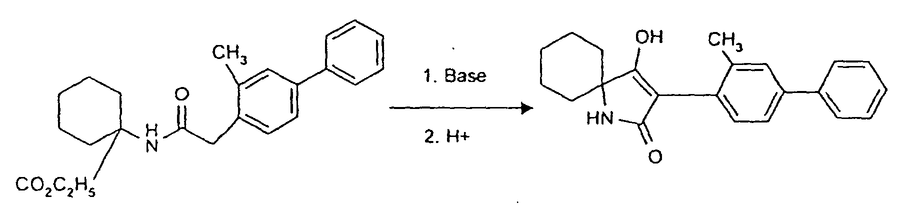

Verwendet man gemäß Verfahren (B) O-[(2-Chlor-4-(4-chlor)-phenyl)-phenylacetyl]-2-hydroxyisobuttersäureethylester, so kann der Verlauf des erfindungsgemäßen Verfahrens durch folgendes Reaktionsschema wiedergegeben werden:

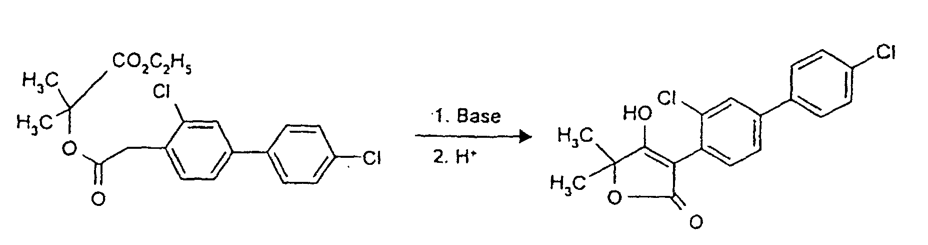

Verwendet man gemäß Verfahren (C) 2-[(2,6-Dimethyl-4-phenyl)-phenyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

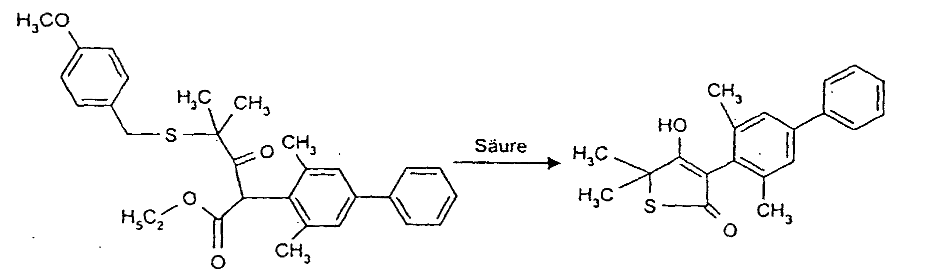

Verwendet man beispielsweise gemäß Verfahren (D-α) (Chlorcarbonyl)-3-[(2-chlor-6-methyl-4-(4-methyl)-phenyl))-phenyl]-keten und 1,2-Diazacyclopentan als Ausgangsverbindungen, so kann der Verlauf des erfindungsgemäßen Verfahrens durch folgendes Reaktionsschema wiedergegeben werden:

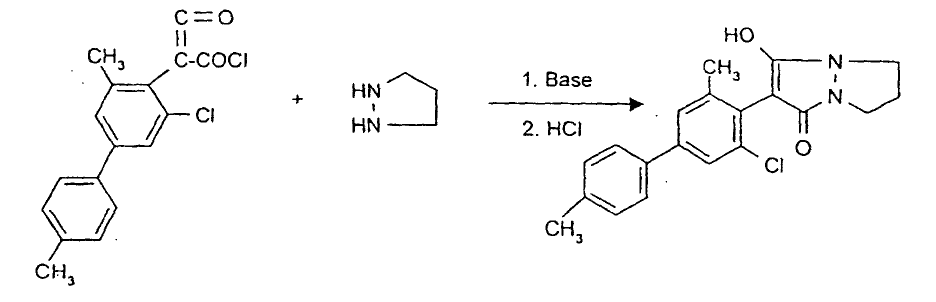

Verwendet man beispielsweise gemäß Verfahren (D-β) 3-[2-Methyl-4-(3-chlor-phenyl)]-phenylmalonsäurediethylester und 1,2-Diazacyclopentan als Ausgangsverbindungen, so kann der Verlauf des erfindungsgemäßen Verfahrens durch das folgende Reaktionsschema wiedergegeben werden:

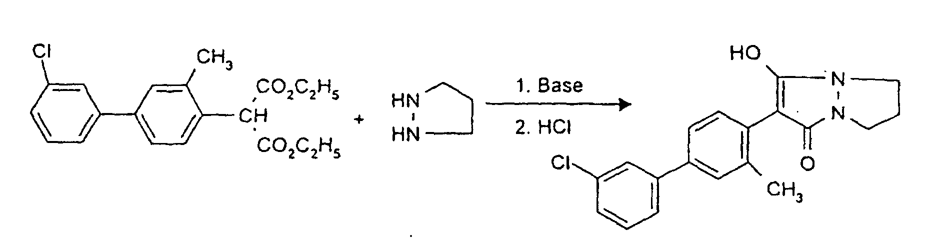

Verwendet man beispielsweise gemäß Verfahren (E) (Chlorcarbonyl)-2-[(2-ethyl-6-methyl-(4-trifluormethoxy-phenyl))-phenyl]-keten und Aceton als Ausgangsverbindungen, so kann der Verlauf des erfindungsgemäßen Verfahrens durch das folgende Reaktionsschema wiedergegeben werden:

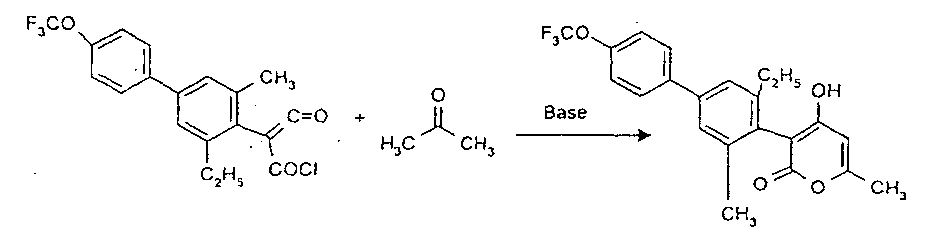

Verwendet man beispielsweise gemäß Verfahren (F) (Chlorcarbonyl)-2-[(2,6-dimethyl-4-phenyl)-phenyl]-keten und Thiobenzamid als Ausgangsverbindungen, so kann der Verlauf des erfindungsgemäßen Verfahrens durch das folgende Reaktionsschema wiedergegeben werden:

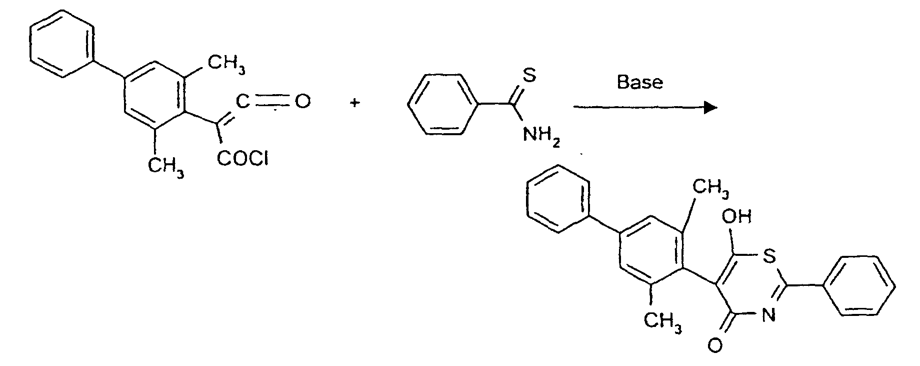

Verwendet man gemäß Verfahren (G) 5-[(2-Chlor-6-methyl-4-phenyl)-phenyl]-2,3-tetramethylen-4-oxo-valeriansäureethylester, so kann der Verlauf des erfindungsgemäßen Verfahrens durch folgendes Reaktionsschema wiedergegeben werden:

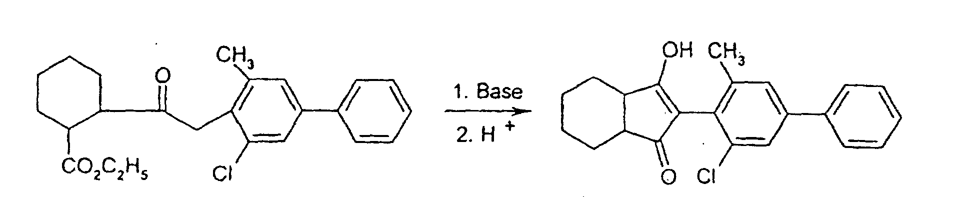

Verwendet man gemäß Verfahren (H) 5-[(2,6-Dichlor-4-phenyl)-phenyl]-2,2-dimethyl-5-oxo-hexansäure-ethylester, so kann der Verlauf des erfindungsgemäßen Verfahrens durch folgendes Reaktionsschema wiedergegeben werden:

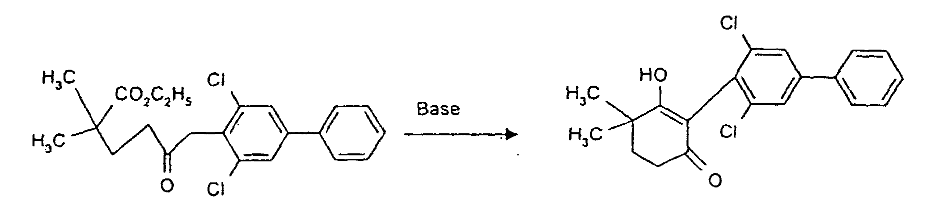

Verwendet man gemäß Verfahren (I) 3-[(2,6-Dimethyl-4-brom)-phenyl]-4,4-(pentamethylen)-pyrrolidin-2,4-dion und 4-Chlorphenylboronsäure als Ausgangsprodukte, so kann der Reaktionsverlauf durch folgendes Schema wiedergegeben werden:

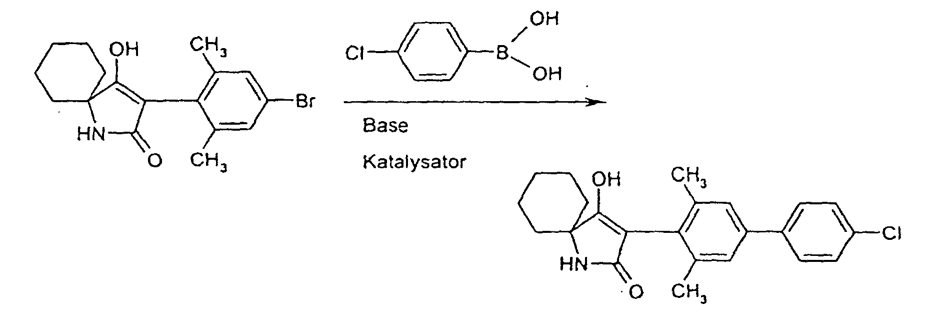

Verwendet man gemäß Verfahren (Jα) 3-[(2-Chlor-4-(3-chlor-phenyl))-phenyl]-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

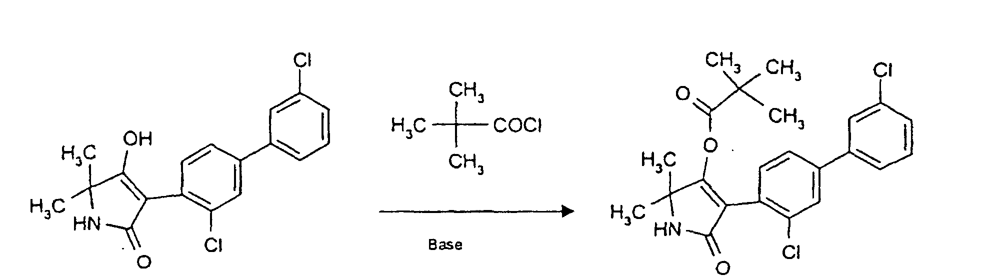

Verwendet man gemäß Verfahren (J) (Variante β) 3-[(2-Ethyl-4-(4-methoxy-phenyl))-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

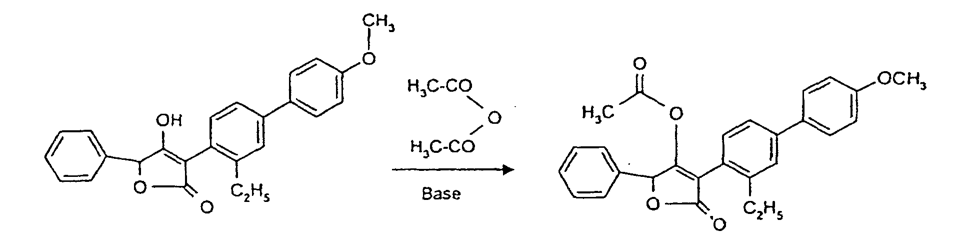

Verwendet man gemäß Verfahren (K) 8-[(2,6-Diethyl-4-phenyl)-phenyl]-1,6-diazabicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

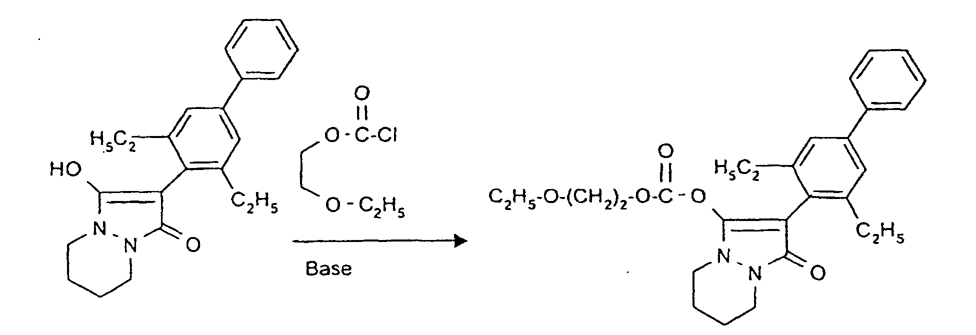

Verwendet man gemäß Verfahren (L), 3-[(2-Chlor-4-(4-fluor-phenyl))-phenyl]-4-hydroxy-5-methyl-6-(3-pyridyl)-pyron und Chlormonothioameisensäuremethylester als Ausgangsprodukte, so kann der Reaktionsverlauf folgendermaßen wiedergegeben werden:

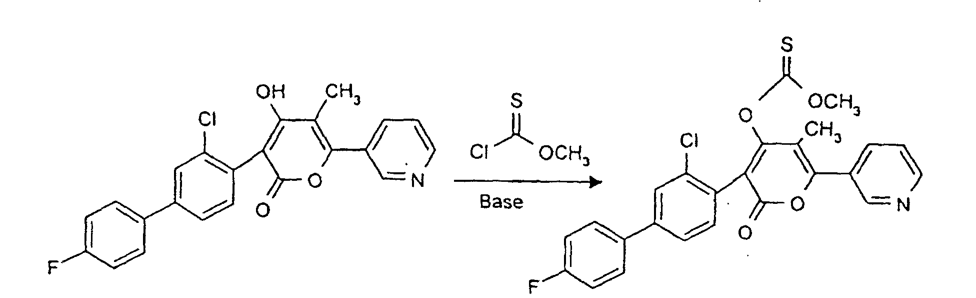

Verwendet man gemäß Verfahren (M) 2-[(2,6-Dimethyl-4-(4-methyl-phenyl))-phenyl]-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

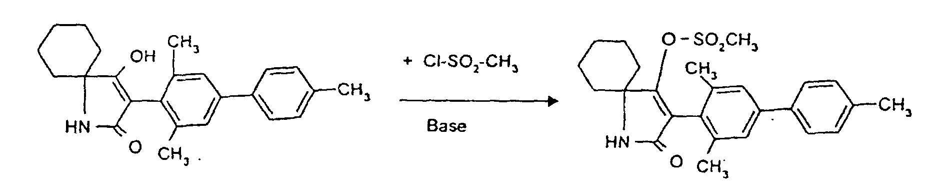

Verwendet man gemäß Verfahren (N) 2-[(2-Methyl-4-phenyl)-phenyl]-4-hydroxy-5,5-dimethyl-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

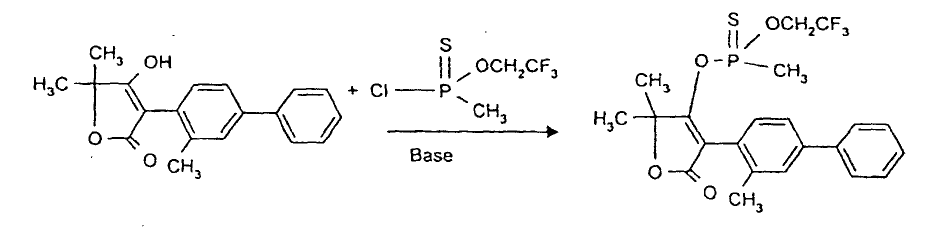

Verwendet man gemäß Verfahren (O) 3-[(2-Trifluormethyl-4-(4-trifluormethylphenyl))-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

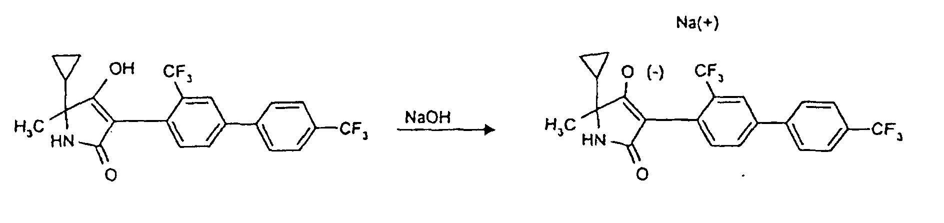

Verwendet man gemäß Verfahren (P) Variante α 3-[(2-Methyl-4-(3-trifluormethylphenyl))-phenyl]-4-hydroxy-5-tetramethylen-Δ³-dihydro-furan-2-on und Ethylisocyanat als Ausgangsprodukte, so kann der Reaktionsverlauf durch folgendes Reaktionsschema wiedergegeben werden:

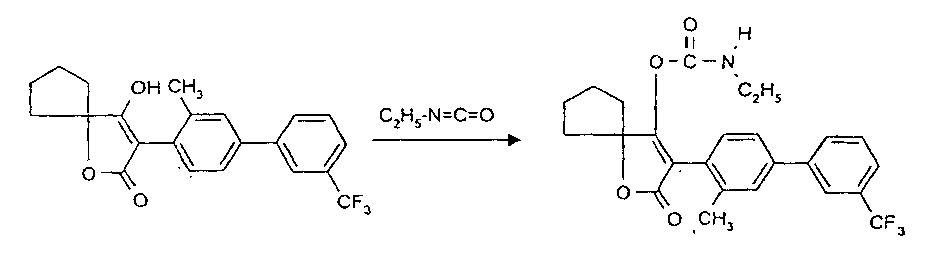

Verwendet man gemäß Verfahren (P) Variante β 3-[(2-Chlor-4-phenyl)-phenyl]-5-methyl-pyrrolidin-2,4-dion und Dimethylcarbamidsäurechlorid als Ausgangsprodukte, so kann der Reaktionsverlauf durch folgendes Schema wiedergegeben werden:

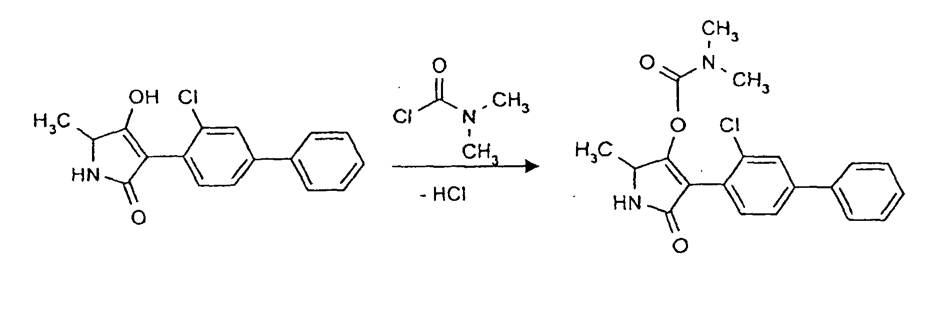

Die beim erfindungsgemäßen Verfahren (a) als Ausgangsstoffe benötigten Verbindungen der Formel (II) 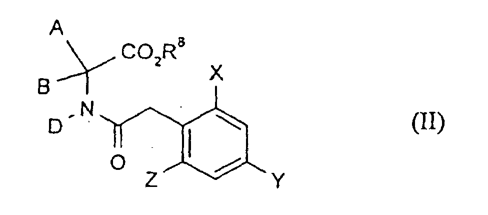 in welcher
- A, B, D, X, Y, Z und R⁸: die oben angegebenen Bedeutungen haben,
sind neu.

Man erhält die Acylaminosäureester der Formel (II) beispielsweise, wenn man Aminosäurederivate der Formel (XXIII) 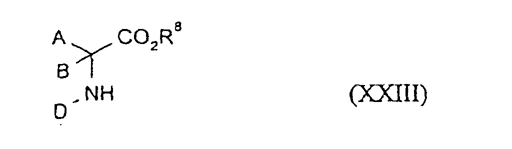 in welcher
- A, B, R⁸ und D: die oben angegebenen Bedeutungen haben,
mit substituierten Phenylessigsäurehalogeniden der Formel (XXIV) 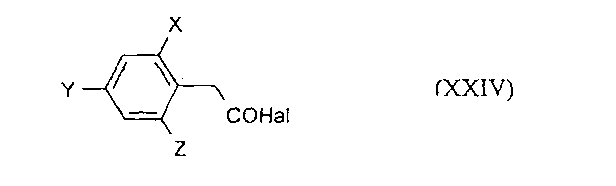 in welcher
- X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXV) 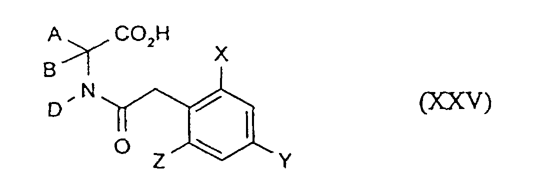 in welcher
- A, B, D, X, Y und Z: die oben angegebenen Bedeutungen haben,
verestert (Chem. Ind. (London) 1568 (1968)).

Die Verbindungen der Formel (XXV) 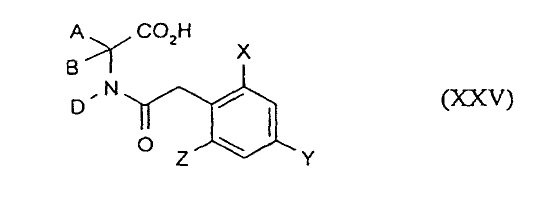 in welcher
- A, B, D, X, Y und Z: die oben angegebenen Bedeutungen haben,
sind neu.

Man erhält die Verbindungen der Formel (XXV), wenn man Aminosäuren der Formel (XXVI) 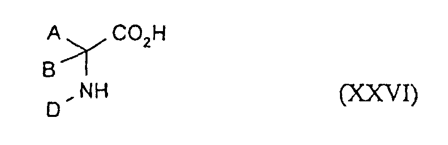 in welcher
- A, B und D: die oben angegebenen Bedeutungen haben,
mit substituierten Phenylessigsäurehalogeniden der Formel (XXIV) 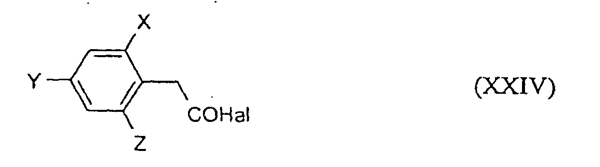 in welcher
- X, Y und Z: die oben angegebenen Bedeutungen haben und
- Hal: für Chlor oder Brom steht,
beispielsweise nach Schotten-Baumann acyliert (Organikum, VEB Deutscher Verlag der Wissenschaften, Berlin 1977, S. 505).

Die Verbindungen der Formel (XXIV) sind neu. Sie lassen sich nach im Prinzip bekannten Verfahren darstellen (s. z.B. H. Henecka, Houben-Weyl, Methoden der Organischen Chemie, Bd. 8, S. 467-469 (1952)).

Man erhält die Verbindungen der Formel (XXIV) beispielsweise, indem man substituierte Phenylessigsäuren der Formel (XXVII) 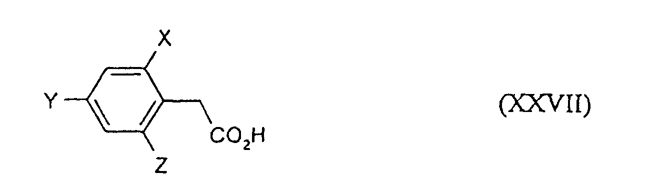 in welcher
- X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXIII) und (XXVI) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXVIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 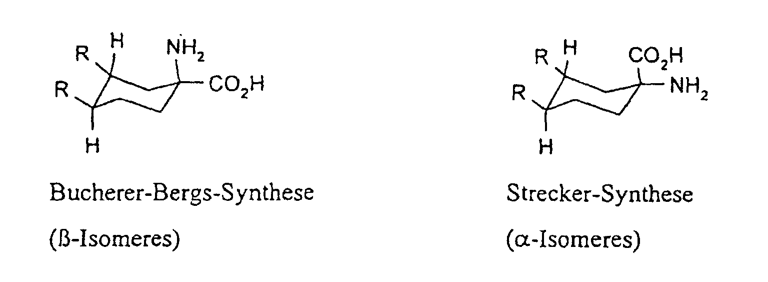 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 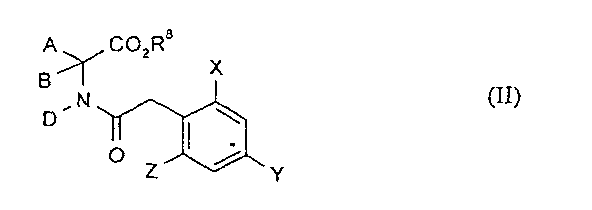 in welcher
- A, B, D, X, Y, Z und R⁸: die oben angegebenen Bedeutungen haben,
herstellen, wenn man Aminonitrile der Formel (XXVIII) 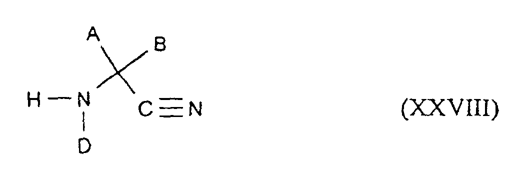 in welcher
- A, B und D: die oben angegebenen Bedeutungen haben,
mit substituierten Phenylessigsäurehalogeniden der Formel (XXIV) 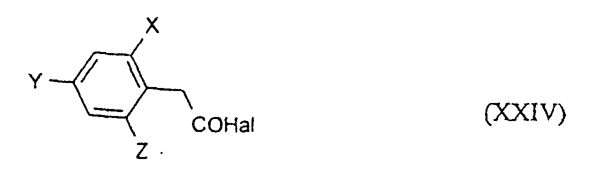 in welcher
- X, Y, Z und Hal: die oben angegebenen Bedeutungen haben,
zu Verbindungen der Formel (XXIX) 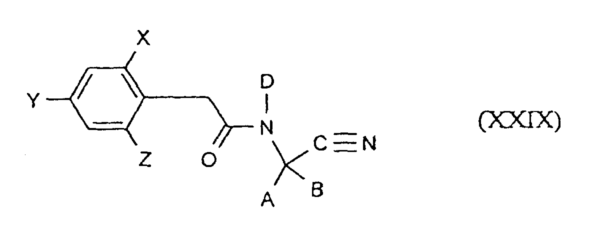 in welcher
- A, B, D, X, Y und Z: die oben angegebenen Bedeutungen haben,
umsetzt,
und diese anschließend einer sauren Alkoholyse unterwirft.

Die Verbindungen der Formel (XXIX) sind ebenfalls neu.

Die bei dem erfindungsgemäßen Verfahren (B) als Ausgangstoffe benötigten Verbindungen der Formel (III) 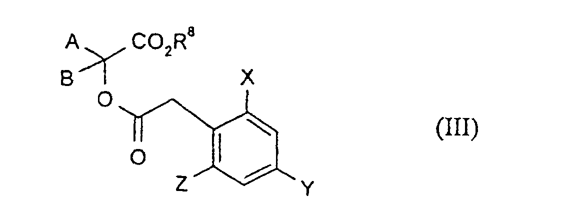 in welcher
- A, B, X, Y, Z und R⁸: die oben angegebenen Bedeutungen haben,
sind neu.

Sie lassen sich nach im Prinzip bekannten Methoden herstellen.

So erhält man die Verbindungen der Formel (III) beispielsweise, wenn man

2-Hydroxycarbonsäureester der Formel (XXX) 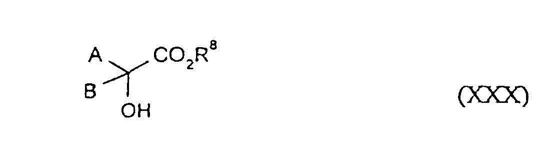 in welcher
- A, B und R⁸: die oben angegebenen Bedeutungen haben,
mit substituierten Phenylessigsäurehalogeniden der Formel (XXIV) 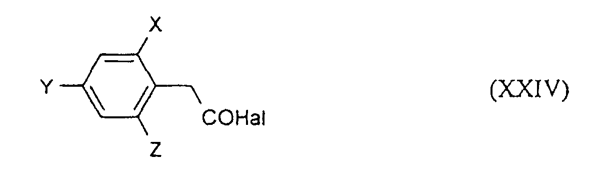 in welcher
- X, Y, Z und Hal: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXVII) 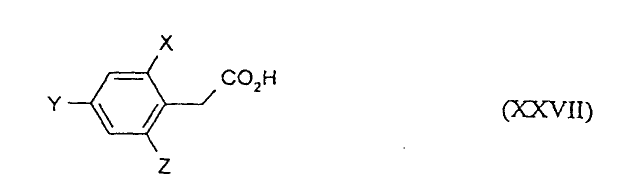 in welcher
- X, Y und Z: die oben angegebenen Bedeutungen haben,
mit α-Halogencarbonsäureestern der Formel (XXXI) 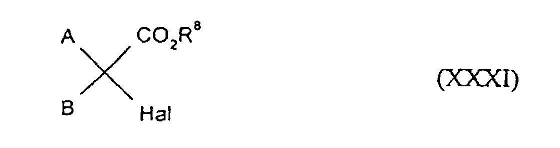 in welcher
- A, B und R⁸: die oben angegebenen Bedeutungen haben und
- Hal: für Chlor oder Brom steht,
alkyliert.

Die Verbindungen der Formel (XXVII) sind neu.

Die Verbindungen der Formel (XXXI) sind bekannt und überwiegend käuflich.

Beispielsweise erhält man die Verbindungen der Formel (XXVII), 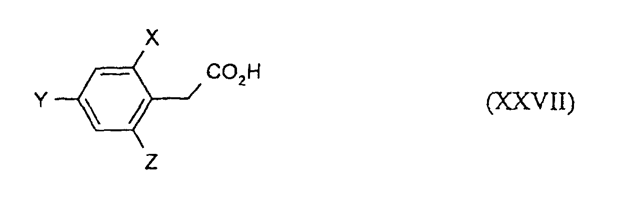 in welcher
- X, Y und Z: die oben angegebenen Bedeutungen haben,

α) wenn man Verbindungen der Formel (XXVII-a) 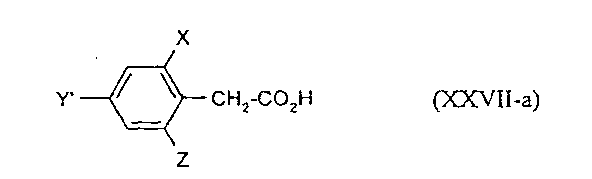 in welcher
   - X und Z: die oben angegebene Bedeutung haben,
   - Y': für Chlor oder Brom, bevorzugt für Brom steht,
   mit Boronsäuren der Formel (XII) 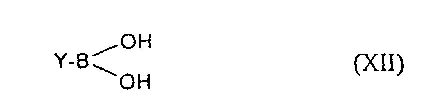 in welcher
   - Y: die oben angegebene Bedeutung hat,
   in Gegenwart eines Lösungsmittels, einer Base und eines Katalysators (bevorzugt eines Palladiumkomplexes, wie z.B. Palladium-tetrakis(triphenylphosphin)) umsetzt oder
β) wenn man Phenylessigsäureester der Formel (XXXII) 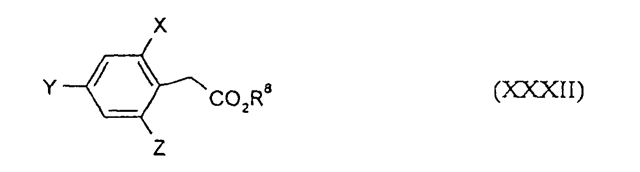 in welcher
   - X, Y, Z und R⁸: die oben angegebene Bedeutung haben,
   in Gegenwart von Säuren oder Basen, in Gegenwart eines Lösungsmittels unter allgemein bekannten Standardbedingungen verseift oder
γ) wenn man Phenylessigsäuren der Formel (XXVII-b) 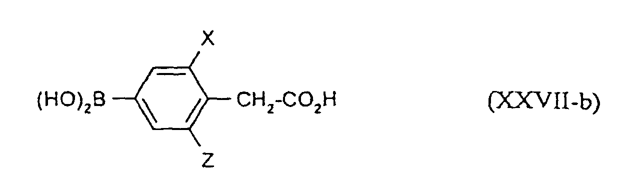 in welcher
   - X und Z: die oben angegebene Bedeutung haben,
   mit Halogenverbindungen der Formel (XXXIII),

   Y-Hal (XXXIII)

   in welcher
   - Y: die oben angegebene Bedeutung hat und
   - Hal: für Chlor, Brom oder Iod steht,
in Gegenwart eines Lösungsmittels, einer Base und eines Katalysators (bevorzugt eines der oben genannten Palladiumkomplexe) umsetzt.

Die Verbindungen der Formeln (XII) und (XXXIII) sind bekannt, teilweise käuflich oder lassen sich nach im Prinzip bekannten Verfahren herstellen. Die Phenylessigsäuren der Formel (XXVII-a) sind teilweise aus WO 96/35 664 und WO 97/02 243 bekannt oder lassen sich nach den dort beschriebenen Verfahren herstellen.

Die Verbindungen der Formeln (XXVII-b) und (XXXII) sind neu.

Man erhält die Verbindungen der Formel (XXVII-b) 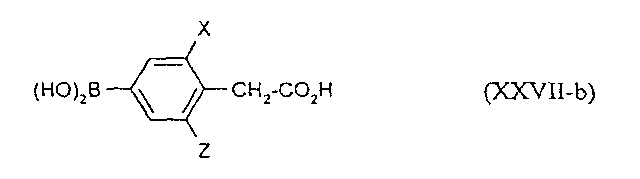 in welcher
- X und Z: die oben angegebene Bedeutung haben,
beispielsweise, wenn man Phenylessigsäuren der Formel (XXVII-a) 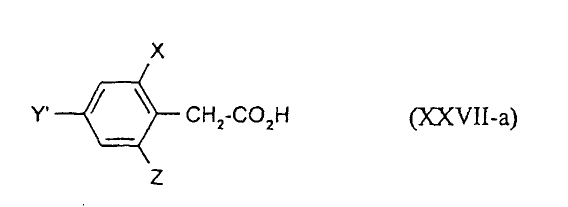 in welcher
- X, Y' und Z: die oben angegebene Bedeutung haben,
mit Lithiumverbindungen der Formel (XXXIV)

Li-R²¹ (XXXIV)

in welcher
- R²¹: für C₁-C₈-Alkyl oder Phenyl, bevorzugt für n-C₄H₉ steht;
und Boronsäureestern der Formel (XXXV)

B(OR⁸)₃ (XXXV)

in welcher
- R⁸: die oben angegebene Bedeutung hat,
in Gegenwart eines Verdünnungsmittels umsetzt.

Die Verbindungen der Formeln (XXXIV) und (XXXV) sind käufliche Verbindungen.

Die Verbindungen der Formel (XXXII) 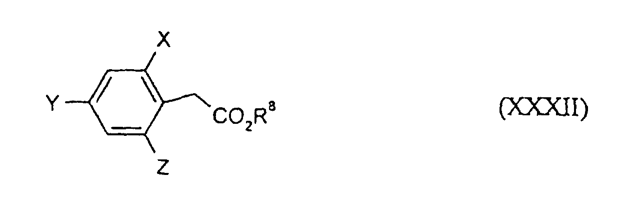 in welcher
- X, Y, Z und R⁸: die oben angegebene Bedeutung haben,
erhält man beispielsweise,
wenn man Phenylessigsäureester der Formel (XXXII-a) 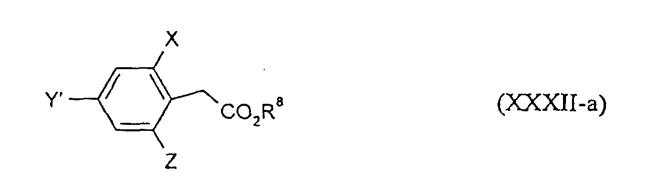 in welcher
- R⁸, X, Y' und Z: die oben angegebene Bedeutung haben,
mit Boronsäuren der Formel (XII) 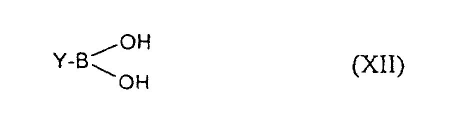 in welcher
- Y: die oben angegebene Bedeutung hat,
in Gegenwart eines Lösungsmittels, einer Base und eines Katalysators (bevorzugt eines der oben genannten Palladiumkomplexe) umsetzt.

Die Phenylessigsäureester der Formel (XXXII-a) sind teilweise aus den Anmeldungen WO 96/35 664 und WO 97/02 243 bekannt oder lassen sich nach den dort beschriebenen Verfahren herstellen.

Die bei dem obigen Verfahren (C) als Ausgangsstoffe benötigten Verbindungen der Formel (IV) 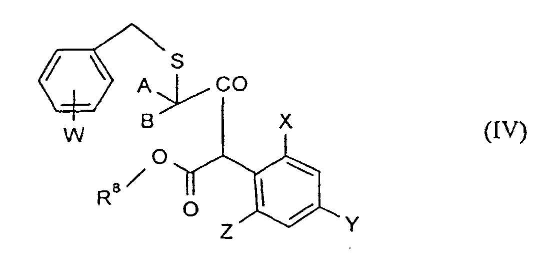 in welcher
- A, B, W, X, Y, Z und R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man
substituierte Phenylessigsäureester der Formel (XXXII) 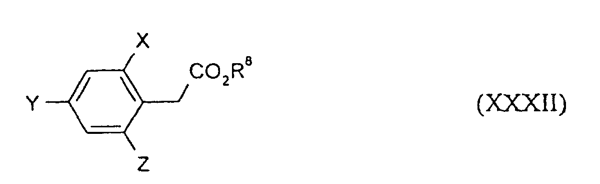 in welcher
- X, Y, R⁸ und Z: die oben angegebenen Bedeutungen haben,
mit 2-Benzylthio-carbonsäurehalogeniden der Formel (XXXVI) 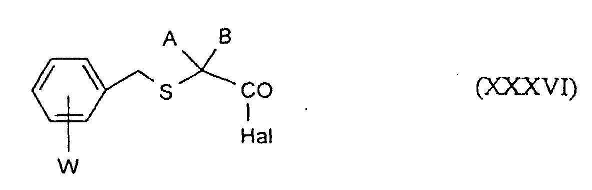 in welcher
- A, B und W: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Verbindungen der Formel (XXXII) sind neu. Man erhält Verbindungen der Formel (XXXII) beispielsweise, wenn man Verbindungen der Formel (XXVII) 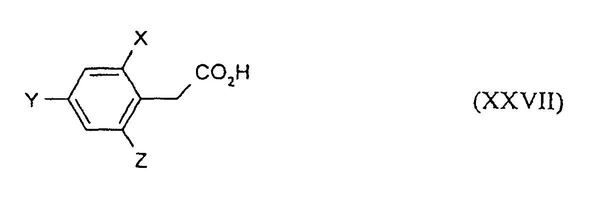 in welcher
- X, Y und Z: die oben angegebene Bedeutung haben,
in Gegenwart von Alkoholen und wasserentziehenden Mitteln (z.B. konz. Schwefelsäure) verestert, oder Alkohole mit Verbindungen der Formel (XXIV) 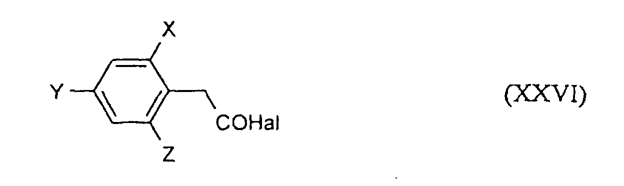 in welcher
- X, Y, Z und Hal: die oben angegebenen Bedeutungen haben
acyliert (Chem. Reviews 52, 237-416 (1953)).

Die Benzylthio-carbonsäurehalogenide der Formel (XXXVI) sind teilweise bekannt und/oder lassen sich nach bekannten Verfahren herstellen (J. Antibiotics (1983), 26, 1589).

Die bei den obigen Verfahren (D), (E) und (F) als Ausgangsstoffe benötigten Halogencarbonylketene der Formel (V) sind neu. Sie lassen sich nach im Prinzip allgemein bekannten Methoden herstellen (vgl. beispielsweise Org. Prep. Proced. Int., 7, (4), 155-158, 1975 und DE 1 945 703). So erhält man z.B. die Verbindungen der Formel (V) 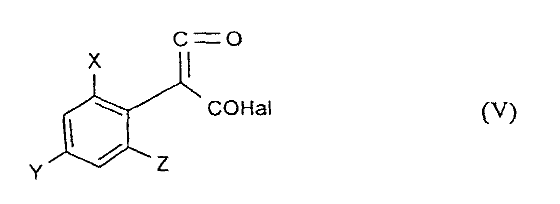 in welcher
- X, Y und Z: die oben angegebenen Bedeutungen haben und
- Hal: für Chlor oder Brom steht,
wenn man
substituierte Phenylmalonsäuren der Formel (XXXVII) 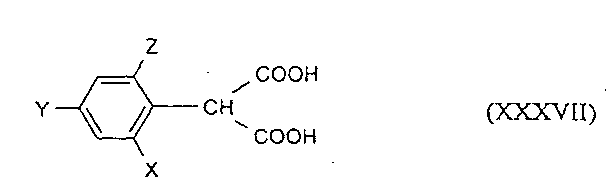 in welcher
- X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Dimethylformamid, Methyl-Sterylformamid oder Triphenylphosphin und gegebenenfalls in Gegenwart von Basen wie z.B. Pyridin oder Triethylamin, umsetzt.

Die substituierten Phenylmalonsäuren der Formel (XXXVII) sind neu. Sie lassen sich nach allgemein bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243).

So erhält man Phenylmalonsäuren der Formel (XXXVII) 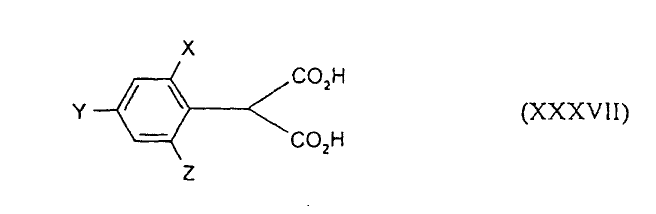 in welcher
- X, Y und Z: die oben angegebenen Bedeutungen haben,
wenn man Phenylmalonsäureester der Formel (VI) 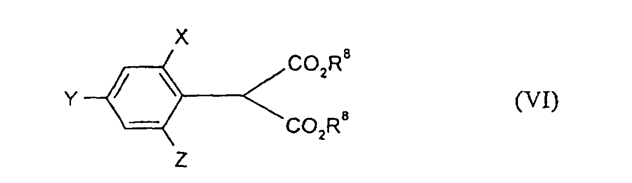 in welcher
- X, Y, Z und R⁸: die oben angegebene Bedeutung haben,
zunächst in Gegenwart einer Base und einem Lösungsmittel verseift und anschließend vorsichtig ansäuert (EP-528 156, WO 96/35 664, WO 97/02 243).

Die Malonsäureester der Formel (VI) 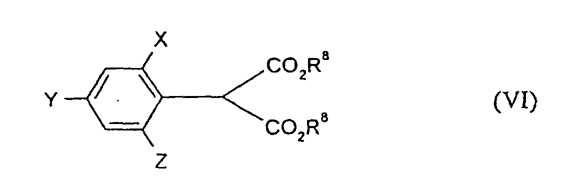 in welcher
- X, Y, Z und R⁸: die oben angegebene Bedeutung haben,
sind neu.

Sie lassen sich nach allgemein bekannten Methoden der Organischen Chemie darstellen (vgl. z.B. Tetrahedron Lett. 27, 2763 (1986) und Organikum VEB Deutscher Verlag der Wissenschaften, Berlin 1977, S. 587 ff.).

Die für das erfindungsgemäße Verfahren (D) als Ausgangsstoffe benötigten Hydrazine der Formel (VII)

A-NH-NH-D (VII),

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP-508 126).

Die für das erfindungsgemäße Verfahren (E) als Ausgangsstoffe benötigten Carbonylverbindungen der Formel (VIII) 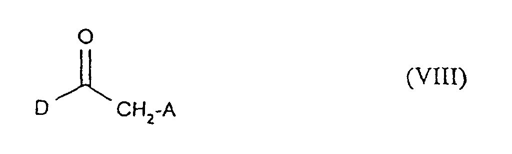 in welcher
- A und D: die oben angegebenen Bedeutungen haben,
oder deren Silylenolether der Formel (VIIIa) 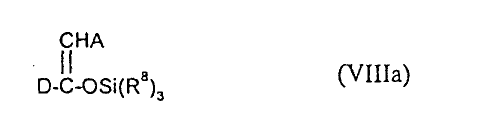 in welcher
- A, D und R⁸: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (F) als Ausgangsstoffe benötigten Ketensäurechloride der Formel (V) wurden bereits oben beschrieben. Die zur Durchführung des erfindungsgemäßen Verfahrens (F) benötigten Thioamide der Formel (IX) 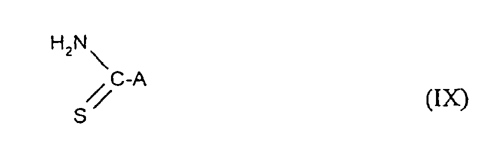 in welcher
- A: die oben angegebene Bedeutung hat,
sind allgemein in der Organischen Chemie bekannte Verbindungen.

Die bei dem obigen Verfahren (G) als Ausgangsstoffe benötigten Verbindungen der Formel (X) 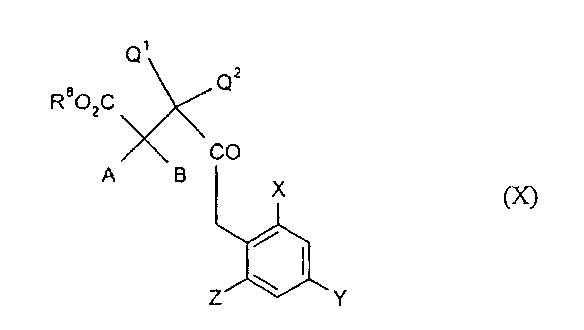 in welcher
- A, B, Q¹, Q², X, Y, Z und R⁸: die oben angegebene Bedeutung haben,
sind neu.

Sie lassen sich nach im Prinzip bekannten Methoden herstellen.

Man erhält die 5-Aryl-4-ketocarbonsäureester der Formel (X) beispielsweise, wenn man 5-Aryl-4-ketocarbonsäuren der Formel (XXXVIII) 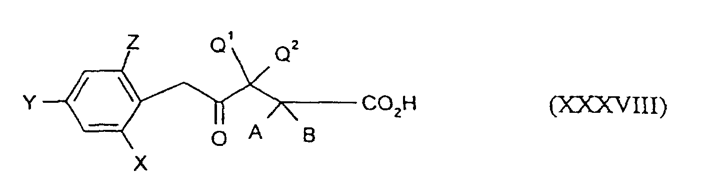 in welcher
- X, Y, Z, A, B, Q¹ und Q²: die oben angegebene Bedeutung haben,
verestert (vgl. z.B. Organikum, 15. Auflage, Berlin, 1977, Seite 499) oder alkyliert (siehe Herstellungsbeispiel).

Die 5-Aryl-4-ketocarbonsäuren der Formel (XXXVIII) 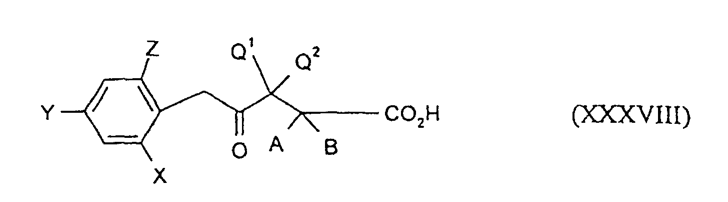 in welcher
- A, B, Q¹, Q², X, Y und Z: die oben angegebene Bedeutung haben,
sind neu, lassen sich aber nach im Prinzip bekannten Methoden herstellen (siehe Herstellungsbeispiel).

Man erhält die 5-Aryl-4-ketocarbonsäuren der Formel (XXXVIII) beispielsweise, wenn man 2-Phenyl-3-oxo-adipinsäureester der Formel (XXXIX) 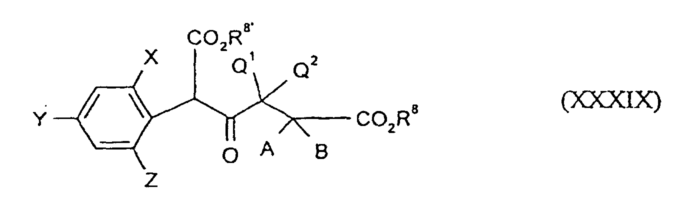 in welcher
- A, B, D¹, D², X, Y und Z: die oben angegebene Bedeutung haben und
- R⁸ und R^{8'}: für Alkyl (insbesondere C₁-C₈-Alkyl) stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XXXIX) 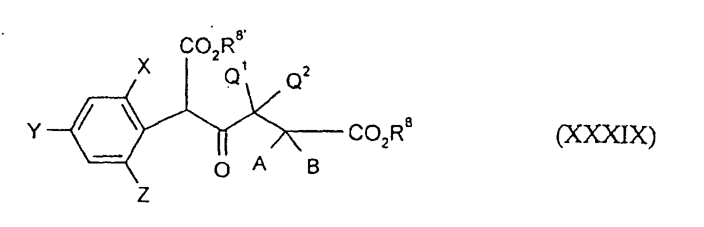 in welcher
- A, B, Q¹, Q², X, Y, Z, R⁸, R^{8'}: die oben angegebene Bedeutung haben,
sind neu.

Man erhält die Verbindungen der Formel (XXXIX) beispielsweise,
wenn man Dicarbonsäurehaloesterchloride der Formel (XL), 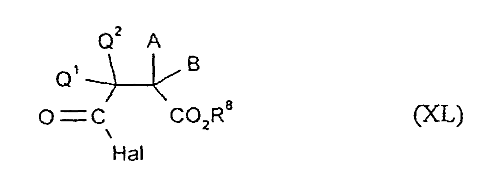 in welcher
- A, B, Q¹, Q² und R⁸: die oben angegebene Bedeutung haben und
- Hal: für Chlor oder Brom steht,
oder Carbonsäureanhydride der Formel (XLI) 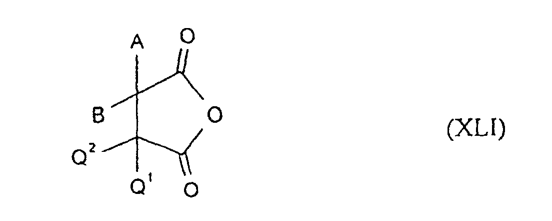 in welcher
- A, B, Q¹ und Q²: die oben angegebene Bedeutung haben,
mit einem Phenylessigsäureester der Formel (XXXII) 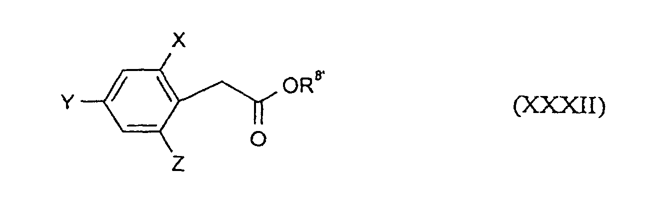 in welcher
- X, Y, Z und R^{8'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XL) und (XLI) sind teilweise bekannte Verbindungen der Organischen Chemie und/oder lassen sich nach im Prinzip bekannten Methoden in einfacher Weise herstellen.

Die bei dem obigen Verfahren (H) als Ausgangsstoffe benötigten Verbindungen der Formel (XI) 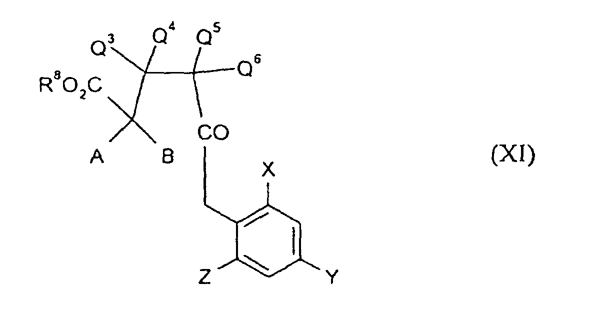 in welcher
- A, B, Q³, Q⁴, Q⁵, Q⁶, X, Y, Z und R⁸: die oben angegebene Bedeutung haben,
sind neu.

Sie lassen sich nach im Prinzip bekannten Methoden herstellen.

Man erhält die 6-Aryl-5-ketocarbonsäureester der Formel (XI) beispielsweise, wenn man 6-Aryl-5-ketocarbonsäuren der Formel (XLII) 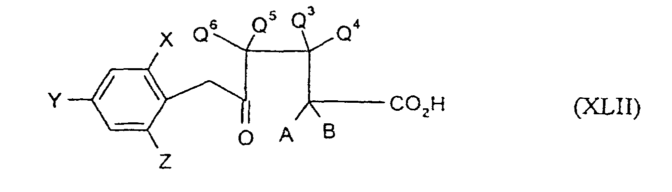 in welcher
- A, B, Q³, Q⁴, Q⁵, Q⁶, X, Y und Z: die oben angegebene Bedeutung haben,
verestert, (vgl. z.B. Organikum, 15. Auflage, Berlin, 1977, Seite 499 und Herstellungsbeispiel).

Die 6-Aryl-5-ketocarbonsäuren der Formel (XLII) 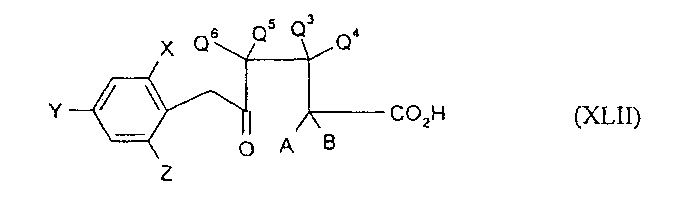 in welcher
- A, B, Q³, Q⁴, Q⁵, Q⁶, X, Y und Z: die oben angegebene Bedeutung haben,
sind neu. Sie lassen sich nach im Prinzip bekannten Methoden herstellen, beispielsweise wenn man
substituierte 2-Phenyl-3-oxo-heptandisäureester der Formel (XLIII) 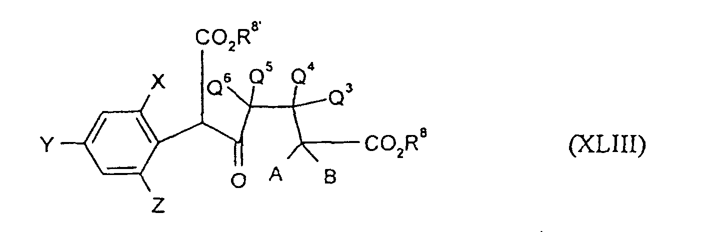 in welcher
- A, B, Q³, Q⁴, Q⁵, Q⁶, X, Y und Z: die oben angegebene Bedeutung haben und
- R⁸ und R^{8'}: für Alkyl (bevorzugt C₁-C₆-Alkyl), stehen,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521 und Herstellungsbeispiel).

Die Verbindungen der Formel (XLIII) 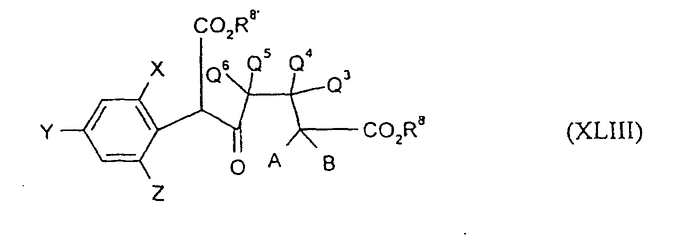 in welcher
- A, B, Q³, Q⁴, Q⁵, Q⁶, X, Y, Z, R⁸ und R^{8'}: die oben angegebene Bedeutung haben,
sind neu. Sie sind erhältlich,
wenn man Dicarbonsäureanhydride der Formel (XLIV), 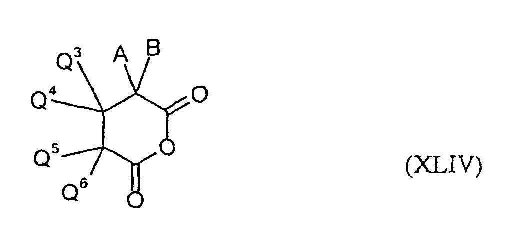 in welcher
- A, B, Q³, Q⁴, Q⁵ und Q⁶: die oben angegebene Bedeutung haben,
mit einem substituierten Phenylessigsäureester der Formel (XXXII) 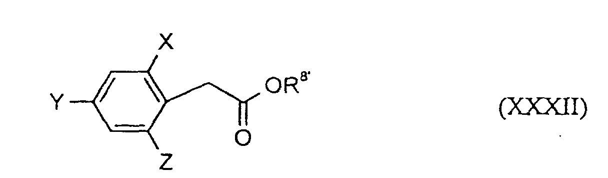 in welcher
- X, Y, Z und R^{8'}: die oben angegebene Bedeutung haben,
in Gegenwart eines Verdünnungsmittels und in Gegenwart einer Base kondensiert.

Die Verbindungen der Formel (XLIV) sind teilweise bekannt und/oder lassen sich nach bekannten Verfahren darstellen.

Die Verbindungen der Formel (XXXII) wurden bereits bei den Vorstufen für das Verfahren (B) beschrieben. Weiterhin erhält man Verbindungen der Formel (XXXII), indem man substituierte 1,1,1-Trichlor-2-phenylethane der Formel (XLV) 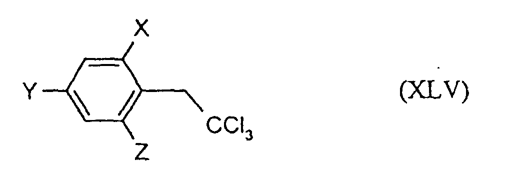 in welcher
- X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE 3 314 249).

Die Verbindungen der Formel (XLV) sind neu, sie lassen sich nach im Prinzip bekannten Verfahren herstellen.

Man erhält die Verbindungen der Formel (XLV) beispielsweise, wenn man Aniline der Formel (XLVI) 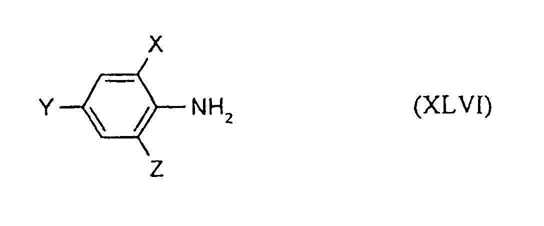 in welcher
- X, Y und Z: die oben angegebene Bedeutung haben,
in Gegenwart eines Alkylnitris der Formel (XLVII)

R²¹-ONO (XLVII)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XLVII) sind bekannte Verbindungen der Organischen chemie. Kupfer(II)-chlorid und Vinylidenchlorid sind lange bekannt und käuflich.

Die Verbindungen der Formel (XLVI) sind neu.

Man erhält beispielsweise Verbindungen der Formel (XLVI) 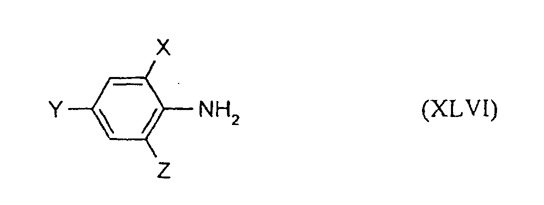 in welcher
- X, Y und Z: die oben angegebene Bedeutung haben,
wenn man Aniline der Formel (XLVI-a) 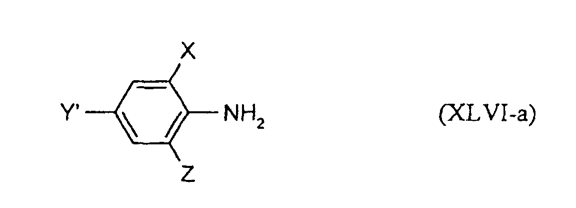 in welcher
- X und Z: die oben angegebene Bedeutung haben,
- Y': für Halogen (bevorzugt für Brom) steht
mit Boronsäuren der Formel (XII) 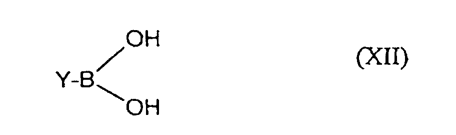 in welcher
- Y: die oben angegebene Bedeutung hat,
in Gegenwart eines Lösungsmittels, einer Base und eines Katalysators (bevorzugt eines Palladiumkomplexes, wie z.B. Palladium-tetrakis(triphenylphosphin) umsetzt.

Die bei dem obigen Verfahren (I) als Ausgangsstoffe benötigten Verbindungen der Formeln (I-1'a) bis (1-8'-a), in welchen A, B, D, Q¹, Q², Q³, Q⁴, Q⁵, Q⁶, X und Z die oben angegebene Bedeutung haben und Y' für Chlor und Brom, bevorzugt für Brom steht, sind teilweise bekannt (WO 96/35 664, WO 97/02 243) oder lassen sich gemäß den dort beschriebenen Verfahren oder nach Verfahren (A) bis (H) herstellen.

Die Boronsäuren der Formel (XII) 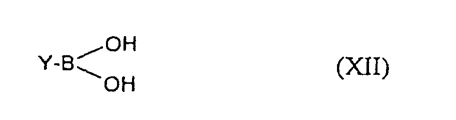 in welcher
- Y: die oben angegebene Bedeutung hat,
sind teilweise käuflich oder lassen sich nach allgemein bekannten Verfahren in einfacher Weise herstellen.

Die zur Durchführung der erfindungsgemäßen Verfahren (J), (K), (L), (M), (N), (O) und (P) außerdem als Ausgangsstoffe benötigten Säurehalogenide der Formel (XIII), Carbonsäureanhydride der Formel (XIV), Chlorameisensäureester oder Chlorameisensäurethioester der Formel (XV), Chlormonothioameisensäureester oder Chlordithioameisensäureester der Formel (XVI), Sulfonsäurechloride der Formel (XVII), Phosphorverbindungen der Formel (XVIII) und Metallhydroxide, Metallalkoxide oder Amine der Formel (XIX) und (XX) und Isocyanate der Formel (XXI) und Carbamidsäurechloride der Formel (XXII) sind allgemein bekannte Verbindungen der Organischen bzw. Anorganischen Chemie.

Die Verbindungen der Formeln (VII), (VIII), (IX), (XIII) bis (XXIII), (XXVI), (XXVIII), (XXX), (XXXVI), (XL), (XLI) und (XLVI-a) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, D, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß Verbindungen der Formel (III), in welcher A, B, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Die Verfahren (D-α) und (D-β) sind dadurch gekennzeichnet, daß man Verbindungen der Formeln (V) oder (VI), in welchen X, Y, Z, R⁸ und Hal die oben angegebenen Bedeutungen haben mit Verbindungen der Formel (VII), in welcher A und D die oben angegebenen Bedeutungen haben, gegebenenfalls in Gegenwart einer Base und gegebenenfalls in Gegenwart eines Verdünnungsmittels umsetzt.

Als Verdünnungsmittel können bei den erfindungsgemäßen Verfahren (D-α) und (D-β) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie, nur im Fall, daß Verbindungen der Formel (VI) eingesetzt werden, Alkohole wie Methanol, Ethanol, Propanol, Iso-Propanol, Butanol, Iso-Butanol und tert.-Butanol.

Als Base kommen in dem Fall, daß Verbindungen der Formel (V) eingesetzt werden, anorganische Basen, insbesondere Alkali- oder Erdalkalicarbonate wie Natriumcarbonat, Kaliumcarbonat oder Calciumcarbonat sowie organische Basen wie beispielsweise Pyridin oder Triethylamin in Betracht und in dem Fall, daß Verbindungen der Formel (VI) eingesetzt werden, Alkalimetall- und Erdalkalimetalloxide, -hydroxide und -carbonate, wie Natriumhydroxid, Kaliumhydroxid, Magnesiumhydroxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)-ammoniumchlorid) oder TDA 1 (= Tris-(methoxyethoxyethyl)-amin) in Betracht, ferner Alkalimetalle wie Natrium oder Kalium, Alkalimmetall- und Erdalkalimetallamide und -hydride, wie Natriumamid, Natriumhydrid und Calciumhydrid, und außerdem auch Alkalimetallalkoholate, wie Natriummethylat und Kalium-tert.-butylat.

Die Reaktionstemperaturen können bei der Durchführung des erfindungsgemäßen Verfahrens (D-α) und (D-β) innerhalb eines größeren Bereiches variiert werden. Im allgemeinen arbeitet man bei Temperaturen zwischen -20°C und 250°C, vorzugsweise zwischen 0°C und 150°C.

Die erfindungsgemäßen Verfahren (D-α) und (D-β) werden im allgemeinen unter Normaldruck durchgeführt.

Bei der Durchführung der erfindungsgemäßen Verfahren (D-α) und (D-β) setzt man die Reaktionskomponenten der Formeln (V) und (VII) oder (VI) und (VII) und die gegebenenfalls eingesetzte deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das erfindungsgemäße Verfahren (E) ist dadurch gekennzeichnet, daß man Carbonylverbindungen der Formel (VIII) oder deren Enolether der Formel (VIII-a) mit Ketensäurehalogeniden der Formel (V) in Gegenwart eines Verdünnungsmittels und gegebenenfalls in Gegenwart eines Säureakzeptors umsetzt.

Als Verdünnungsmittel können bei dem erfindungsgemäßen Verfahren (E) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante E) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VIII) und (V), in welchen A, D, X, Y und Z die oben angegebenen Bedeutungen haben und Hal für Halogen steht, und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (F) ist dadurch gekennzeichnet, daß man Thioamide der Formel (IX) mit Ketensäurehalogeniden der Formel (V) in Gegenwart eines Verdünnungsmittels und gegebenenfalls in Gegenwart eines Säureakzeptors umsetzt.

Als Verdünnungsmittel können bei der erfindungsgemäßen Verfahrensvariante F) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (F) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (F) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (F) wird zweckmäßigerweise unter Normaldruck durchgeführt.

Bei der Durchführung des erfindungsgemäßen Verfahrens (F) setzt man die Reaktionskomponenten der Formeln (IX) und (V), in welchen A, X, Y und Z die oben angegebenen Bedeutungen haben und Hal für Halogen steht und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (G) ist dadurch gekennzeichnet, daß man Verbindungen der Formel (X), inw elcher A, B, Q¹, Q², X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (G) alle gegenüber den Reaktionsteilnehmem inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanot, tert.-Butanol eingesetzt werden.

Als Basen (Deprotonierungsmittel) können bei der Durchführung des erfindungsgemäßen Verfahrens (G) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natriummethylat, Natrium-ethylat und Kalium-tert.-butylat einsetzbar.

Die Reaktionstemperaturen können bei der Durchführung des erfindungsgemäßen Verfahrens (G) innerhalb eines größeren Bereiches variiert werden. Im allgemeinen arbeitet man bei Temperaturen zwischen -75°C und 250°C, vorzugsweise zwischen -50°C und 150°C.

Das erfindungsgemäße Verfahren (G) wird im allgemeinen unter Normaldruck durchgeführt.

Bei der Durchführung des erfindungsgemäßen Verfahrens (G) setzt man die Reaktionskomponenten der Formel (X) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (H) ist dadurch gekennzeichnet, daß man Verbindungen der Formel (XI), in welcher A, B, Q³, Q⁴, Q⁵, Q⁶, X, Y, Z und R⁸ die oben angegebene Bedeutung haben, in Gegenwart von Basen einer intramolekularen Kondensation unterwirft.

Als Verdünnungsmittel können bei dem erfindungsgemäßen Verfahren (H) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (H) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (H) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (H) wird im allgemeinen unter Normaldruck durchgeführt.

Bei der Durchführung des erfindungsgemäßen Verfahrens (H) setzt man die Reaktionskomponenten der Formel (XII) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Zur Durchführung des erfindungsgemäßen Verfahrens (I) sind Palladium(0)-Komplexe als Katalysator geeignet. Eingesetzt wird beispielsweise Tetrakis-(triphenylphosphin)palladium. Es eignen sich auch Palladium(II)-Verbindungen wie Bis(triphenylphosphin)palladium(II)chlorid.

Als Säureakzeptoren zur Durchführung des erfindungsgemäßen Verfahrens (I) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Kaliumfluorid, Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (I) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Dicalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylenglykolmonomethylether; Wasser.

Die Reaktionstemperatur kann bei dem erfindungsgemäßen Verfahren (I) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (I) werden Boronsäure der Formel (XII) und Verbindungen der Formeln (I-1-a) bis (1-8-a) im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-1-a) bis (I-8-a) ein. Die Base setzt man im allgemeinen in einem Überschuß ein.

Das Verfahren (J-α) ist dadurch gekennzeichnet, daß man Verbindungen der Formeln (I-1-a) bis (I-8-a) jeweils mit Carbonsäurehalogeniden der Formel (XIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (J-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgerührt werden.

Als Säurebindemittel kommen bei der Umsetzung nach dem erfindungsgemäßen Verfahren (J-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (J-α) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (J-α) werden die Ausgangsstoffe der Formeln (I-1-a) bis (I-8-a) und das Carbonsäurehalogenid der Formel (XIII) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (J-β) ist dadurch gekennzeichnet, daß man Verbindungen der Formeln (I-1-a) bis (I-8-a) mit Carbonsäureanhydriden der Formel (XIV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (J-β) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (J-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (J-β) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (J-β) werden die Ausgangsstoffe der Formeln (I-1-a) bis (I-8-a) und das Carbonsäureanhydrid der Formel (XIV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (K) ist dadurch gekennzeichnet, daß man Verbindungen der Formeln (I-1-a) bis (I-8-a) jeweils mit Chlorameisensäureestern oder Chlorameisensäurethiolestern der Formel (XV) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (K) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (K) alle gegenüber den Chlorameisensäureestern bzw. Chlorameisensäurethiolestem inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (K) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (K) wird im allgemeinen unter Normaldruck durchgeführt.

Bei der Durchführung des erfindungsgemäßen Verfahrens (K) werden die Ausgangsstoffe der Formeln (I-1-a) bis (I-8-a) und der entsprechende Chlorameisensäureester bzw. Chlorameisensäurethiolester der Formel (XV) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (L) ist dadurch gekennzeichnet, daß man Verbindungen der Formeln (I-1-a) bis (I-8-a) jeweils mit Verbindungen der Formel (XVI) in Gegenwart eines Verdünnungsmittels und gegebenenfalls in Gegenwart eines Säurebindemittels umsetzt.

Beim Herstellungsverfahren (L) setzt man pro Mol Ausgangsverbindung der Formeln (I-1-a) bis (I-8-a) ca. 1 Mol Chlormonothioameisensäureester bzw. Chlordithioameisensäureester der Formel (XVI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (M) ist dadurch gekennzeichnet, daß man Verbindungen der Formeln (I-1-a) bis (I-8-a) jeweils mit Sulfonsäurechloriden der Formel (XVII) gegebenenfalls in Gegenwart eines Verdünnungsmittels und gegebenenfalls in Gegenwart eines Säurebindemittels umsetzt.

Beim Herstellungsverfahren (M) setzt man pro Mol Ausgangsverbindung der Formel (I-1-a bis I-8-a) ca. 1 Mol Sulfonsäurechlorid der Formel (XVII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (N) ist dadurch gekennzeichnet, daß man Verbindungen der Formeln (I-1-a) bis (I-8-a) jeweils mit Phosphorverbindungen der Formel (XVIII) gegebenenfalls in Gegenwart eines Verdünnungsmittels und gegebenenfalls in Gegenwart eines Säurebindemittels umsetzt.

Beim Herstellungsverfahren (N) setzt man zum Erhalt von Verbindungen der Formeln (I-1-e) bis (I-8-e) auf 1 Mol der Verbindungen (I-1-a) bis (I-8-a), 1 bis 2, vorzugsweise 1 bis 1,3 Mol der Phosphorverbindung der Formel (XVIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (O) ist dadurch gekennzeichnet, daß man Verbindungen der Formeln (I-1-a) bis (I-8-a) mit Metallhydroxiden bzw. Metallalkoxiden der Formel (XIX) oder Aminen der Formel (XX), gegebenenfalls in Gegenwart eines Verdünnungsmittels, umsetzt.

Als Verdünnungsmittel können bei dem erfindungsgemäßen Verfahren (O) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (O) wird im allgemeinen unter Normaldruck durchgeführt.

Die Reaktionstemperaturen liegen im allgemeinen zwischen -20°C und 100°C, vorzugsweise zwischen 0°C und 50°C.

Das erfindungsgemäße Verfahren (P) ist dadurch gekennzeichnet, daß man Verbindungen der Formeln (I-1-a) bis (I-8-a) jeweils mit (P-α) Verbindungen der Formel (XXI) gegebenenfalls in Gegenwart eines Verdünnungsmittels und gegebenenfalls in Gegenwart eines Katalysators oder (P-β) mit Verbindungen der Formel (XXII) gegebenenfalls in Gegenwart eines Verdünnungsmittels und gegebenenfalls in Gegenwart eines Säurebindemittels umsetzt.

Bei Herstellungsverfahren (P-α) setzt man pro Mol Ausgangsverbindung der Formeln (I-1-a) bis (I-8-a) ca. 1 Mol Isocyanat der Formel (XXI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (P-β) setzt man pro Mol Ausgangsverbindung der Formeln (I-1-a) bis (I-8-a) ca. 1 Mol Carbamidsäurechlorid der Formel (XXII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (1-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp..

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Omithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Wirkstoffe zeichnen sich durch eine hohe insektizide und akarizide Wirksamkeit nach Blatt- und Bodenanwendung aus.

Sie lassen sich mit besonders gutem Erfolg zur Bekämpfung von pflanzenschädigenden Insekten einzetzen, wie beispielsweise gegen die Larven des Meerrettichblattkäfers (Phaedon cochleariae), gegen die Larven der grünen Reiszikade (Nephotettix cincticeps) und gegen die Larven der grünen Pfirsichblattlaus (Myzus persicae).

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-FettsäureEster, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarböxim, Oxyfenthiin,
   Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-a-(Methoxyimino)-N-methyl-2-phenoxy-phenylacetamid,
   {2-Methyl-1-[[[1-(4-methylphenyl)-ethyl]-amino]-carbonyl]-propyl}-carbaminsäure-1-isopropylester
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropancarboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin-Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Isazofos, Isofenphos, Isoxathion, Ivermectin,
   Kempolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Mevinphos, Milbemectin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propoxur, Prothiofos, Prothoat, Pymetrozine, Pyraclofos, Pyresmethrin, Pyrethrum, Pyridaben, Pyridathion, Pycimidifen, Pyriproxyfen,
   Quinalphos,
   Ribavirin
   Salithion, Sebufos, Silafluofen, Spinosad, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypermethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
   YI 5302
   Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon
   Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera, Aegilops, Phalaris.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondem erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Wiedeflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlomitrofen, Chlorsulfuron, Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Omithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Beispiel

| **Myzus-Test** | |
|---|---|
| Lösungsmittel | 1 Gewichtsteil Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigt z.B. die folgende Verbindung der Herstellungsbeispiele gute Wirksamkeit:

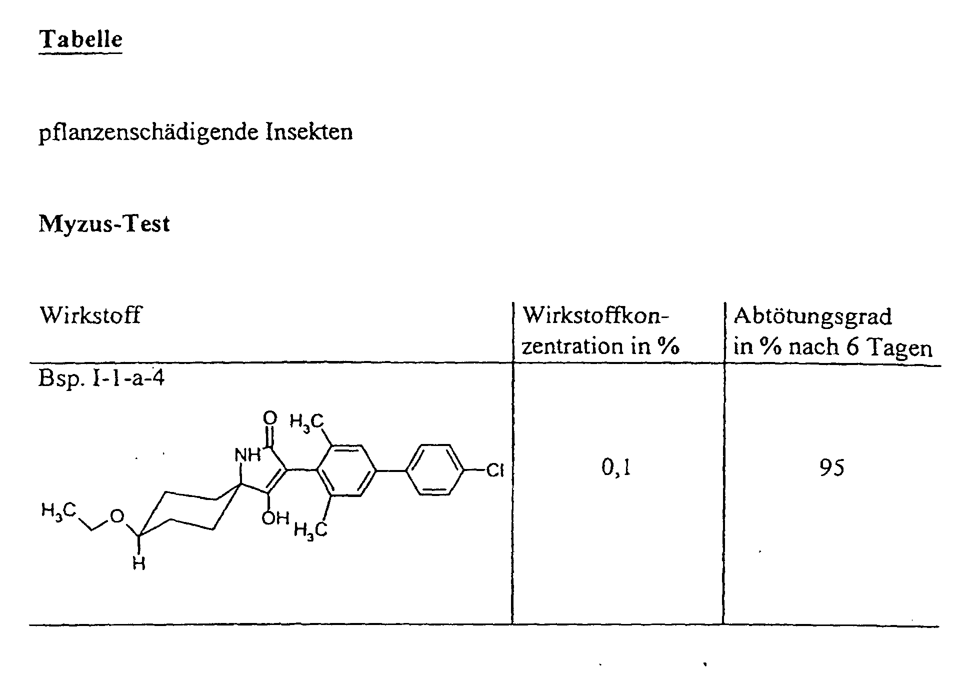

### Beispiel

| **Nephotettix-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Blätter noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test zeigen z.B. die folgenden Verbindungen der Herstellungsbeispiele gute Wirksamkeit:

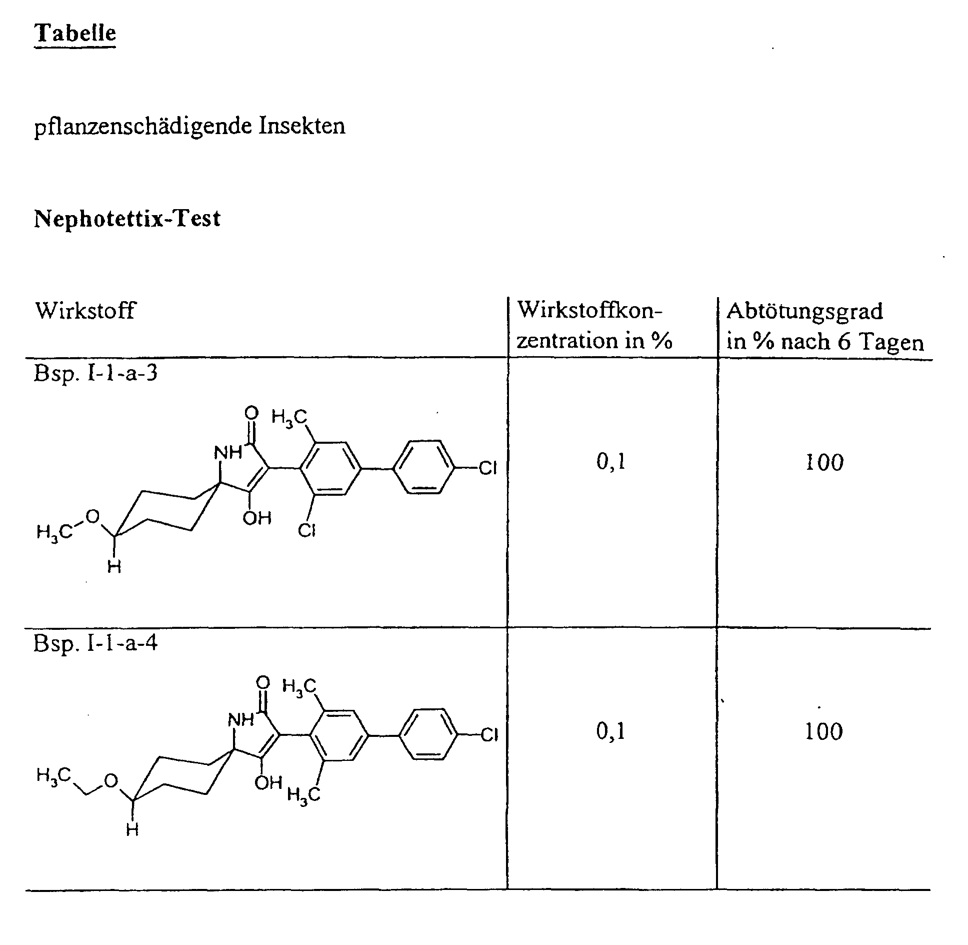

### Beispiel

| **Phaedon-Larven-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test zeigen z.B. die folgenden Verbindungen der Herstellungsbeispiele gute Wirksamkeit:

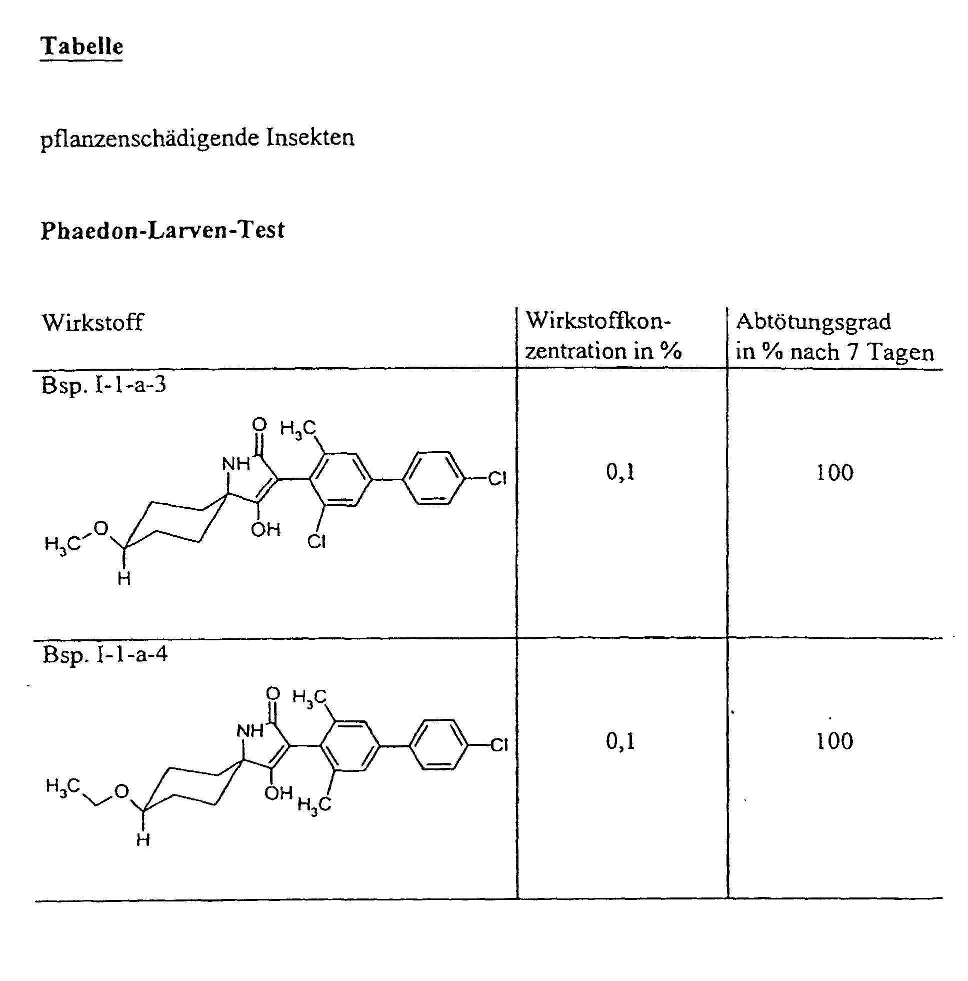

### Beispiel

| **Post-emergence-Test** | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so, daß die jeweils gewünschten Wirkstoffmengen pro Flächeneinheit ausgebracht werden. Nach 3 Wochen wird der Schädigungsgrad der Pflanzen bonitiiert in 5 Schädigung im Vergleich zur Entwicklung der unbehandelten Kontrolle.

Es bedeuten:
- 0% =: keine Wirkung (wie unbehandelte Kontrolle)
- 100% =: totale Vernichtung

| **Post-emergence / Gewächshaus** | | | | | |
|---|---|---|---|---|---|
| | g ai./ha | Zuckerrüben | Avena fatua | Setaria | Sinapis |
| Bsp. I-1-a-2 | 250 | 20 | 70 | 100 | 70 |
| | | | | | |

| | g ai./ha | Avena fatua | Setaria | Abutilon | Amaranthus |
|---|---|---|---|---|---|
| Bsp. I-1-a-4 | 250 | 90 | 100 | 80 | 80 |

### Beispiel

| **Pre-emergence-Test** | | |
|---|---|---|
| Lösungsmittel | 5 Gewichtsteile | Aceton |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiiert in % Schädigung im Vergleich zur Entwicklung der unbehandelten Kontrolle.

Es bedeuten:
- 0% =: keine Wirkung (wie unbehandelte Kontrolle)
- 100% =: totale Vernichtung

| **Pre-emergence / Gewächshaus** | | | | | |
|---|---|---|---|---|---|
| | g ai./ha | Alopecurus | Setaria | Amaranthus | Galium |
| Bsp. I-1-a-3 | 250 | 100 | 100 | 100 | 90 |

### Beispiel I-1-a-1

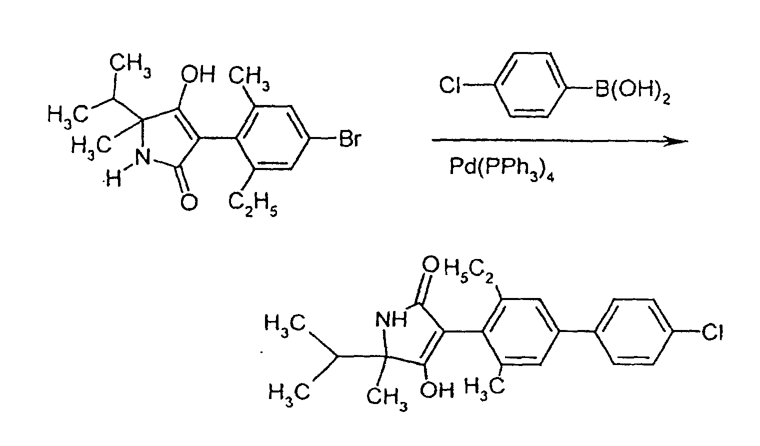

Unter Argon versetzt man 1,1 g 3-[(4-Brom-2-ethyl-6-methyl)-phenyl]-5-isopropyl-5-methyl-pyrrolidin-2,4-dion gemäß Beispiel I-1-a-4 aus WO 97/02243 in 20 ml 1,2-Dimethoxyethan mit 0,6 g 4-Chlorphenylboronsäure und 180 mg Tetrakis(triphenylphosphin)palladium. Man rührt 15 Min. bei 20°C, gibt dann 15 ml 20 %ige wäßrige Natriumcarbonatlösung zu und rührt einen Tag bei 80°C. Dann wird filtriert, das Filtrat mit Wasser versetzt und die wässrige Phase angesäuert. Man saugt ab und erhält 0,75 g Produkt (65 % der Theorie).
Fp.: 245°C

In Analogie zu Beispiel (I-1-a-1) und (I-1-a-43) bzw. gemäß den allgemeinen Angaben zur Herstellung von Verbindungen der Formel (I-1-a) erhält man folgende Verbindungen der Formel (I-1-a)

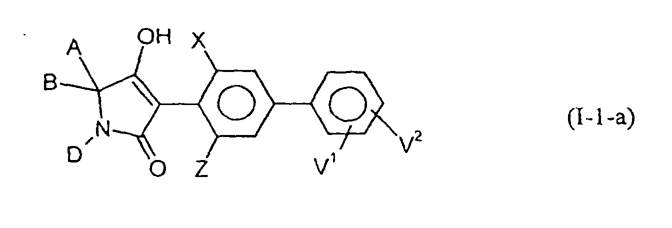

| Bsp.- Nr. | X | Z | V¹ | V² | D | A | B | Fp.°C | Isomer |
|---|---|---|---|---|---|---|---|---|---|
| I-1-a-2 | CH₃ | CH₃ | 4-Cl | H | H | -(CH₂)₂-CHOCH₃-(CH₂)₂- | | > 240 | β |
| I-1-a-3 | CH₃ | Cl | 4-Cl | H | H | -(CH₂)₂-CHOCH₃-(CH₂)₂- | | 211 | β |
| I-1-a-4 | CH₃ | Cl | 4-Cl | H | H | -(CH₂)₂-CHOC₂H₅-(CH₂)₂- | | >230 | β |
| I-1-a-5 | CH₃ | CH₃ | 4-Cl | H | H | -CH₂-O-(CH₂)₃- | | >240 | - |
| I-1-a-6 | C₂H₅ | CH₃ | 4-Cl | H | H | CH₃ | CH₃ | >240 | - |
| I-1-a-7 | C₂H₅ | C₂H₅ | 4-Cl | H | H | -(CH₂)₂-CHCH₃-(CH₂)₂- | | 188 | β |
| I-1-a-8 | CH₃ | Cl | 4-Cl | H | H | -(CH₂)₂-O-(CH₂)₂- | | 165-167 | - |
| I-1-a-9 | C₂H₅ | Cl | 4-Cl | H | H | -(CH₂)₂-CHOCH₃-(CH₂)₂- | | >240 | β |
| I-1-a-10 | C₂H₅ | Cl | 4-Cl | H | H | -(CH₂)₂-CHCH₃-(CH₂)₂- | | >240 | β |
| I-1-a-11 | CH₃ | Cl | 4-Cl | H | H | i-C₃H₇ | CH₃ | >240 | - |
| I-1-a-12 | CH₃ | CH₃ | 4-Cl | H | H | CH₃ | CH₃ | >240 | - |
| I-1-a-13 | C₂H₅ | C₂H₅ | 4-Cl | H | H | i-C₃H₇ | CH₃ | 218 | - |
| I-1-a-14 | CH₃ | CH₃ | 4-Cl | H | H | -(CH₂)₅- | | >240 | - |
| I-1-a-15 | CH₃ | CH₃ | 3-Cl | H | H | -(CH₂)₂-CHOCH₃-(CH₂)₂- | | 135 | β |
| I-1-a-16 | CH₃ | CH₃ | 2-Cl | H | H | -(CH₂)₂CHOCH₃(CH₂)₂- | | >245 | β |
| I-1-a-17 | CH₃ | CH₃ | 4-Cl | H | H | -(CH₂)₂-O-(CH₂)₂- | | >235 | - |
| I-1-a-18 | CH₃ | CH₃ | 4-Cl | H | H | -(CH₂)₂CHOC₂H₅(CH₂)₂- | | >245 | β |
| I-1-a-19 | CH₃ | CH₃ | 4-Cl | H | H | -(CH₂)₂-CHCH₃-(CH₂)₂- | | >245 | β |
| I-1-a-20 | CH₃ | CH₃ | 4-Cl | H | H | -CH₂-CHCH₃-(CH₂)₃- | | >245 | β |
| I-1-a-21 | CH₃ | CH₃ | 4-Cl | H | -(CH₂)₄- | | H | >240 | - |
| I-1-a-22 | CH₃ | CH₃ | 4-Cl | H | i-C₃H₇ | H | H | >249 | - |
| I-1-a-23 | CH₃ | CH₃ | 2-CH₃ | 5-CH₃ | H | -CH₂-O-(CH₂)₃- | | 148 | - |
| I-1-a-24 | CH₃ | CH₃ | 2-CH₃ | 6-CH₃ | H | -CH₂-O-(CH₂)₃- | | >250 | - |
| I-1-a-25 | CH₃ | CH₃ | 3-CH₃ | 5-CH₃ | H | -CH₂-O-(CH₂)₃- | | >250 | - |
| I-1-a-26 | CH₃ | Cl | 4-Cl | H | H | -(CH₂)₅- | | >250 | - |
| I-1-a-27 | CH₃ | Cl | 3-CF₃ | 5-CF₃ | H | -(CH₂)₅- | | >250 | - |
| I-1-a-28 | CH₃ | Cl | 4-CF₃ | H | H | -(CH₂)₅- | | >250 | - |
| I-1-a-29 | CH₃ | Cl | 2-Cl | 4-Cl | H | -(CH₂)₅- | | >250 | - |
| I-1-a-30 | CH₃ | Cl | 3-Cl | 5-Cl | H | -(CH₂)₅- | | >250 | - |
| I-1-a-31 | C₂H₅ | C₂H₅ | 4-Cl | H | H | -(CH₂)₂-CHCH₃-(CH₂)₂- | | >250 | - |
| I-1-a-32 | C₂H₅ | C₂H₅ | 3-CF₃ | 5-CF₃ | H | -(CH₂)₂CHCH₃-(CH₂)₂- | | 242-244 | β |
| I-1-a-33 | C₂H₅ | C₂H₅ | 4-CF₃ | H | H | -(CH₂)₂CHCH₃-(CH₂)₂- | | >250 | β |
| I-1-a-34 | CH₃ | CH₃ | 4-Cl | H | H | i-C₃H₇ | CH₃ | Wachs | - |
| I-1-a-35 | C₂H₅ | C₂h₅ | 2-Cl | 4-Cl | H | -(CH₂)₂CHCH₃-(CH₂)₂- | | >250 | β |
| I-1-a-36 | C₂H₅ | C₂H₅ | 3-Cl | 5-Cl | H | -(CH₂)₂CHCH₃-(CH₂)₂- | | >250 | β |
| I-1-a-37 | CH₃ | CH₃ | 2-Cl | 4-Cl | H | i-C₃H₇ | CH₃ | 115-117 | - |
| I-1-a-38 | CH₃ | CH₃ | 3-Cl | 5-Cl | H | i-C₃H₇ | CH₃ | 233-234 | - |
| I-1-a-39 | C₂H₅ | Cl | 4-Cl | H | H | CH₃ | CH₃ | >250 | - |
| I-1-a-40 | C₂H₅ | Cl | 3-Cl | 5-Cl | H | CH₃ | CH₃ | 125-127 | - |
| I-1-a-41 | CH₃ | Cl | 4-Cl | H | H | CH₃ | CH₃ | >250 | - |
| I-1-a-42 | CH₃ | Cl | 3-CF₃ | 5-CF₃ | H | CH₃ | CH₃ | >250 | - |
| I-1-a-43 | CH₃ | CH₃ | 4-Cl | H | H | i-C₃H₇ | CH₃ | >250 | - |
| I-1-a-44 | CH₃ | CH₃ | 4-CH₃ | H | H | -(CH₂)₂-CHOCH₃-(CH₂)₂- | | >235 | β |
| I-1-a-45 | CH₃ | CH₃ | 2-CH₃ | 5-F | H | -(CH₂)₂-CHOCH₃-(CH₂)₂- | | >235 | β |
| I-1-a-46 | CH₃ | CH₃ | 2-CH₃ | 5-CH₃ | H | -(CH₂)₂-CHOCH₃-(CH₂)₂- | | 168 | β |
| I-1-a-47 | CH₃ | CH₃ | 2-OCH₃ | H | H | -(CH₂)₂-CHOCH₃-(CH₂)₂- | | | β |
| I-1-a-48 | CH₃ | CH₃ | 3-CH₃ | 5-CH₃ | H | -(CH₂)₂-CHOCH₃-(CH₂)₂- | | 238 | β |
| I-1-a-49 | CH₃ | CH₃ | 2-Cl | 4-Cl | H | -(CH₂)₂-CHOCH₃-(CH₂)₂- | | 188 | β |
| I-1-a-50 | CH₃ | CH₃ | 2-Cl | 3-Cl | H | -(CH₂)₂-CHOCH₃-(CH₂)₂- | | >235 | β |

### Beispiel I-1-b-1

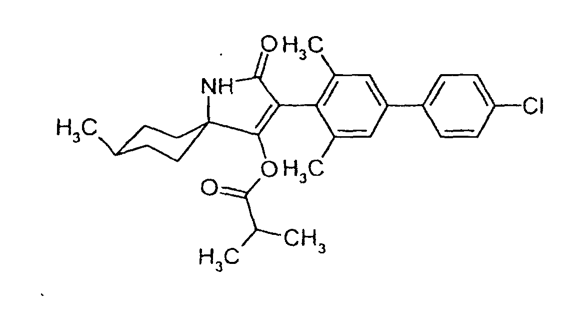

Zu 1,58 g der Verbindung gemäß Beispiel I-1-a-19 in 40 ml absolutem Essigsäureethylester gibt man zunächst 0,67 ml (4,8 mmol) Triethylamin und dann bei Rückflußtemperatur 0,5 ml (0,005 mol) Isobuttersäurechlorid in 5 ml absolutem Essigsäureethylester. Bei dieser Temperatur wird gerührt, bis die Reaktion beendet ist (dünnschichtchromatographische Kontrolle). Dann wird eingeengt, in Methylenchlorid aufgenommen, 2 mal mit 30 ml 0,5 N NaOH gewaschen, getrocknet und eingeengt. Der Rückstand wird aus Methyl-tert.-butylether (MTBE)/n-Hexan umkristallisiert. Ausbeute 1,27 g (68 % der Theorie). Fp.: >247°C.

In Analogie zu Beispiel (I-1-b-1) bzw. gemäß den allgemeinen Angaben zur Herstellung von Verbindungen der Formel (I-1-b) erhält man folgende Verbindungen der Formel (I-1-b)

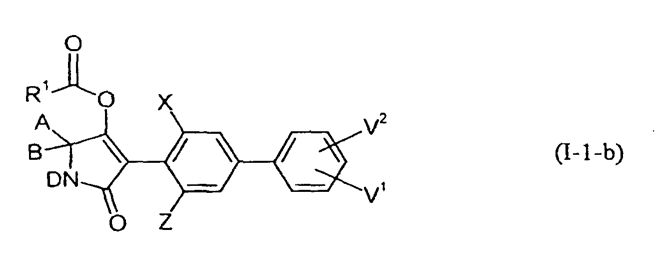

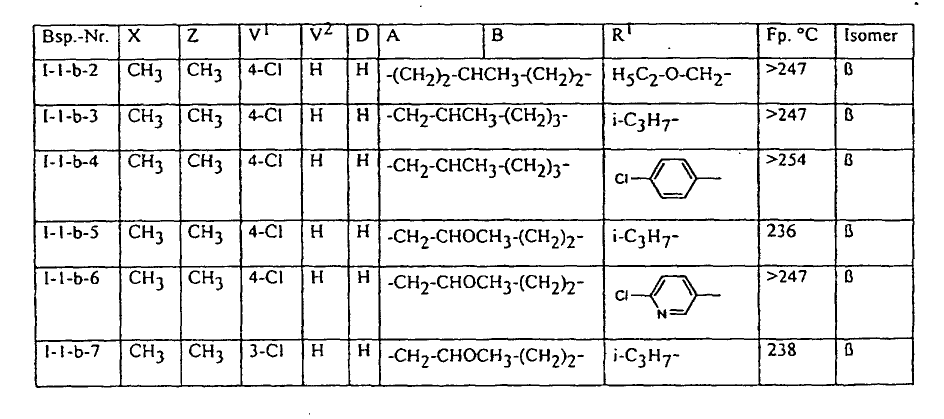

### Beispiel I-1-c-1

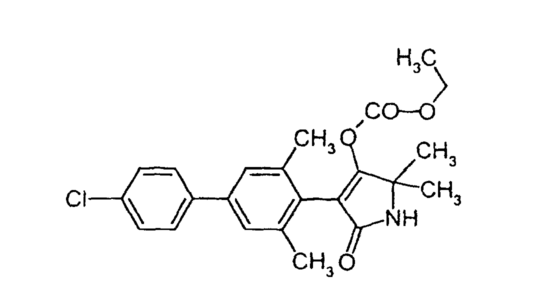

Zu 0,35 g der Verbindung gemäß Beispiel I-1-a-12 und 0,15 ml Triethylamin in 30 ml Methylenchlorid gibt man bei -10°C bis 0°C 0,15 g Chlorameisensäure in 5 ml Methylenchlorid und rührt noch 2 Stunden bei Raumtemperatur. Das Reaktionsgemisch wird über Kieselgel mit Methylenchlorid/Essigester 5:3 als Laufmittel filtriert, dann wird eingeengt, in wenig Methylenchlorid gelöst und das Produkt durch Zugabe von Hexan gefällt. Ausbeute 0,23 g (56 % der Theorie). Fp.: 129°C.

In Analogie zu Beispiel (I-1-c-1) bzw. gemäß den allgemeinen Angaben zur Herstellung von Verbindungen der Formel (I-1-c) erhält man folgende Verbindungen der Formel (I-1-c)

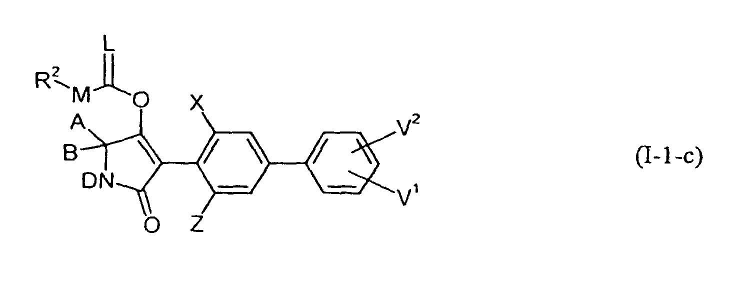

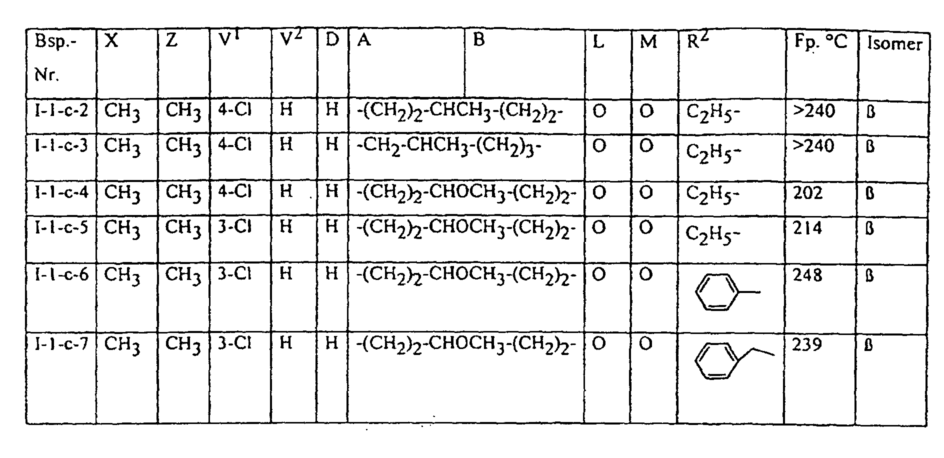

### Beispiel I-2-a-1

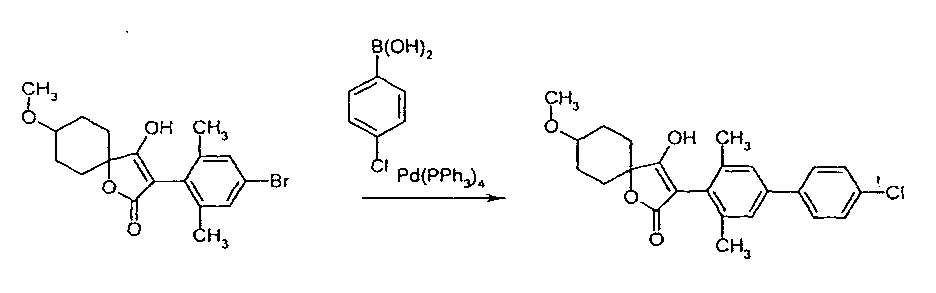

1,0 g (2,6 mmol) 3-[(2,6-Dimethyl-4-brom)-phenyl]-5,5-[(3-methoxy)-pentamethylen]-4-hydroxy-Δ³-dihydrofuran-2-on gemäß Beispiel I-2-a-15 aus WO 97/02243 werden in 20 ml Diethoxyethan suspendiert, 0,5 g (3,2 mmol) 4-Chlorphenylboronsäure und 180 mg (0,156 mmol) Tetrakis(triphenylphosphin)palladium zugegeben und 15 Min. bei Raumtemperatur gerührt, anschließend werden 13 ml 20 %ige Natriumcarbonatlösung zugesetzt und 24 h bei 80°C gerührt.

Zur Aufarbeitung wurde eingeengt, zwischen wäßriger Zitronensäure und Methylenchlorid verteilt, getrocknet und eingeengt. Zur weiteren Reinigung wurde das Rohprodukt zwischen 1N NaOH und Methylenchlorid verteilt, die wäßrige Phase angesäuert, das ausgefallene Produkt abgesaugt und getrocknet.
Ausbeute: 0,38 g (35 % d. Th.) kristalliner Feststoff
Fp.: 215-217°C

### Beispiel I-2-a-5

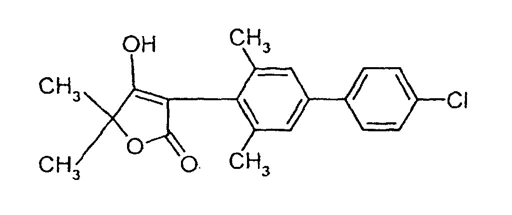

Zu 7,82 mmol der Verbindung gemäß Beispiel (III-1) in 10 ml DMF tropft man langsam 9,4 ml einer 1 M Lösung von Kalium-tert.-butylat in DMF und rührt über Nacht bei Raumtemperatur. Das Lösungsmittel wird abdestilliert, der Rückstand in Wasser aufgenommen und mit verdünnter Salzsäure angesäuert. Man rührt noch 2 Stnden und saugt ab.
Ausbeute: 2,78 g, Fp. 285-287°C.

Analog zu Beispiel (I-2-a-1) und (I-2-a-5) bzw. gemäß den allgemeinen Angaben zur Herstellung von Verbindungen der Formel (I-2-a) erhält man folgende Verbindungen der Formel (I-2-a)

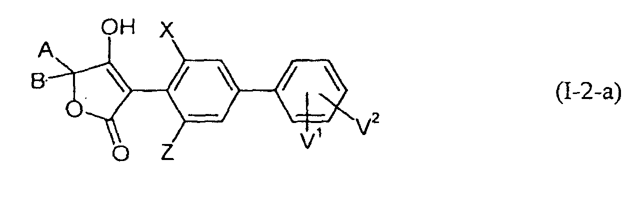

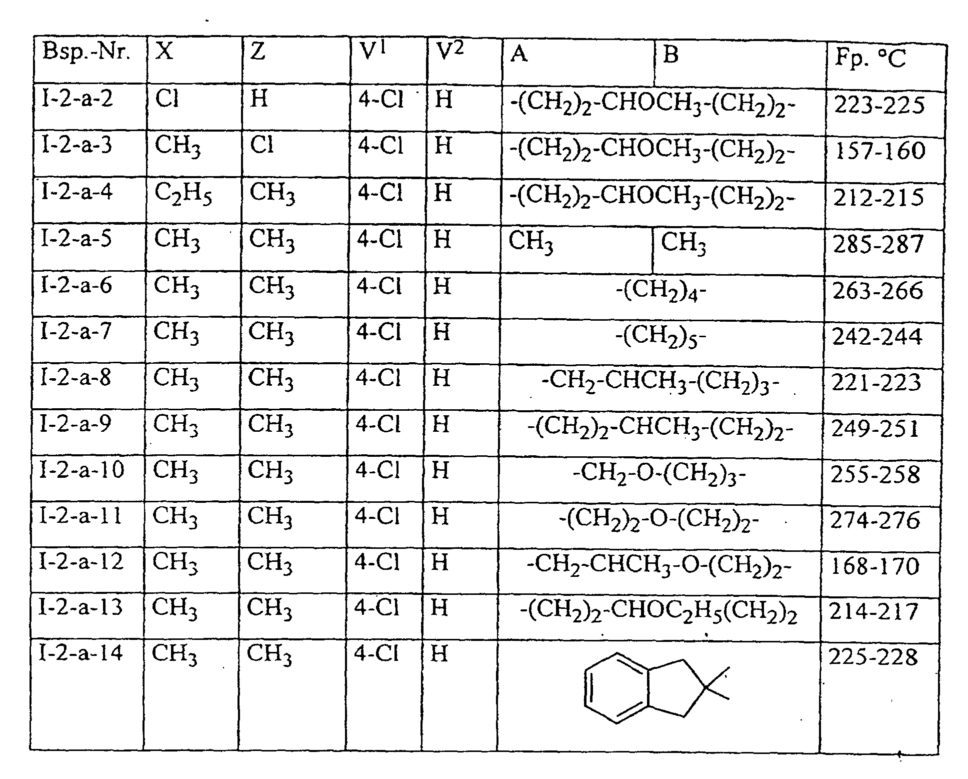

### Beispiel I-2-b-1

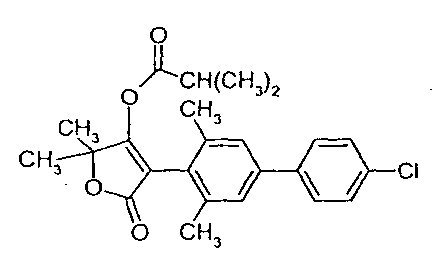

Zu 1,03 g der Verbindung gemäß Beispiel I-2-a-5 in 20 ml Methylenchlorid gibt man 0,32 g Triethylamin und dann 0,33 g Isobuttersäurechlorid. Man rührt über Nacht, schüttelt den Ansatz mit verdünnter wäßriger Citronensäure und 1 N NaOH, trocknet und engt die organische Phase ein. Ausbeute 1.16 g.

¹H-NMR (CDCl₃): 1.05 ppm (d, 6H); 1.55 ppm (s, 6H); 2,25 ppm (s, 6H); 2.65 ppm (m, 1H), 7.25-7.50 ppm (m, 6H).

Analog zu Beispiel (I-2-b-1) bzw. gemäß den allgemeinen Angaben zur Herstellung von Verbindungen der Formel (I-2-b) erhält man folgende Verbindungen der Formel (I-2-b)

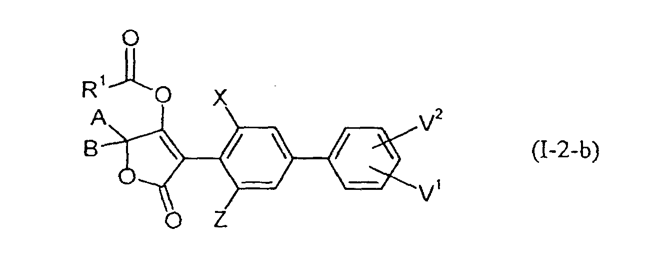

| Bsp.- Nr. | X | Z | V¹ | V² | A | B | R¹ | Fp. °C |
|---|---|---|---|---|---|---|---|---|
| I-2-b-2 | CH₃ | CH₃ | 4-Cl | H | -(CH₂)₄- | | i-C₃H₇- | Öl, |
| I-2-b-3 | CH₃ | CH₃ | 4-Cl | H | -(CH₂)₅- | | i-C₃H₇- | Öl |
| I-2-b-4 | CH₃ | CH₃ | 4-Cl | H | -CH₂-CHCH₃-(CH₂)₃- | | i-C₃H₇- | Öl |
| I-2-b-5 | CH₃ | CH₃ | 4-Cl | H | -(CH₂)₂-CHCH₃-(CH₂)₂- | | i-C₃H₇- | Öl |
| I-2-b-6 | CH₃ | CH₃ | 4-Cl | H | -CH₂-O-(CH₂)₃- | | i-C₃H₇- | Öl |
| I-2-b-7 | CH₃ | CH₃ | 4-Cl | H | -(CH₂)₂-O-(CH₂)₂- | | i-C₃H₇- | Öl |
| I-2-b-8 | CH₃ | CH₃ | 4-Cl | H | -CH₂-CHCH₃-O-(CH₂)₂- | | i-C₃H₇- | Öl |
| I-2-b-9 | CH₃ | CH₃ | 4-Cl | H | -(CH₂)₂-CHOC₂H₅-(CH₂)₂- | | i-C₃H₇- | Öl |

### Beispiel I-2-c-1

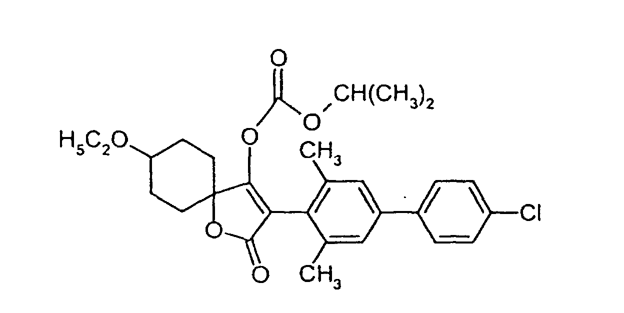

Analog zu Beispiel (I-2-b-1) erhält man die oben gezeigte Verbindung ausgehend von der Verbindung gemäß Beispiel (I-2-a-13) und Chlorameisensäureisopropylester.

### Beispiel I-3-a-1

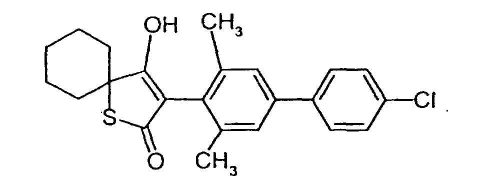

45,0 g (81 mmol) der Verbindung gemäß Beispiel (IV-1) werden in 91 ml Trifluoressigsäure und 210 ml Toluol über Nacht unter Rückfluß erhitzt. Nach dem Einengen wird der Rückstand in 200 ml MTBE und 600 ml Wasser aufgenommen und durch Zugabe von NaOH pH 14 eingestellt. Man tropft die organische Phase in 1 l 1N HCl, rührt 2 Stunden, saugt ab, wäscht mit Cyclohexan und trocknet. Ausbeute 18,3 g (57 % der Theorie). Fp.: >250°C.

### Beispiel I-3-b-1

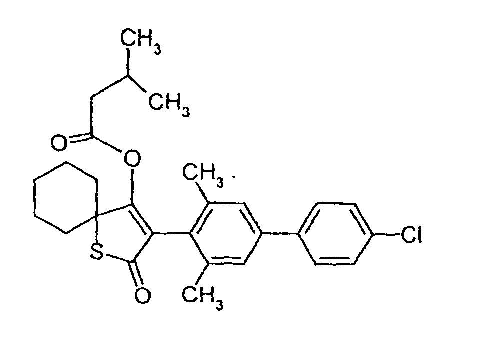

Zu 2,0 g der Verbindung gemäß Beispiel (I-3-a-1) und 1,04 ml Triethylamin in 15 ml absolutem Methylenchlorid tropft man unter Eiskühlung eine Lösung von 0,79 ml Isovaleriansäurechlorid in 3 ml absolutem Methylenchlorid und rührt noch 2 Stunden bei Raumtemperatur. Man wäscht mit 10 %iger wäßriger Citronensäure und extrahiert mit Methylenchlorid. Die organische Phase wird mit 1N NaOH gewaschen, getrocknet und eingeengt. Ausbeute 2,1 g (87 % der Theorie) eines Öls.

¹H-NMR (400 MHz, CDCl₃):δ = 0,9 (dd, 6 H, CH/CH₃)₂, 1,4 - 2,0 (m, 10H, Cyclohexyl) 2,15 (s, 6H, 2 x Ar-CH₃), 7,4 - 7,7 (m, 6H, Ar-H) ppm.

### Beispiel I-3-c-1

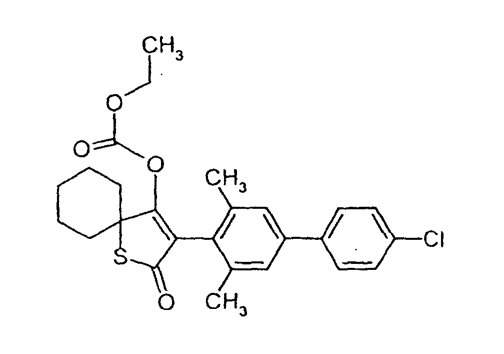

Analog zu Beispiel (I-3-b-1) erhält man die oben gezeigte Verbindung durch Umsetzung der Verbindung gemäß Beispiel (I-3-a-1) mit Chlorameisensäureethylester. Ausbeute 2,1 g (89 % der Theorie). Fp. 167 bis 170°C.

### Beispiel (I-5-a-1)

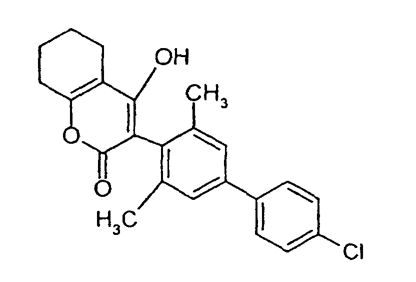

Zu 2,6 g der Verbindung gemäß Beispiel (I-5-a-1) aus WO 97/02 243 in 21 ml Dimethoxyethan und 18 ml 1N Na₂CO₃-Lösung werden 1,8 g 4-Chlorphenylboronsäure und 266,3 mg Bis(triphenylphosphin)palladium(II)chlorid gegeben. Man rührt über Nacht unter Rückfluß, säuert mit verdünnter HCl an und engt ein. Der Rückstand wird chromatographisch an Kieselgel gereinigt (Laufmittel Cyclohexan/Essigsäureethylester 4/1 → 2/1). Ausbeute 2,55 g (89 % der Theorie); Fp.: >250°C.

### Beispiel (I-5-a-2)

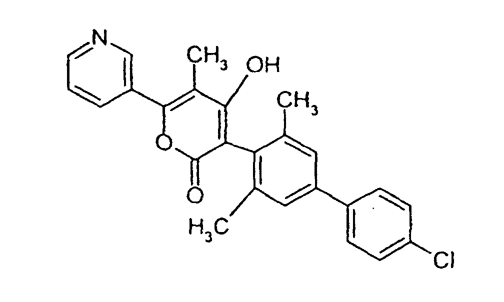

Analog zu Beispiel I-5-a-1 erhält man die oben gezeigte Verbindung ausgehend von der Verbindung gemäß Beispiel (I-5-a-6) aus WO 97/02 243. Fp. 107-108°C.

### Beispiel I-7-a-1

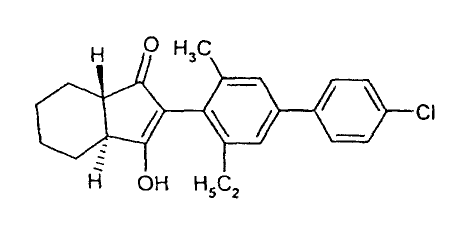

1,5 g (4,3 mmol) der Verbindung der Formel 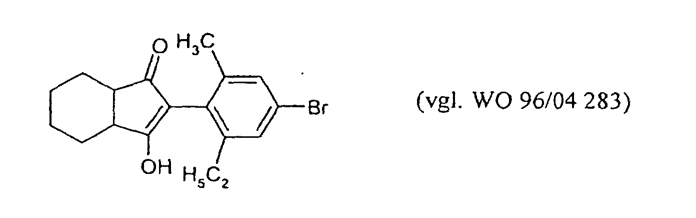 werden in 10 ml Dimethoxyethan gelöst und mit 10 ml 1 M Na₂CO₃-Lösung versetzt. Man gibt 1,0 g (6,45 mmol) p-Chlorphenylboronsäure und zuletzt als Katalysator 254 mg (0,22 mmol) Pd(PPh₃)₄ zu (Ph = Phenyl). Man erhitzt über Nacht unter Rückfluß, filtriert und wäscht mit Essigsäureethylester nach. Das Filtrat wird mit Wasser versetzt und je 3 mal mit Essigsäureethylester und Methyl-tert.-butylether (MTBE) extrahiert. Die vereinigten organischen Phasen werden mit Wasser und gesättigter NaCl-Lösung gewaschen, getrocknet und eingeengt.
Rohausbeute: 1,85 g.

Nach Säulenchromatographie an Kieselgel mit Cyclohexan/Essigsäureethylester 15/1 erhält man 100 mg der oben gezeigten Verbindung.

Man erhält zwei weitere Fraktionen (300 mg und 400 mg), Rotamere des cis-Isomeren.

### Beispiel I-7-a-2

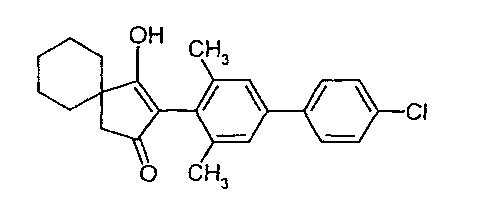

20,5 g der Verbindung gemäß Beispiel (X-1) in 50 ml absolutem DMF werden mit 8,44 g Kalium-tert.-butylat versetzt und 1 Stunde auf 80°C erwärmt. Die Mischung wird unter Eiskühlung langsam in 21 1N HCl gegeben. Man extrahiert mit Methylenchlorid, trocknet und engt ein. Ausbeute 18 g (95 % der Theorie), Fp.: 192-195°C.

Analog zu Beispiel (I-7-a-1) und (I-7-a-2) bzw. gemäß den allgemeinen Angaben zur Herstellung von Verbindungen der Formel (I-7-a) erhält man folgende Verbindungen der Formel (I-7-a):

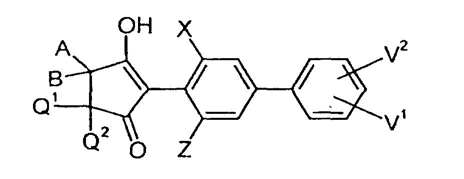

| Bsp.- Nr. | X | Z | V¹ | V² | B | A | Q¹ | Q² | Fp. °C |
|---|---|---|---|---|---|---|---|---|---|
| I-7-a-3 | CH₃ | CH₃ | 3-Cl | H | -(CH₂)₅ | | H | H | >250 |
| I-7-a-4 | CH₃ | CH₃ | 4-Cl | H | H | -(CH₂)₄- | | H | >250 |
| I-7-a-5 | CH₃ | CH₃ | 3-Cl | H | H | -(CH₂)₄- | | H | 211-213 |
| I-7-a-6 | CH₃ | CH₃ | 4-Cl | H | -(CH₂)₃-CHCH₃-CH₂- | | H | H | 243-244 |
| I-7-a-7 | CH₃ | CH₃ | 3-Cl | H | -(CH₂)₃-CHCH₃-CH₂- | | H | H | Wachs |

### Beispiel I-7-b-1

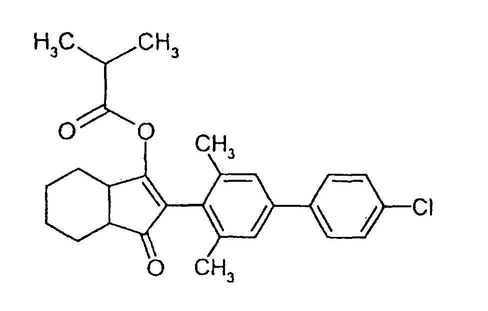

Analog zu Beispiel (I-3-b-1) erhält man die oben gezeigte Verbindung durch Umsetzung der Verbindung gemäß Beispiel (I-7-a-1) mit Isobuttersäurechlorid. Fp.: Öl.

¹H-NMR (400 MHz; DMSO):δ = 0,9-1,0 (d, 6H, HC-CH₃); 1,2-2,0 (m, 8H, Cyclohexyl-H); 2,1 2,15 (s, 6H, 2 x ArCH₃); 2,65 (m, 1H, CHCH₃); 7,4 (s, 2H, Ar-H); 7,5-7,7 (d. 4H, Ar-H) ppm.

### Beispiel 1-7-b-2

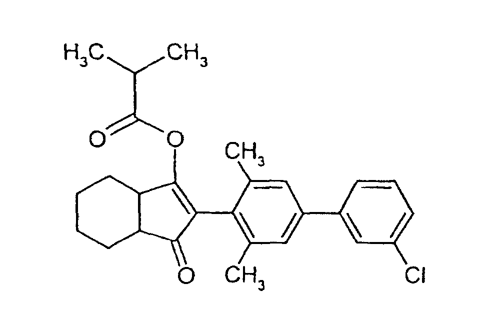

Analog zu Beispiel (I-3-b-1) erhält man die oben gezeigte Verbindung durch Umsetzung der Verbindung gemäß Beispiel (I-7-a-5) mit Isobuttersäurechlorid als Öl.

¹H-NMR (400 MHz; DMSO):δ = 1-1,1 (d. 6H, CH-CH₃), 42,0 (m, 8H, Cyclohexyl-H); 2,15, 2,2 (s. 6H, 2 x ArCH₃); 2,6 (m, 1 H, CH-CH₃); 2,9, 3,4 (m, 2H, Cyclohexyl C-H); 7,3-7,6 (m, 6H, Ar-H) ppm.

Analog zu Beispiel (I-7-b-1) bzw. gemäß den allgemeinen angaben zur Herstellung von Verbindungen der Formel (I-7-b) erhält man folgende Verbindungen der Formel (I-7-b)

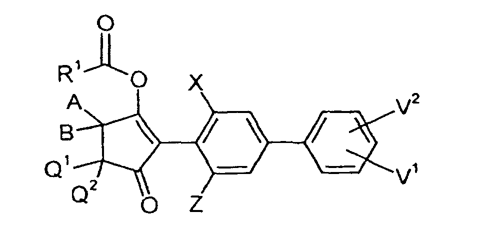

| Bsp.- Nr. | X | Z | V¹ | V² | B | A | Q¹ | Q² | R¹ | Fp. °C |
|---|---|---|---|---|---|---|---|---|---|---|
| I-7-b-3 | CH₃ | CH₃ | 4-Cl | H | -(CH₂)₅- | | H | H | i-C₄H₉ | Öl |

### Beispiel I-7-c-1

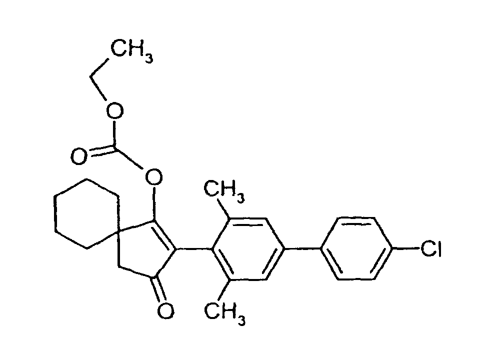

Analog zu Beispiel (I-3-b-1) erhält man die oben gezeigte Verbindung durch Umsetzung der Verbindung gemäß Beispiel (I-7-a-2) mit Chlorameisensäureethylester als Wachs.

### Beispiel I-8-a-1

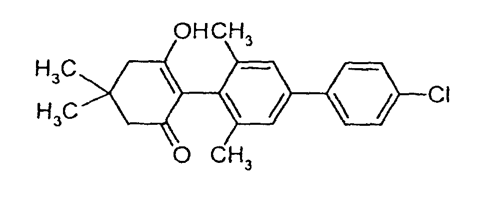

6,0 g der Verbindung gemäß Beispiel (XI-1) werden in 20 ml DMF vorgelegt, mit 2,63 g Kalium-tert.-butylat versetzt und 1 Stunde auf 80°C erwärmt. Dann gibt man langsam unter Eiskühlung in 1 l 1N HCl, saugt ab und trocknet. Ausbeute 5,15 g (93 % der Theorie). Fp.: 222-224°C.

### Beispiel I-8-a-2

Analog zu Beispiel (I-8-a-1) erhält man 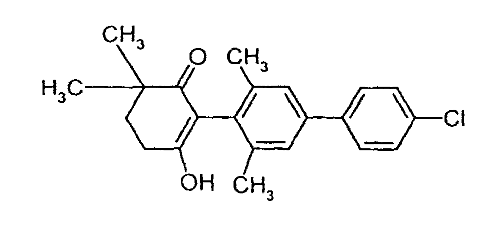 vom Fp. 117-122°C.

### Beispiel I-8-b-1

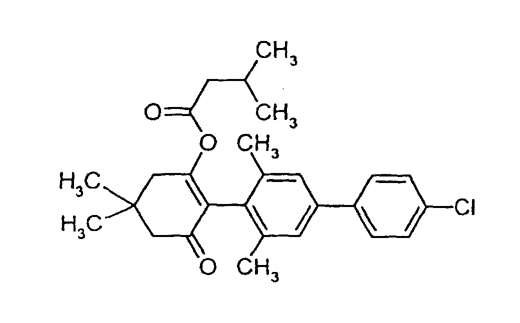

Analog zu Beispiel (I-3-b-1) erhält man die oben gezeigte Verbindung durch Umsetzung der Verbindung gemäß Beispiel (I-8-a-1) und Isovaleriansäurechlorid als Öl.

¹H-NMR (400 MHz; CDCl₃):δ = 0,55 (d, 6H, CH(CH₃)₂), 1,2 (s, 6H, C(CH₃)₂), 1,6 (m, 1H, CH(CH₃)₂), 2,1 (s, 6H, 2 x Ar-CH₃), 7,3-7,65 (m, 6H, Ar-H) ppm.

### Beispiel I-8-b-2

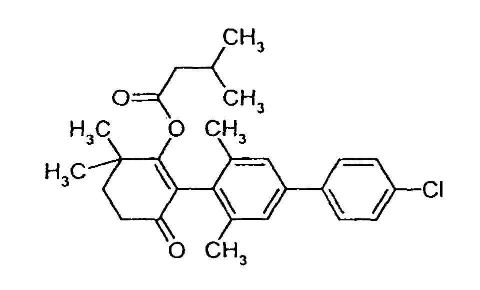

Analog zu Beispiel (I-3-b-1) erhält man die oben gezeigte Verbindung durch Umsetzung der Verbindung gemäß Beispiel (I-8-a-2) mit Isovaleriansäurechlorid als Öl.

¹H-NMR (400 MHz; CDCl₃):δ = 0,55 (d, 6H, CH(CH₃)₂), 1,2 (s, 6H, C(CH₃)₂), 1,6 (m, 1H, CH(CH₃)₂), 2,0:2,7 (t, 2x2H, CH₂-CH₂), 2,05 (s, 6H, 2 x Ar-CH₃), 7,3-7,65 (m, 6H, Ar-H) ppm.

### Beispiel I-8-c-1

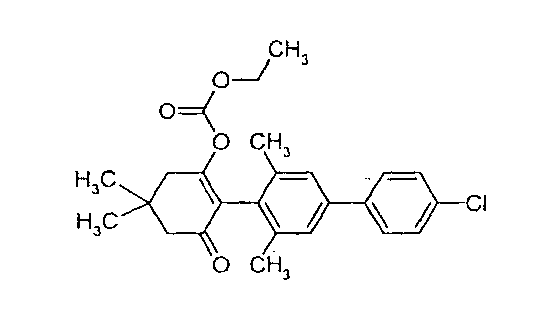

Analog zu Beispiel (I-3-b-1) erhält man die oben gezeigte Verbindung durch Umsetzung der Verbindung gemäß Beispiel (I-8-a-1) mit Chlorameisensäureethylester als wachsartige Substanz.

¹H-NMR (400 MHz; CDCl₃):δ = 1,05 (t, 3H, CH₂CH₃, 1,2 (s, 6H, C(CH₃)₂), 2,1 (s, 6H, 2 x Ar - CH₃), 2,45:2,7 (s, 2x2H, CH₂), 4,1 (q, 2H, CH₂-CH3) 7,35-7,7 (m, 6H, Ar-H) ppm.

### Beispiel I-8-c-2

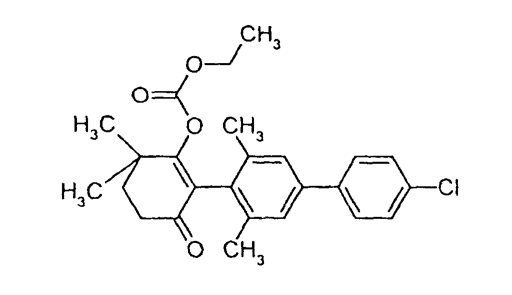

Analog zu Beispiel I-3-b-1 erhält man die oben gezeigte Verbindung durch Umsetzung der Verbindung gemäß Beispiel (I-8-a-2) mit Chlorameisensäureethylester als wachsartige Substanz.

¹H-NMR (400 MHz; CDCl₃):δ = 1,05 (t, 3H, CH₂CH₃, 1,15 (s, 6H, C(CH₃)₂), 2,0 : 2,7 (t, 2x2H, CH₂-CH₂) 2,05 (s, 6H, 2 x Ar-CH₃), 4,05 (q, 2H, CH₂-CH3) 7,3-7,7 (m, 6H, Ar-H) ppm.

### Beispiel II-1

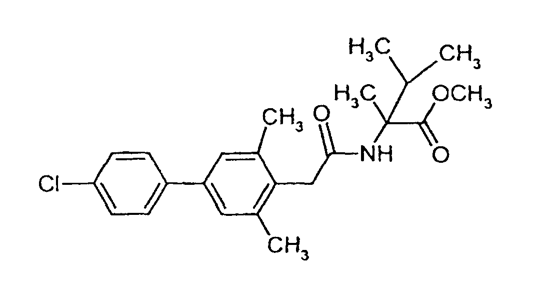

Bei einer Temperatur von 30 bis 40°C tropft man 7,75 g der Verbindung gemäß Beispiel XXIX-1 in 80 ml Methylenchlorid zu 10,3 g konzentrierter Schwefelsäure und rührt noch 2 Stunden bei dieser Temperatur. Dann tropft man 14 ml Methanol zu und rührt weitere 6 Stunden bei 70°C. Dann gießt man auf 110 g Eis, extrahiert mit Methylenchlorid, wäscht mit wäßriger NaHCO₃-Lösung, trocknet und engt ein. Nach Umkristallisation aus MTBE/n-Hexan wird säulenchromatographisch an Kieselgel weiter gereinigt (Laufmittel Methylenchlorid/Essigsäureethylester 5/3). Ausbeute 4,24 g (50 % der Theorie). Fp.: 142°C.

### Beispiel II-2

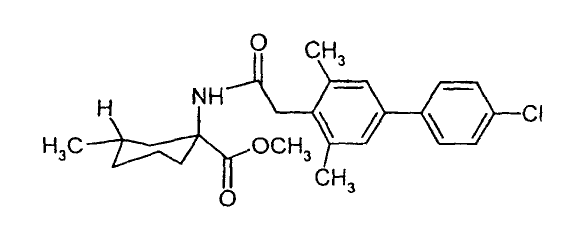

Zu 4,57 g 3-Methyl-1-amino-cyclohexancarbonsäure-methylester x Hydrochlorid und 10 g gemahlenes K₂CO₃ in 20 ml Acetonitril tropft man bei 5 bis 10°C 5,86 g 2,6-Dimethyl-4-(4-chlorphenyl)-phenylessigsäurechlorid gemäß Beispiel (XXIV-1) in 10 ml Acetonitril und rührt noch 3 Stunden bei Raumtemperatur. Man gießt in 200 ml Eiswasser, saugt ab, nimmt in Methylenchlorid auf, trocknet und engt ein. Es wird aus MTBE/n-Hexan umkristallisiert. Ausbeute 7,12 g (83 % der Theorie), Fp.: 169°C.

In Analogie zu den Beispielen (II-1) und (II-2) bzw. gemäß den allgemeinen Angaben zur Herstellung von Verbindungen der Formel (II) erhält man folgende Verbindungen der Formel (II)

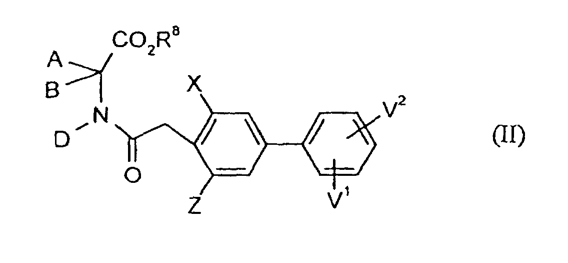

| Bsp.- Nr. | X | Z | V¹ | V² | D | A | B | R⁸ | Fp. °C | Isomer |
|---|---|---|---|---|---|---|---|---|---|---|
| II-3 | CH₃ | CH₃ | 3-Cl | H | H | -(CH₂)₂-CHOCH₃-(CH₂)₂- | | CH₃ | 88 | β |
| II-4 | CH₃ | CH₃ | 4-Cl | H | H | -(CH₂)₂-CHOCH₃-(CH₂)₂- | | CH₃ | 127 | β |
| II-5 | CH₃ | CH₃ | 2-Cl | H | H | -(CH₂)₂-CHOCH₃-(CH₂)₂- | | CH₃ | 75 | β |
| II-6 | CH₃ | CH₃ | 4-Cl | H | H | -(CH₂)₂-O-(CH₂)₂- | | CH₃ | 179 | - |
| II-7 | CH₃ | CH₃ | 4-Cl | H | H | -(CH₂)₂-OCHC₂H₅-(CH₂)₂- | | CH₃ | 146 | β |
| II-8 | CH₃ | CH₃ | 4-Cl | H | H | -(CH₂)₂-CHCH₃-(CH₂)₂- | | CH₃ | 167 | β |
| II-9 | CH₃ | CH₃ | 4-CH₃ | H | H | -(CH₂)₂-CHOCH₃-(CH₂)₂- | | CH₃ | 159 | β |
| II-10 | CH₃ | CH₃ | 2-CH₃ | 5-F | H | -(CH₂)₂-CHOCH₃-(CH₂)₂- | | CH₃ | 138 | β |
| II-11 | CH₃ | CH₃ | 2-CH₃ | 5-CH₃ | H | -(CH₂)₂-CHOCH₃-(CH₂)₂- | | CH₃ | 71 | β |
| II-12 | CH₃ | CH₃ | 2-CH₃ | 3-CH₃ | H | -(CH₂)₂-CHOCH₃-(CH₂)₂- | | CH₃ | 133 | β |
| II-13 | CH₃ | CH₃ | 2-OCH₃ | H | H | -(CH₂)₂-CHOCH₃-(CH₂)₂- | | CH₃ | 156 | β |
| II-14 | CH₃ | CH₃ | 3-CH₃ | 5-CH₃ | H | -(CH₂)₂-CHOCH₃-(CH₂)₂- | | CH₃ | 169 | β |
| II-15 | CH₃ | CH₃ | 2-Cl | 4-Cl | H | -(CH₂)₂-CHOCH₃-(CH₂)₂- | | CH₃ | 125 | β |
| II-16 | CH₃ | CH₃ | 2-Cl | 3-Cl | H | -(CH₂)₂-CHOCH₃-(CH₂)₂- | | CH₃ | 127 | β |
| II-17 | CH₃ | CH₃ | 4-Cl | H | -(CH₂)₄- | | H | C₂H₅ | Öl | - |
| II-18 | CH₃ | CH₃ | 4-Cl | H | -(CH₂)₂-S-CH₂- | | H | C₂H₅ | 81 | - |
| II-19 | CH₃ | CH₃ | 4-Cl | H | i-C₃H₇ | H | H | C₂H₅ | 119 | - |

### Beispiel XXIX-1

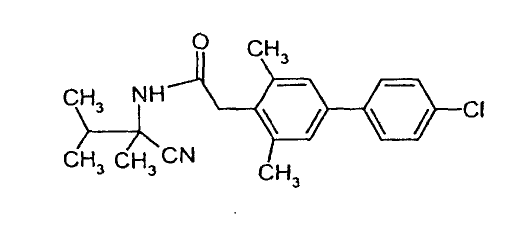

Zu 3,7 g 2-Amino-2,3-dimethyl-buttersäurenitril und 13,8 g gemahlenem K₂CO₃ in 30 ml Acetonitril tropft man bei 5 bis 10°C 8,79 g 2,6-Dimethyl-4-(4-chlorphenyl)-phenylessigsäurechlorid in 15 ml Acetonitril und rührt noch 3 Stunden bei Raumtemperatur. Man gießt in 250 ml Eiswasser, saugt ab und wäscht mit Wasser. Man nimmt in Methylenchlorid auf, trocknet und engt ein, anschließend wird chromatographisch aus Kieselgel gereinigt (Laufmittel n-Hexan/Essigsäureethylester 3/1). Ausbeute 8,24 g (74 % der Theorie), Fp. 180°C.

### Beispiel (III-1)

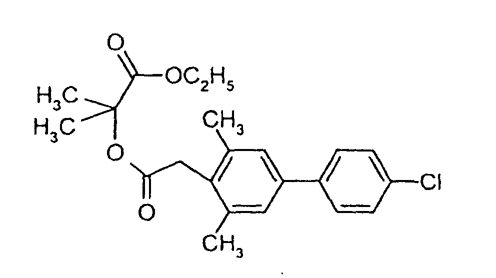

Das Gemisch aus 2,29 g der Verbindung gemäß Beispiel (XXIV-1) und 1,03 g Hydroxyisobuttersäureethylester wird über Nacht auf 140°C erhitzt.

GC/MS: m/e = 115 (8 %), 179 (34 %), 229 (100 %), 256 (12 %), 388 (20 %).

In Analogie zu Beispiel (III-1) bzw. gemäß den allgemeinen Angaben zur Herstellung von Verbindungen der Formel (III) erhält man folgende Verbindungen der Formel (I-2-a)'

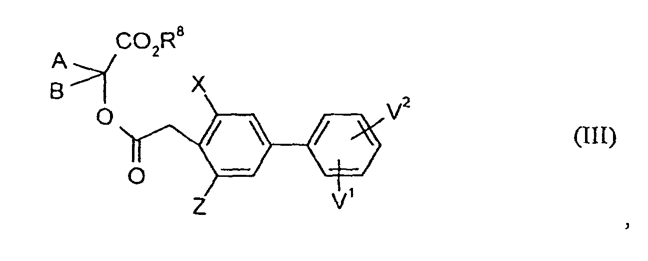

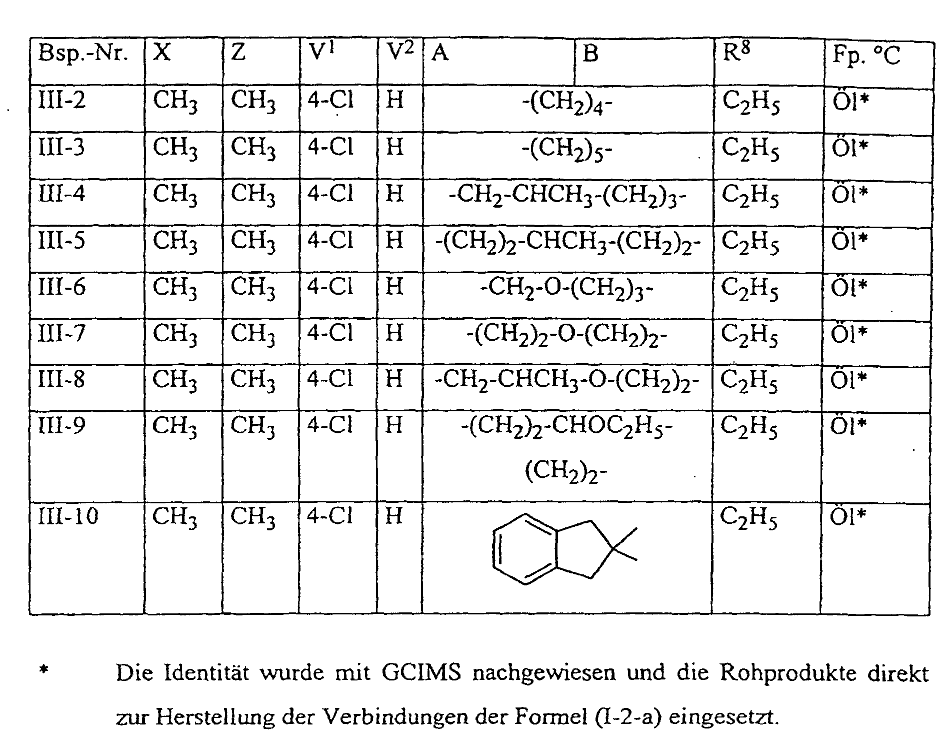

### Beispiel (IV-1)

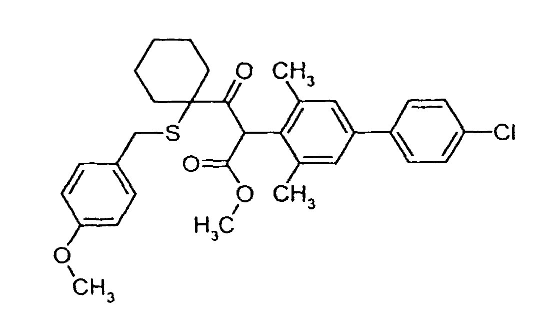

A: 15,0 g der Verbindung 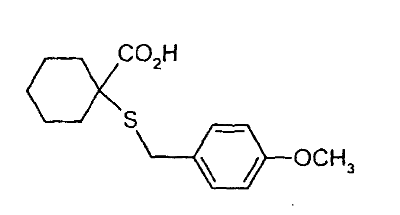 9,63 g Thionylchlorid und 1 Tropfen DMF wurden 5 Minuten bei Raumtemperatur und dann bei 100°C gerührt, bis die Gasentwicklung beendet ist. Dann wird eingeengt und im Hochvakuum getrocknet.
B: Zu 45,8 ml (96,3 mmol) einer Lithiumdiisopropylamid (LDA)-Lösung in 100 ml absolutem Tetrahydrofuran (THF) tropft man bei 0°C 25,3 g der Verbindung gemäß Beispiel (XXXII-1) und rührt noch 30 Minuten bei dieser Temperatur. Dann tropft man das nach A erhaltene Säurechlorid in 30 ml THF zu, entfernt die Kühlung und rührt noch 1 Stunde. Man setzt 300 ml MTBE und einige Tropfen Wasser zu, wäscht 2 mal mit je 300 ml 10 %iger wäßriger Ammoniumchloridlösung, trocknet und engt ein. Ausbeute 45 g (100 % der Theorie) als Öl.

¹H-NMR (CDCl₃, 400 MHz):δ = 1,5 - 2,0 (m, 10H, Cyclohexyl), 2,4 (s, 6H, 2 x Ar-CH₃), 3,1 ; 3,3 (d, 2H, S-CH₂) 3,6; 3,7 (s, 2 x 3H, 2 x OCH₃), 6,8-7,7 (m, 10H, Ar-H) ppm.

### Beispiel X-1

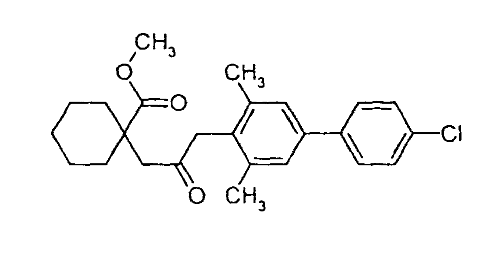

70,0 g der Verbindung gemäß Beispiel (XXXVIII-1), 24,05 g Kaliumcarbonat und 74,4 g Methyliodid werden in 400 ml Aceton 16 Stunden unter Rückfluß gerührt. Man filtriert und engt ein. Der Rückstand wird an Kieselgel chromatographiert (Laufmittel Methylenchlorid/Petrolether 4/1). Ausbeute 20,5 g (41 % der Theorie) als Öl.

¹H-NMR (CDCl₃, 400 MHz):δ = 1,3 - 1,8 (m, 10 H, Cyclohexyl), 2,2 (s, 6H, 2 x Ar-CH₃), 2,95 (s, 2H, CH₂CO), 3,55 (s, 3H, OCH₃), 3,85 (s, 2H, Ar-CH₂), 7,3 - 7,65 (m, 6H, Ar-H) ppm.

### Beispiel (XXXVIII-1)

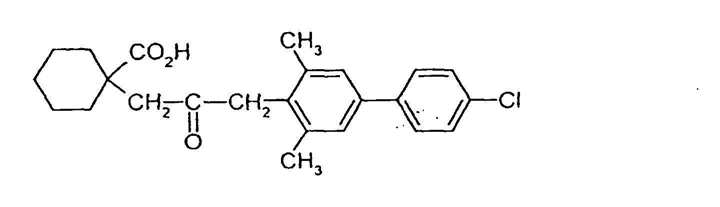

Zu 100 ml LDA-Lösung (2 molar) und 200 ml THF wird bei -15°C eine Lösung von 51,9 g der Verbindung gemäß Beispiel (XXXII-1) in 80 ml THF getropft und anschließend 1 Stunde bei 0°C gerührt. Dann tropft man bei -15°C eine Lösung von 20,2 g der Verbindung 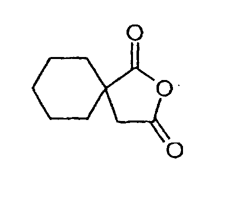 in 30 ml THF zu. Man rührt 2 Stunden bei Raumtemperatur, gibt 300 ml Wasser und 80 g Ammoniumchlorid zu und säuert mit konzentriertem HCl an. Man extrahiert mit Ether und engt die Etherphase ein. Der Rückstand wird mit 200 g KOH und 660 ml Wasser zwei Tage unter Rückfluß gekocht. Nach dem Abkühlen wird mit konzentriertem HCl angesäuert und mit Ether extrahiert. Das nach dem Einengen verbleibende Rohprodukt, das als Öl vorlag, wird ohne weitere Reinigung weiter umgesetzt. Ausbeute 70 g (100 % der Theorie).

### Beispiel (XI-1)

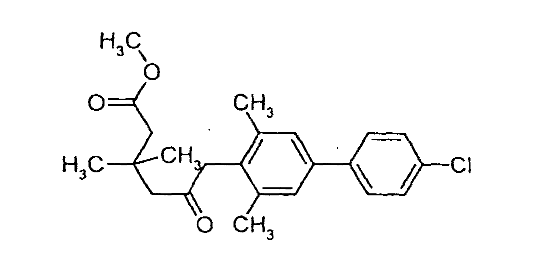

36 g der Verbindung gemäß Beispiel (XLII-1), 13,2 g Kaliumcarbonat und 40,8 g Methyliodid werden in 200 ml Aceton 16 Stunden unter Rückfluß gekocht. Man filtriert, engt ein und reinigt den Rückstand säulenchromatographisch an Kieselgel (Laufmittel Methylenchlorid/Petrolether 2/1). Ausbeute 12 g (52 % der Theorie), Öl.

¹H-NMR (400 MHz, CDCl₃):δ = 1,05 (s, 6H, C(CH₃)₂, 2,2 (s, 6H, 2 x Ar-CH₃), 2,4; 2,7 (s, 2 x 2H, CO-CH₂), 3,6 (s, 3H, OCH₃), 3,85 (s, 2H, Ar-CH₂), 7,3-7,65 (m, 6 H, Ar-H) ppm.

### Beispiel (XI-2)

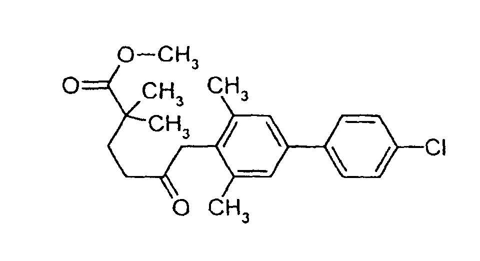

Analog zu Beispiel (XI-1) erhält man die oben gezeigte Verbindung, ebenfalls als Öl.

¹H-NMR (400 MHz, CDCl₃):δ = 1,1 (s, 6H, C(CH₃)₂), 1,75; 2,55 (t, 2 x 2H, CH₂CH₂), 2,2 (s, 6H, 2 x Ar-CH₃), 3,6 (s, 3H, OCH₃), 3,9 (s, 2H, ArCH₂, 7,3-7,7 (m, 6H, Ar-H) ppm.

### Beispiel (XLII-1)

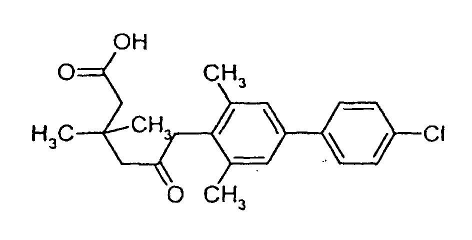

Zu 50 ml LDA-Lösung (2 molar) in 100 ml THF wird bei -15°C eine Lösung von 27 g der Verbindung gemäß Beispiel (XXXII-1) in 30 ml THF getropft und 1 Stunde bei 0°C gerührt. Dann tropft man bei -15°C eine Lösung von 8,6 g der Verbindung 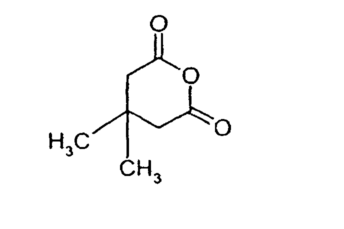 in 20 ml THF zu. Man rührt 2 Stunden bei Raumtemperatur, dann werden 150 ml Wasser und 40 g Ammoniumchlorid zugegeben und mit konzentrierter HCl angesäuert. Man extrahiert mit Ether und engt die Etherphase ein. Der Rückstand wird mit 100 g KOH und 330 ml Wasser zwei Tage unter Rückfluß gekocht. Nach dem Abkühlen wird mit konzentrierter HCl angesäuert und mit Ether extrahiert. Das nach dem Einengen verbleibende Rohprodukt (36 g) wird ohne weitere Reinigung weiter umgesetzt.

### Beispiel (LXII-2)

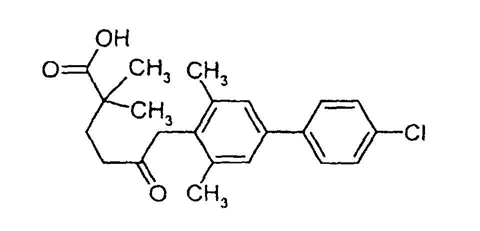

Analog zu Beispiel (LXII-1) wird die oben gezeigte Verbindung erhalten.

### Beispiel XXIV-1

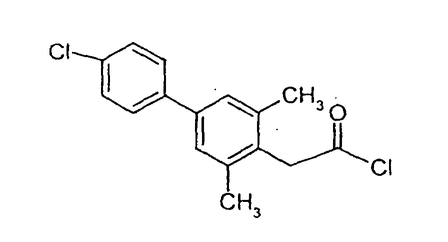

69 g Säure gemäß Beispiel XXVII-1 wird mit 55 ml Thionylchlorid auf 70°C erwärmt, bis die Gasentwicklung beendet ist.

Überschüssiges Thionylchlorid wird anschließend im Vakuum entfernt. Ausbeute 54,9 g (74 % der Theorie), Fp.: 102°C.

In Analogie zu Beispiel (XXIV-1) bzw. gemäß den allgemeinen Angaben zur Herstellung der Verbindungen (XXIV) erhält man folgende Verbindungen der Formel (XXIV)

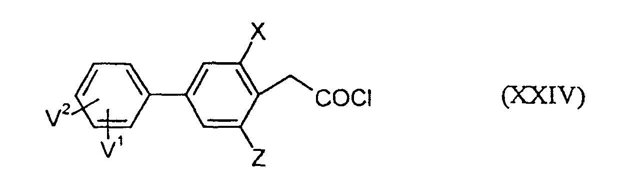

| Bsp.-Nr. | X | Z | V¹ | V² | Fp. °C |
|---|---|---|---|---|---|
| XXIV-2 | CH₃ | CH₃ | 3-Cl | H | * |
| XXIV-3 | CH₃ | CH₃ | 2-Cl | H | * |
| XXIV-4 | CH₃ | CH₃ | 4-CH₃ | H | * |
| XXIV-5 | CH₃ | CH₃ | 2-CH₃ | 5-F | * |
| XXIV-6 | CH₃ | CH₃ | 2-CH₃ | 5-CH₃ | * |
| XXIV-7 | CH₃ | CH₃ | 2-CH₃ | 3-CH₃ | * |
| XXIV-8 | CH₃ | CH₃ | 2-OCH₃ | H | * |
| XXIV-9 | CH₃ | CH₃ | 3-CH₃ | 5-CH₃ | * |
| XXIV-10 | CH₃ | CH₃ | 2-Cl | 4-Cl | * |
| XXIV-11 | CH₃ | CH₃ | 2-Cl | 3-Cl | * |

| | | | | | |
|---|---|---|---|---|---|
| * Die Säurechloride wurden ohne weitere Reinigung zur Synthese von Verbindungen der Formel (II) verwendet. | | | | | |

### Beispiel (XXVII-1)

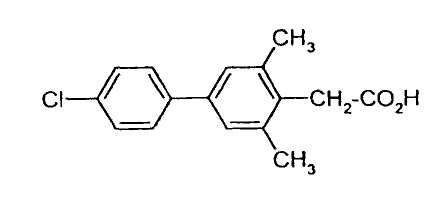

6 g der Verbindung gemäß Beispiel (XXXII-1), 1,2 g Lithiumhydroxid, 20 ml Ethanol, 100 ml Wasser und 100 ml THF werden über Nacht bei Raumtemperatur gerührt. Das THF wird im Vakuum entfernt und die verbleibende wäßrige Lösung mehrmals mit Methylenchlorid extrahiert. Die wäßrige Phase wird mit konzentrierter HCl angesäuert und das ausgefallene Produkt abgesaugt. Ausbeute 5 g (96 % der Theorie), Fp. 205°C.

Analog zu Beispiel (XXVII-1) bzw. gemäß den allgemeinen Angaben zur Herstellung der Verbindungen (XXVII) erhält man folgende Verbindungen der Formel (XXIV)

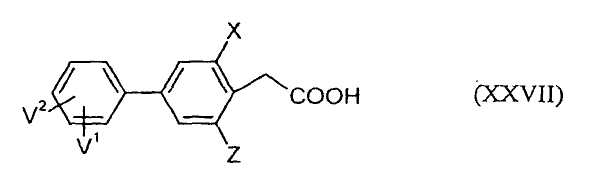

| Bsp.-Nr. | X | Z | V¹ | V² | Fp. °C |
|---|---|---|---|---|---|
| XXVII-2 | CH₃ | CH₃ | 3-Cl | H | 143 |
| XXVII-3 | CH₃ | CH₃ | 2-Cl | H | 129 |
| XXVII-4 | CH₃ | CH₃ | 4-CH₃ | H | 154 |
| XXVII-5 | CH₃ | CH₃ | 2-CH₃ | 5-F | 120 |
| XXVII-6 | CH₃ | CH₃ | 2-CH₃ | 5-CH₃ | 141 |
| XXVII-7 | CH₃ | CH₃ | 2-CH₃ | 3-CH₃ | 155 |
| XXVII-8 | CH₃ | CH₃ | 2-OCH₃ | H | 151 |
| XXVII-9 | CH₃ | CH₃ | 3-CH₃ | 5-CH₃ | 173 |
| XXVII-10 | CH₃ | CH₃ | 2-Cl | 4-Cl | 166 |
| XXVII-11 | CH₃ | CH₃ | 2-Cl | 3-Cl | 158 |

### Beispiel (XXXII-1)

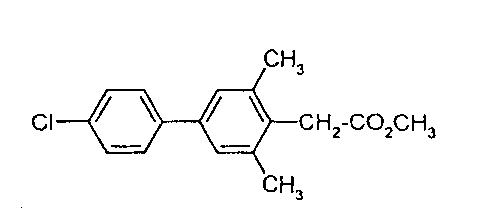

30 g der Verbindung gemäß Beispiel (XLV-1) werden mit 19,8 g 88 %iger KOH in 1000 ml Methanol über Nacht unter Rückfluß gekocht. Nach dem Abkühlen wird mit 20 ml konzentrieter Schwefelsäure versetzt und 1 Stunde unter Rückfluß gekocht. Der Feststoff wird abgesaugt und mit Methanol gewaschen. Das Methanol im Filtrat wird im Vakuum entfernt, der Rückstand mit Wasser versetzt und mit Methylenchlorid extrahiert. Die organische Phase wird getrocknet und eingeengt. Ausbeute 1 g (10 % der Theorie), Öl.

¹H-NMR (400 MHz, CDCl₃):δ = 2,38 (s, 6H, Ar-CH₃), 3,70 (s, 3H, OCH₃), 3,73 (s, 2H, CH₂), 7,23 (s, 2H ArH), 7,36, 7,39 (AA', BB', 2H, ArH), 7,48, 7,51 (AA', BB', 2H, Ar-H) ppm.

### Beispiel (XXXII-2)

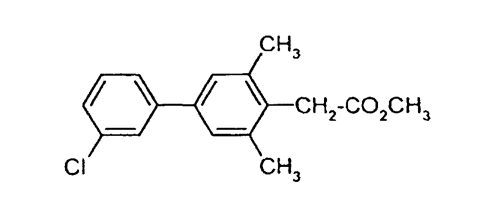

Zu 7,68 g 4-Brom-2,6-dimethylphenylessigsäuremethylester, in 85 ml Dimethoxyethan werden unter Argon 6,1 g 3-Chlorphenylboronsäure, 0,15 g Bis(triphenylphosphin)palladium(II)chlorid und 65 ml 1M Na₂CO₃-Lösung eingetragen und über Nacht unter Rückfluß gerührt. Man verdünnt mit Wasser und extrahiert mit Essigsäureethylester. Man wäscht die organische Phase mit Ammoniumchloridlösung, Wasser und gesättigter Kochsalzlösung, trocknet und engt ein. Ausbeute 4,3 g (36 % der Theorie), Fp.: 56°C.

In Analogie zu Beispiel (XXXII-1) und (XXXII-2) bzw. gemäß den allgemeinen Angaben zur Herstellung der Verbindungen (XXXII) erhält man folgende Verbindungen der Formel (XXXII)

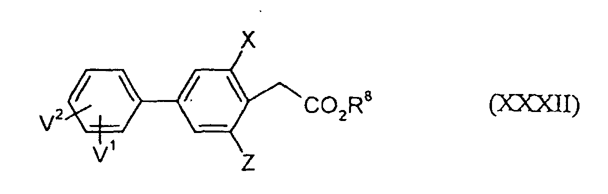

| Bsp.-Nr. | X | Z | V¹ | V² | R⁸ | Fp. °C |
|---|---|---|---|---|---|---|
| XXXII-2 | CH₃ | CH₃ | 3-CI | H | CH₃ | 56 |
| XXXII-3 | CH₃ | CH₃ | 2-Cl | H | CH₃ | Öl |
| XXXII-4 | CH₃ | CH₃ | 4-CH₃ | H | CH₃ | 137 |
| XXXII-5 | CH₃ | CH₃ | 2-CH₃ | -5-F | CH₃ | Öl |
| XXXII-6 | CH₃ | CH₃ | 2-CH₃ | 5-CH₃ | CH₃ | Öl |
| XXXII-7 | CH₃ | CH₃ | 2-CH₃ | 3-CH₃ | CH₃ | Öl |
| XXXII-8 | CH₃ | CH₃ | 2-OCH₃ | H | CH₃ | 85 |
| XXXII-9 | CH₃ | CH₃ | 3-CH₃ | 5-CH₃ | CH₃ | Öl |
| XXXII-10 | CH₃ | CH₃ | 2-Cl | 4-Cl | CH₃ | Öl |
| XXXII-11 | CH₃ | CH₃ | 2-Cl | 3-Cl | CH₃ | Öl |

### Beispiel XLVI-1

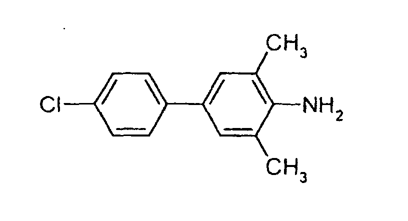

5 g 4-Brom-2,6-dimethylanilin, 3,88 g 4-Chlorphenylboronsäure und 0,11 g Bis(triphenylphosphin)palladium(II)chlorid in 48,8 ml 1M Na₂CO₃-Lösung und 65 ml Dimethoxyethan werden über Nacht unter Rückfluß erhitzt. Man versetzt mit Wasser und extrahiert mit Essigsäureethylester. Die organische Phase wird mit Ammoniumchloridlösung, Wasser und gesättigter Kochsalzlösung gewaschen, getrocknet und eingeengt. Ausbeute 4 g (77 % der Theorie), Fp.: 96°C.

## Patentansprüche

1. Verbindungen der Formel (I) 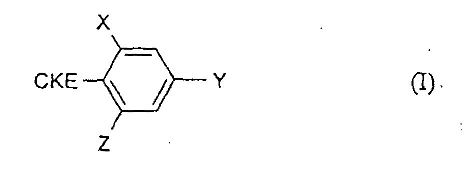 in welcher
X für Fluor, Chlor, Methyl, Ethyl, Propyl, iso-Propyl,
Y für einen der Reste 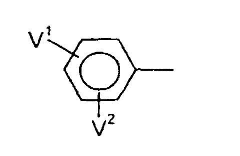 steht,
V¹ für Wasserstoff, Fluor, Chlor, Brom, Methyl, Ethyl, n-Propyl, iso-Propyl, n-Butyl, iso-Butyl, tert.-Butyl, Methoxy, Ethoxy, n-Propoxy, iso-Propoxy, Trifluormethyl, Trifluormethoxy, Nitro, Cyano oder Phenyl steht,
V² für Wasserstoff, Fluor, Chlor, Methyl, Ethyl, n-Propyl, iso-Propyl, Methoxy, Ethoxy, Trifluormethyl oder Trifluormethoxy stehen,
Z für Wasserstoff, Fluor, Chlor, Methyl, Ethyl, n-Propyl, steht,
CKE für eine der Gruppen 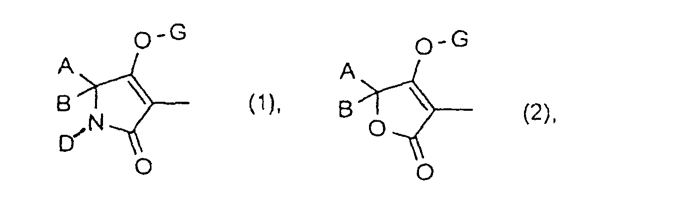 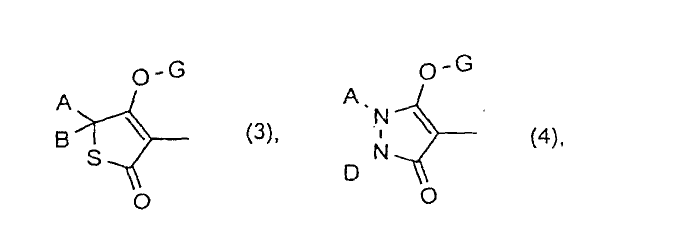 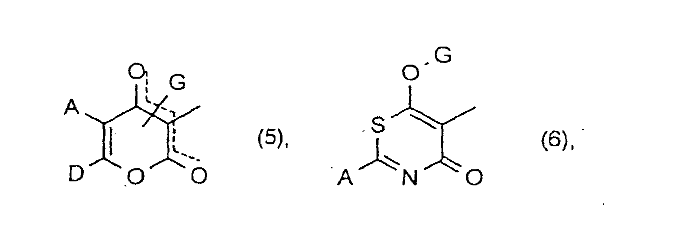 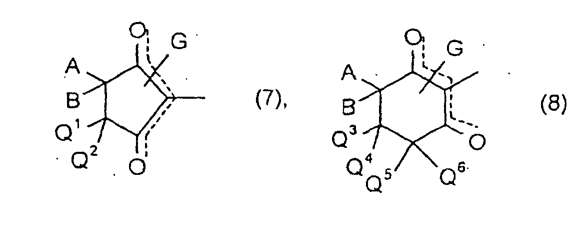 steht,
A für Wasserstoff, jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder (jedoch nicht im Fall der Verbindungen der Fonneln (I-5), (I-7) und (I-8)) für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl steht,
B für C₁-C₄-Alkyl steht, oder
A, B und das Kohlenstoffatom an das sie gebunden sind für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, n-Propyl, Isopropyl, Butyl, iso-Butyl, sec.-Butyl, tert.-Butyl, Trifluormethyl, Methoxy, Ethoxy, n-Propoxy, iso-Propoxy, n-Butoxy, iso-Butoxy, sek.-Butoxy, tert.-Butoxy, Fluor oder Chlor substituiert ist oder
A, B und das Kohlenstoffatom, an das sie gebunden sind, für C₅-C₆-Cycloalkyl stehen, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
D für Wasserstoff, für jeweils gegebenenfalls durch Fluor oder Chlor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder C₃-C₆-Cycloalkyl, in welchem gegebenenfalls eine Methylengruppe durch Sauerstoff oder Schwefel ersetzt ist oder (jedoch nicht im Fall der Verbindungen der Formeln (I-1) und (I-4)) für jeweils gegebenenfalls durch Fluor, Chlor, Methyl, Ethyl, n-Propyl, iso-Propyl, Methoxy, Ethoxy, Trifluormethyl oder Trifluormethoxy substituiertes Phenyl, Furanyl, Pyridyl, Thienyl oder Benzyl steht,
oder
A und D gemeinsam für gegebenenfalls substituiertes C₃-C₅-Alkandiyl stehen, worin gegebenenfalls ein Kohlenstoffatom durch Schwefel oder Saüerstoff ersetzt ist und welches gegebenenfalls durch Hydroxy, C₁-C₆-Alkyl oder C₁-C₄-Alkoxy substituiert ist oder
A und D (im Fall der Verbindungen der Formel (I-1)) gemeinsam mit den Atomen, an die sie gebunden sind, für eine der folgenden Gruppen AD stehen: 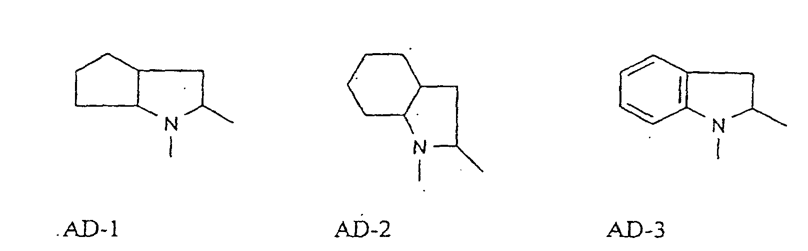 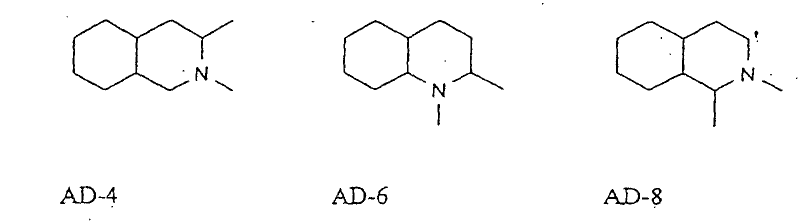 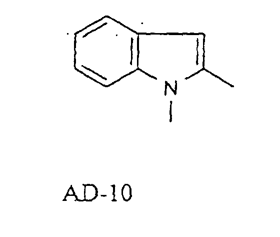
A und Q¹ gemeinsam für gegebenenfalls einfach oder zweifach durch Fluor, Hydroxy, Methyl oder Methoxy substituiertes C₃-C₄-Alkandiyl oder Butendiyl stehen, oder
Q¹ für Wasserstoff steht,
Q² für Wasserstoff steht,
Q⁴, Q⁵ und Q⁶ unabhängig voneinander für Wasserstoff, Methyl oder Ethyl stehen,
Q³ für Wasserstoff, Methyl, Ethyl oder C₃-C₆-Cycloalkyl stehen, worin gegebenenfalls eine Methylengruppe durch Sauerstoff oder Schwefel ersetzt ist oder
Q³ und Q⁴ gemeinsam mit dem Kohlenstoff, an den sie gebunden sind, für gegebenenfalls durch Methyl oder Methoxy substituiertes gesättigtes C₅-C₆-Cycloalkyl stehen, in welchem gegebenenfalls ein Ringglied durch Sauerstoff oder Schwefel ersetzt ist,
G für Wasserstoff (a) oder für eine der Gruppen 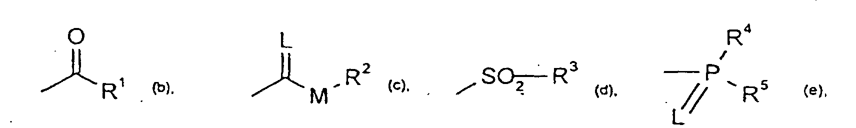 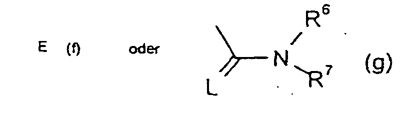 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₁-C₄-alkyl oder gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl, Pyridyl, Pyrimidyl, Thiazolyl oder Pyrazolyl,
für gegebenenfalls durch Fluor, Chlor, Methyl oder Ethyl substituiertes Phenoxy-C₁-C₂-alkyl oder
für jeweils gegebenenfalls durch Fluor, Chlor, Amino, Methyl oder Ethyl substituiertes Pyridyloxy-C₁-C₂-alkyl, Pyrimidyloxy-C₁-C₂-alkyl oder Thiazolyloxy-C₁-C₂-alkyl steht,
R² für jeweils gegebenenfalls durch Fluor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für gegebenenfalls durch Fluor substituiertes Methyl, Ethyl, n-Propyl, Isopropyl oder jeweils gegebenenfalls durch Fluor, Chlor, Brom, Methyl, tert.-Butyl, Methoxy, Trifluormethyl, Trifluormethoxy, Cyano oder Nitro substituiertes Phenyl oder Benzyl steht,
R⁴ und R⁵ unabhängig voneinander für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio stehen, und
R⁶ und R⁷ unabhängig voneinander für Wasserstoff, für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls durch Fluor, Chlor, Brom, Trifluormethyl, Methyl oder Methoxy substituiertes Phenyl, für gegebenenfalls durch Fluor, Chlor, Brom, Methyl, Trifluormethyl oder Methoxy substituiertes Benzyl, oder zusammen für einen C₅-C₆-Alkylenrest stehen, in welchem gegebenenfalls eine Methylengruppe durch Sauerstoff oder Schwefel ersetzt ist.

2. Verfahren zur Herstellung von Verbindungen der Formel (I) gemäß Anspruch 1, **dadurch gekennzeichnet, daß** man
(A) Verbindungen der Formel (I-1-a) 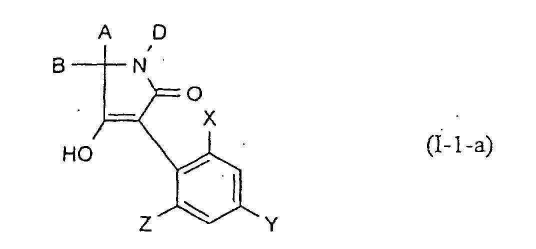 in welcher
A, B, D, X, Y und Z die in Anspruch 1 angegebenen Bedeutungen haben,
erhält, wenn man
N-Acylaminosäureester der Formel (II) 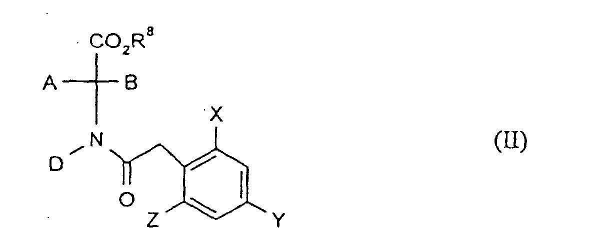 in welcher
A, B, D, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 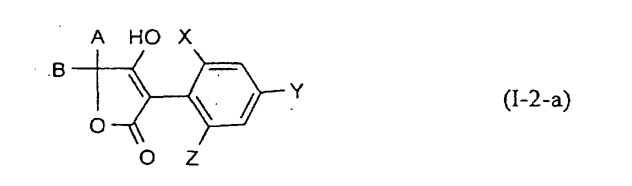 in welcher
A, B, X, Y und Z die oben angegebenen Bedeutungen haben,
erhält, wenn man
Carbonsäureester der Formel (III) 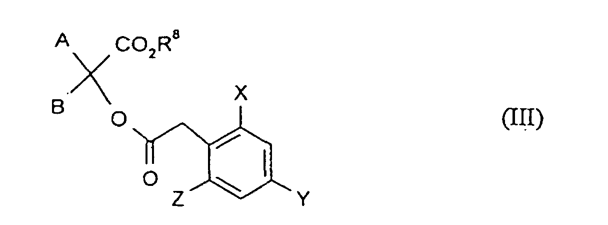 in welcher
A, B, X, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-3-a) 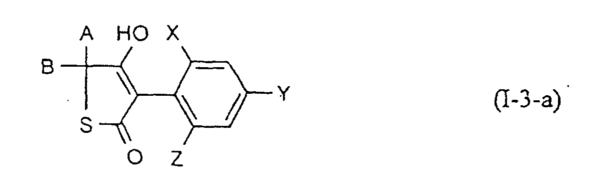 in welcher
A, B, X, Y und Z die oben angegebenen Bedeutungen haben,
erhält, wenn man
β-Ketocarbonsäureester der Formel (IV) 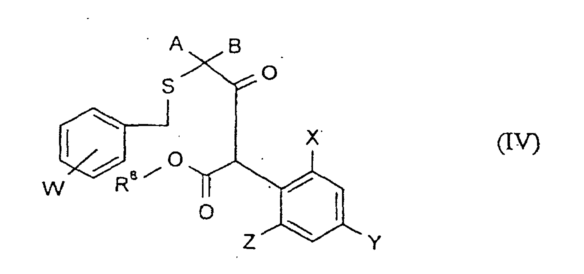 in welcher
A, B, X, Y, Z und R⁸ die oben angegebenen Bedeutungen haben und
W für Wasserstoff, Halogen, Alkyl oder Alkoxy steht,
gegebenenfalls in Gegenwart eines Verdünnungsmittels und in Gegenwart einer Säure intramolekular cyclisiert,
(D) Verbindungen der Formel (I-4-a) 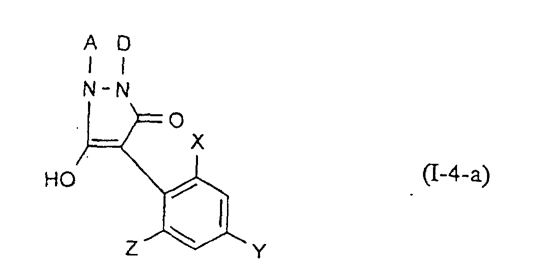 in welcher
A, D, X, Y und Z die oben angegebenen Bedeutungen haben,
erhält, wenn man
(α) Halogencarbonylketene der Formel (V) 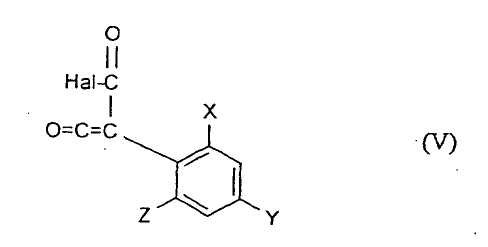 in welcher
X, Y und Z die oben angegebenen Bedeutungen haben
und
Hal für Halogen steht,
oder
(β) Malonsäurederivate der Formel (VI) 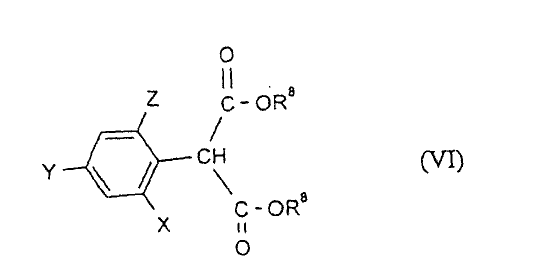 in welcher
R⁸, X, Y und Z die oben angegebenen Bedeutungen haben,
mit Hydrazinen der Formel (VII)
A-NH-NH-D (VII)
in welcher
A und D die oben angegebenen Bedeutungen haben,
gegebenenfalls in Gegenwart eines Verdünnungsmittels und gegebenenfalls in Gegenwart einer Base umsetzt,
(E) Verbindungen der Formel (I-5-a) 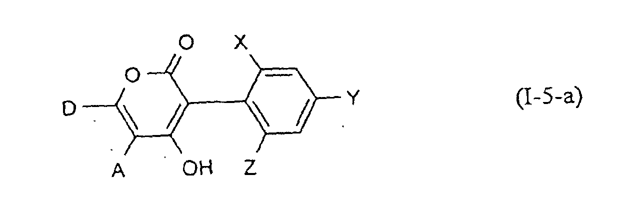 in welcher
A, D, X, Y und Z die oben angegebenen Bedeutungen haben,
erhält, wenn man
Carbonylverbindungen der Formel (VIII) 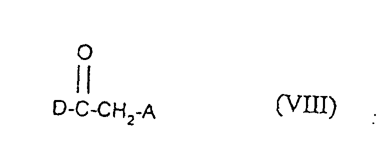 in welcher
A und D die oben angegebenen Bedeutungen haben,
oder deren Silylenolether der Formel (VIIIa) 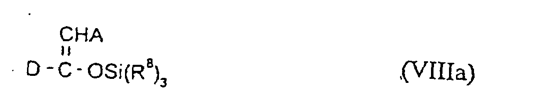 in welcher
A, D und R⁸ die oben angegebene Bedeutung haben,
mit Ketensäurehalogeniden der Formel (V) 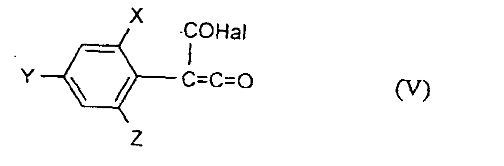 in welcher
X, Y und Z die oben angegebenen Bedeutungen haben und
Hal für Halogen steht,
gegebenenfalls in Gegenwart eines Verdünnungsmittels und gegebenenfalls in Gegenwart eines Säureakzeptors umsetzt,
(F) Verbindungen der Formel (I-6-a) 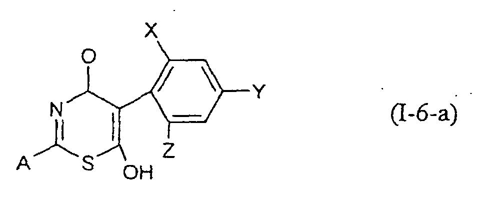 in welcher
A, X, Y und Z die oben angegebene Bedeutung haben,
erhält, wenn man Thioamide der Formel (IX) 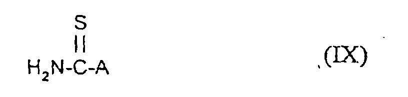 in welcher
A die oben angegebene Bedeutung hat,
mit Ketensäuzehalogeniden der Formel (V) 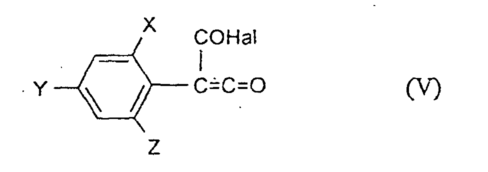 in welcher
Hal, X, Y und Z die oben angegebenen Bedeutungen haben,
gegebenenfalls in Gegenwart eines Verdünnungsmittels und gegebenenfalls in Gegenwart eines Säureakzeptors umsetzt,
(G) Verbindungen der Formel (I-7-a) 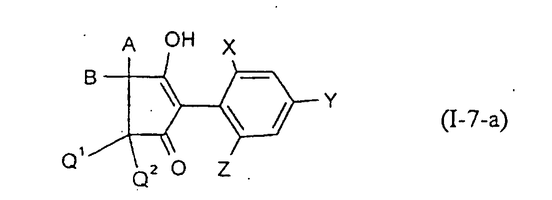 in welcher
A, B,Q¹, Q², W, X, Y und Z die in Anspruch 1 angegebene Bedeutung haben,
erhält, wenn man
Ketocarbonsäureester der Formel (X) 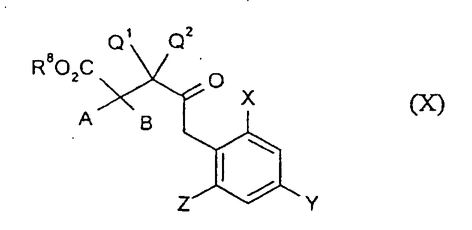 in welcher
A, B, Q¹, Q², X, Y und Z die oben angegebene Bedeutung haben, und
R⁸ für Alkyl steht,
gegebenenfalls in Gegenwart eines Verdünnungsmittels und in Gegenwart einer Base intramolekular cyclisiert,
(H) daß man Verbindungen der Formel (I-8-a) 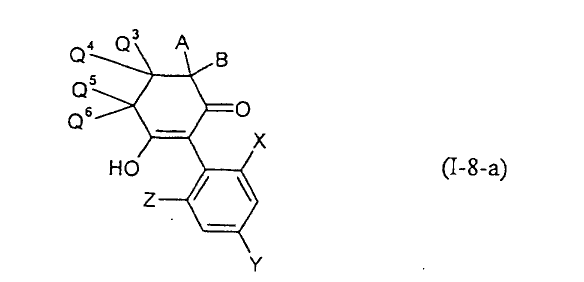 in welcher
A, B, Q³, Q⁴, Q⁵, Q⁶, X, Y und Z die in Anspruch 1 angegebene Bedeutung haben,
erhält, wenn man
6-Aryl-5-keto-hexansäureester der Formel (XI) 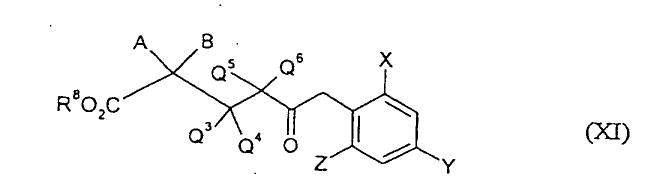 in welcher
A, B, Q³, Q⁴, Q⁵, Q⁶, X, Y und Z die oben angegebene Bedeutung haben
und
R⁸ für Alkyl steht,
in Gegenwart eines Verdünnungsmittels und in Gegenwart einer Base intramolekular kondensiert,
(I) Verbindungen der oben gezeigten Formeln (I-8-a), in welchen A, B, D, Q¹, Q², Q³, Q⁴, Q⁵, Q⁶, X, Y und Z die oben angegebene Bedeutung haben, erhält, wenn man Verbindungen der Formel (I-1'-a) bis (I-8'-a), 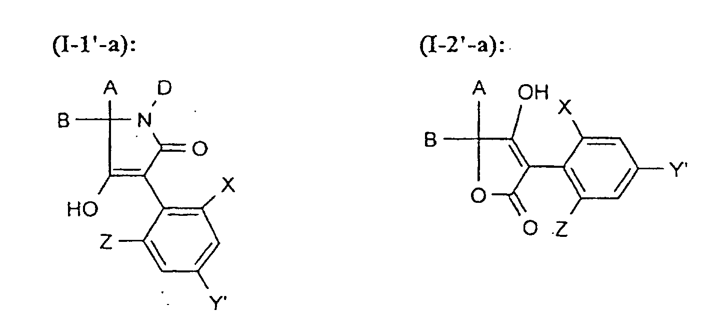 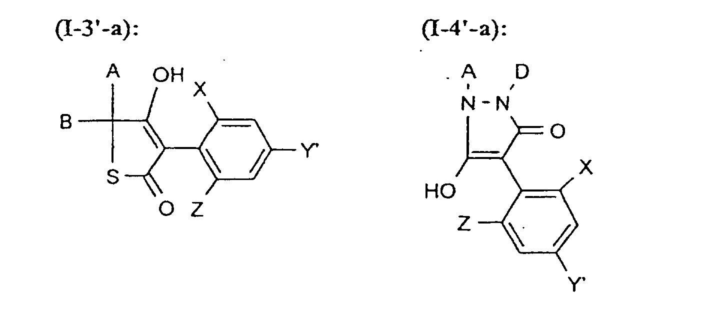 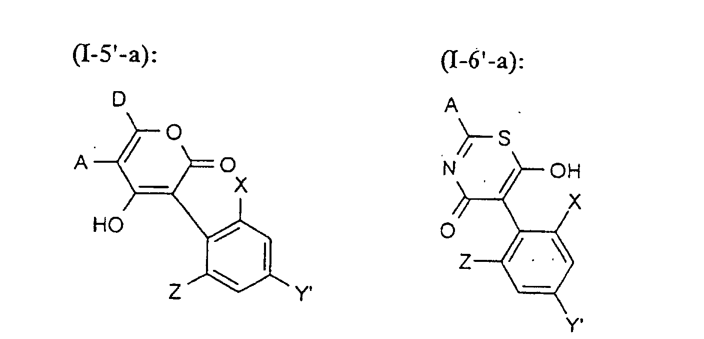 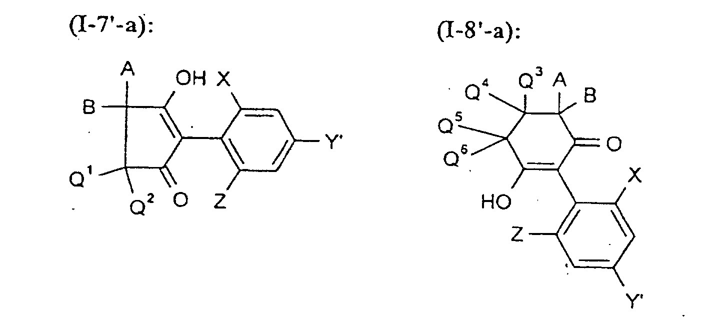 in welchen
A, B, D, Q¹, Q², Q³, Q⁴, Q⁵, Q⁶, X und Z die oben angegebene Bedeutung haben und
Y' für Chlor, Brom oder Jod steht,
mit Boronsäuren der Formel (XII) 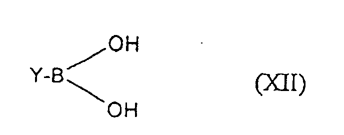 in welcher
Y die oben angegebene Bedeutung hat,
in Gegenwart eines Lösungsmittels, einer Base und eines Katalysators umsetzt und anschließend gegebenenfalls die so erhaltenen Verbindungen der Formeln (I-1-a) bis (I-8-a) jeweils
(Jα) mit Säurehalogeniden der Formel (XIII) 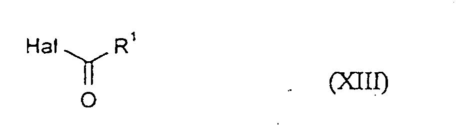 in welcher
R¹ die in Anspruch 1 angegebene Bedeutung hat und
Hal für Halogen steht
oder
(β) mit Carbonsäureanhydriden der Formel (XIV)
R¹-CO-O-CO-R¹ (XIV)
in welcher
R¹ die oben angegebene Bedeutung hat,
gegebenenfalls in Gegenwart eines Verdünnungsmittels und gegebenenfalls in Gegenwart eines Säurebindemittels umsetzt, oder jeweils
(K) mit Chlorameisensäureestern oder Chlorameisensäurethioestern der Formel (XV)
R²-M-CO-Cl (XV)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt, oder jeweils
(L) mit Chlormonothioameisensäureestern oder Chlordithioameisensäureestern der Formel (XVI) 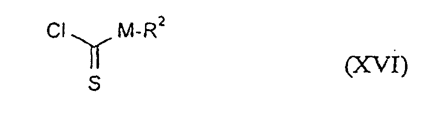 in welcher
M und R² die oben angegebenen Bedeutungen haben,
gegebenenfalls in Gegenwart eines Verdünnungsmittels und gegebenenfalls in Gegenwart eines Säurebindemittels umsetzt, oder jeweils
(M) mit Sulfonsäurechloriden der Formel (XVII)
R³-SO₂-Cl (XVII)
in welcher
R³ die in Anspruch 1 angegebene Bedeutung hat,
gegebenenfalls in Gegenwart eines Verdünnungsmittels und gegebenenfalls in Gegenwart eines Säurebindemittels umsetzt, oder jeweils
(N) mit Phosphorverbindungen der Formel (XVIII) 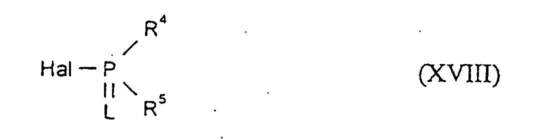 in welcher
L, R⁴ und R⁵ die in Anspruch 1 angegebenen Bedeutungen haben und
Hal für Halogen steht,
gegebenenfalls in Gegenwart eines Verdünnungsmittels und gegebenenfalls in Gegenwart eines Säurebindemittels umsetzt, oder jeweils
(O) mit Metallverbindungen oder Aminen der Formeln (XIX) oder (XX)
Me(OR¹⁰)ₜ (XIX) 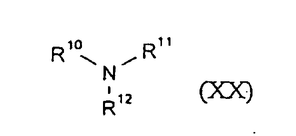
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt, oder jeweils
(Pα) mit Isocyanaten oder Isothiocyanaten der Formel (XXI)
R⁶-N=C=L (XXI)
in welcher
R⁶ und L die in Anspruch 1 angegebenen Bedeutungen haben,
gegebenenfalls in Gegenwart eines Verdünnungsmittels und gegebenenfalls in Gegenwart eines Katalysators umsetzt, oder jeweils
(β) mit Carbamidsäurechloriden oder Thiocarbamidsäurechloriden der Formel (XXII) 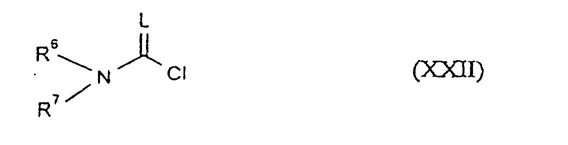 in welcher
L, R⁶ und R⁷ die in Anspruch 1 angegebenen Bedeutungen haben,
gegebenenfalls in Gegenwart eines Verdünnungsmittels und gegebenenfalls in Gegenwart eines Säurebindemittels, umsetzt.

3. Verbindungen der Formel (II) 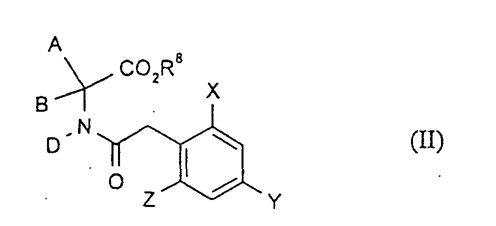 in welcher
A, B, D, X, Y und Z die in Anspruch 1 angegebenen Bedeutungen haben und
R⁸ für Alkyl steht.

4. Verbindungen der Formel (XXIV) 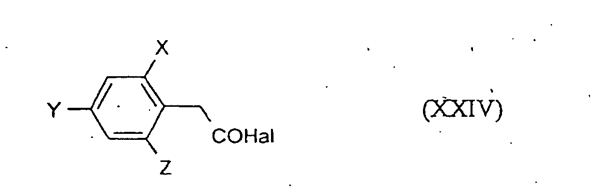 in welcher
X, Y und Z die in Anspruch 1 angegebenen Bedeutungen haben und
Hal für Chlor oder Brom steht.

5. Verbindungen der Formel (XXV) 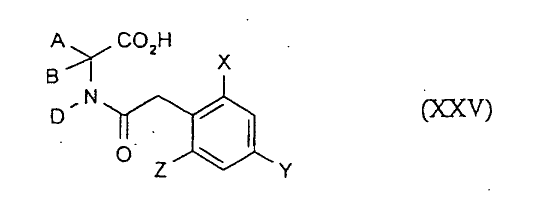 in welcher
A, B, D, X, Y und Z die in Anspruch 1 angegebenen Bedeutungen haben.

6. Verbindungen der Formel (XXIX) 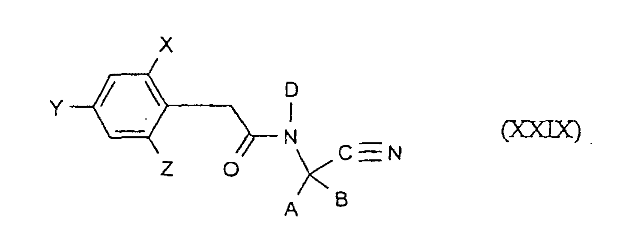 in welcher
A, B, D, X, Y und Z die in Anspruch 1 angegebenen Bedeutungen haben.

7. Verbindungen der Formel (III) 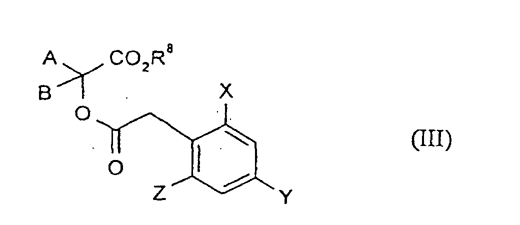 in welcher
A, B, X, Y und Z die oben angegebenen Bedeutungen haben und
R⁸ für Alkyl steht.

8. Verbindungen der Formel (XXVII) 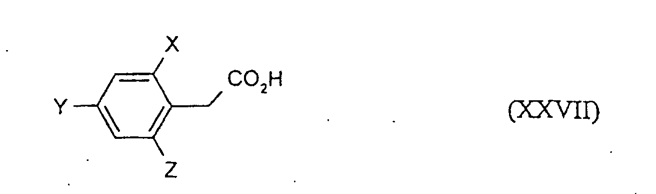 in welcher
X, Y und Z die in Anspruch 1 angegebenen Bedeutungen haben.

9. Verbindungen der Formel (IV) 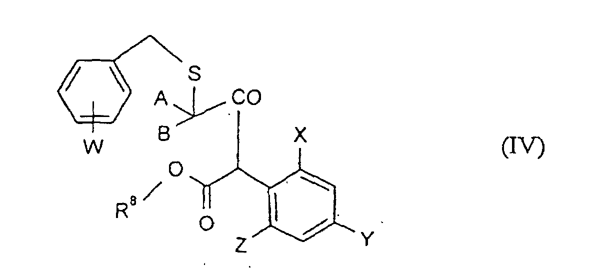 in welcher
A, B, W, X, Y und Z die in Anspruch 1 angegebenen Bedeutungen haben und
R⁸ für Alkyl steht.

10. Verbindungen der Formel (V) 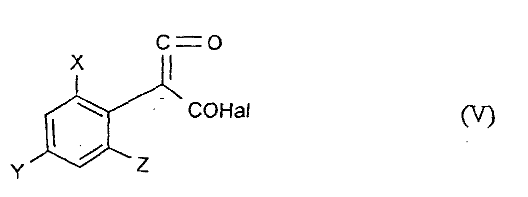 in welcher
X, Y und Z die in Anspruch 1 angegebenen Bedeutungen haben und
Hal für Chlor oder Brom steht.

11. Verbindungen der Formel (XXXVII) 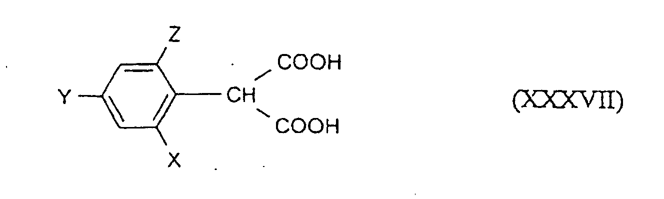 in welcher
X, Y und Z die in Anspruch 1 angegebenen Bedeutungen haben.

12. Verbindungen der Formel (VI) 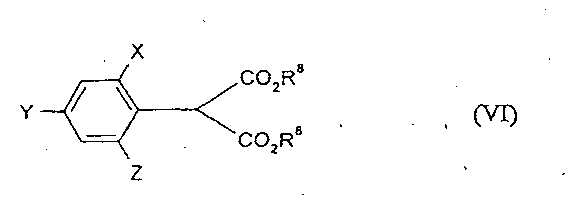 in welcher
X, Y und Z die in Anspruch 1 angegebene Bedeutung haben und
R⁸ für Alkyl steht.

13. Verbindungen der Formel (X) 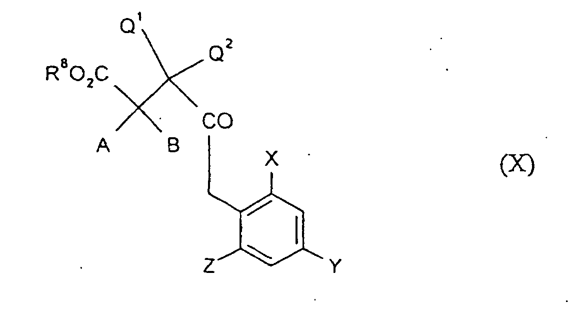 in welcher
A, B, Q¹, Q², X, Y und Z die in Anspruch 1 angegebene Bedeutung haben und
R⁸ für Alkyl steht.

14. Verbindungen der Formel (XXXVIII) 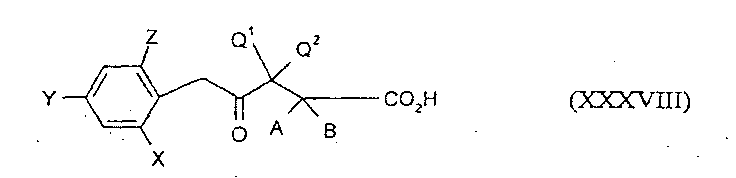 in welcher
X, Y, Z, A, B, Q¹ und Q² die in Anspruch 1 angegebene Bedeutung haben.

15. Verbindungen der Formel (XXXIX) 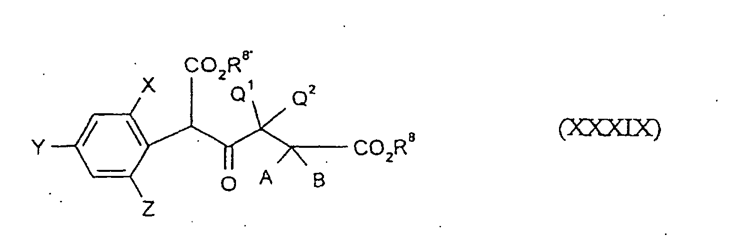 in welcher
A, B, D¹, D², X, Y und Z die in Anspruch 1 angegebene Bedeutung haben und
R⁸ und R^{8'} für Alkyl stehen.

16. Verbindungen der Formel (XI) 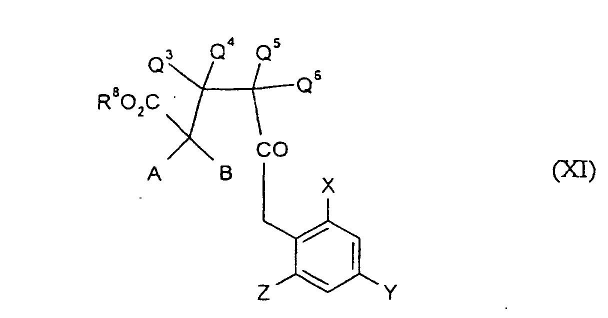 in welcher
A, B, Q³, Q⁴, Q⁵, Q⁶, X, Y und Z die in Anspruch 1 angegebene Bedeutung haben und
R⁸ für Alkyl steht.

17. Verbindungen der Formel (XLII) 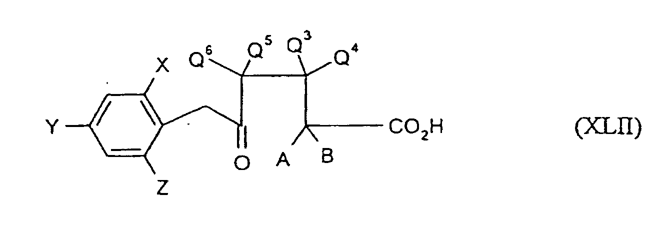 in welcher
A, B, Q³, Q⁴, Q⁵, Q⁶, X, Y und Z die in Anspruch 1 angegebene Bedeutung haben.

18. Verbindungen der Formel (XLIII) 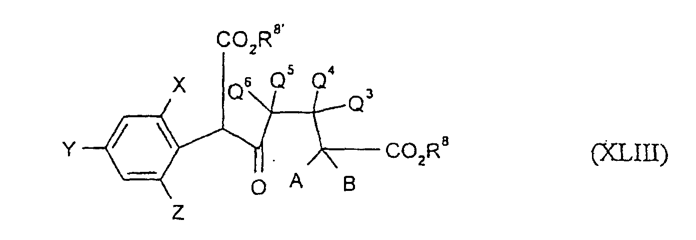 in welcher
A, B, Q³, Q⁴, Q⁵, Q⁶, X, Y und Z die in Anspruch 1 angegebene Bedeutung haben und
R⁸ und R^{8'} für Alkyl stehen.

19. Schädlingsbekämpfungsmittel und/oder Unkrautbekämpfungsmittel, **gekennzeichnet durch** einen Gehalt an mindestens einer Verbindung der Formel (I) gemäß Anspruch 1.

20. Verwendung von Verbindungen der Formel (I) gemäß Anspruch 1 zur Bekämpfung von Schädlingen im Pflanzenschutz, Haushaltsbereich, Hygienebereich und Vorratsschutz.

21. Verfahren zur Bekämpfung von Schädlingen im Pflanzenschutz, Haushaltsbereich, Hygienebereich und Vorratsschutz, **dadurch gekennzeichnet, daß** man Verbindungen der Formel (I) gemäß Anspruch 1 auf die Schädlinge und/oder ihren Lebensraum einwirken läßt.

22. Verfahren zur Herstellung von Schädlingsbekämpfungsmitteln und/oder Unkrautbekämpfungsmitteln, **dadurch gekennzeichnet, daß** man Verbindungen der Formel (I) gemäß Anspruch 1 mit Streckmitteln und/oder oberflächenaktiven Mitteln vermischt.

## Claims

1. Compounds of the formula (I) 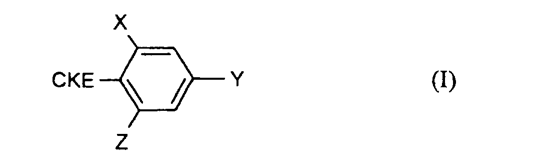 in which
X represents fluorine, chlorine, methyl, ethyl, propyl, iso-propyl,
Y represents one of the radicals 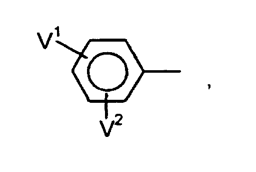
V¹ represents hydrogen, fluorine, chlorine, bromine, methyl, ethyl, n-propyl, iso-propyl, n-butyl, iso-butyl, tert-butyl, methoxy, ethoxy, n-propoxy, iso-propoxy, trifluoromethyl, trifluoromethoxy, nitro, cyano or phenyl,
V² represents hydrogen, fluorine, chlorine, methyl, ethyl, n-propyl, isopropyl, methoxy, ethoxy, trifluoromethyl or trifluoromethoxy,
Z represents hydrogen, fluorine, chlorine, methyl, ethyl, n-propyl,
CKE represents one of the groups 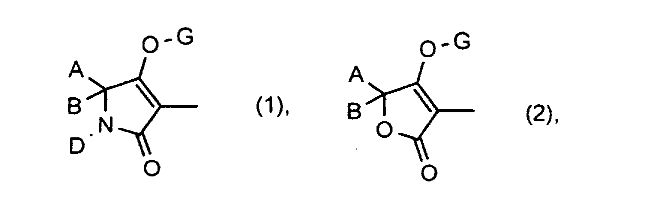 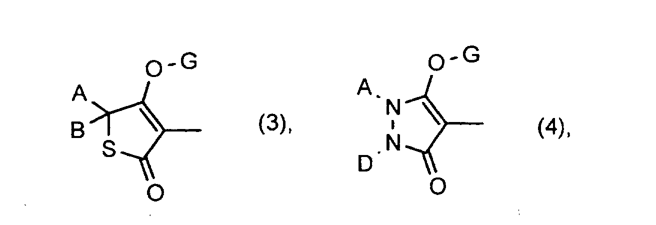 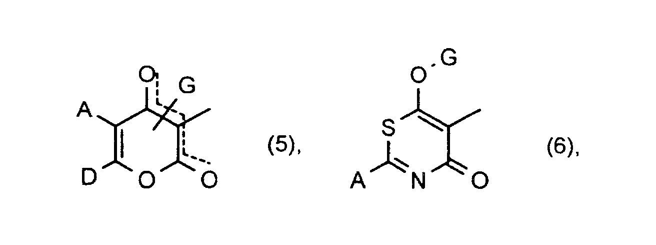 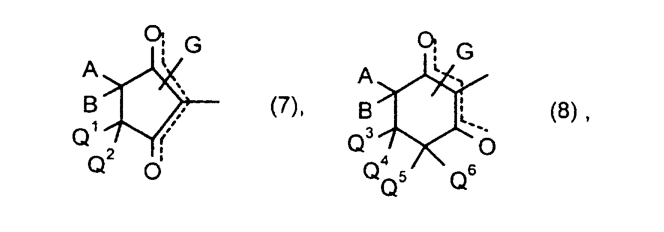
A represents hydrogen, in each case optionally fluorine-substituted C₁-C₈-alkyl or C₁-C₆-alkoxy-C₁-C₄-alkyl, optionally fluorine-, methyl-, ethyl- or methoxy-substituted C₃-C₆-cycloalkyl in which optionally one ring member is replaced by oxygen or sulphur or (but not in the case of the compounds of the formulae (1-5), (1-7) and (I-8)) represents in each case optionally fluorine-, chlorine-, bromine-, methyl-, ethyl-, n-propyl-, iso-propyl-, methoxy-, ethoxy-, trifluoromethyl-, trifluoromethoxy-, cyano- or nitro-substituted phenyl or benzyl,
B represents C₁-C₄-alkyl, or
A, B and the carbon atom to which they are attached represent saturated C₅-C₆-cycloalkyl in which optionally one ring member is replaced by oxygen or sulphur and which is optionally monosubstituted by methyl, ethyl, n-propyl, isopropyl, butyl, iso-butyl, sec-butyl, tert-butyl, trifluoromethyl, methoxy, ethoxy, n-propoxy, iso-propoxy, n-butoxy, iso-butoxy, sec-butoxy, tert-butoxy, fluorine or chlorine, or
A, B and the carbon atom to which they are attached represent C₅-C₆-cycloalkyl in which two substituents together with the carbon atoms to which they are attached represent C₂-C₄-alkanediyl or C₂-C₄-alkenediyl in which in each case optionally one methylene group is replaced by oxygen or sulphur, or, butadienediyl,
D represents hydrogen, represents in each case optionally fluorine- or chlorine-substituted C₁-C₈-alkyl, C₃-C₄-alkenyl, C₁-C₆-alkoxy-C₂-C₄-alkyl, C₁-C₄-alkylthio-C₂-C₄-alkyl or C₃-C₆-cycloalkyl in which optionally one methylene group is replaced by oxygen or sulphur or (but not in the case of the compounds of the formulae (I-1) and (I-4)) represents in each case optionally fluorine-, chlorine-, methyl-, ethyl-, n-propyl-, iso-propyl-, methoxy-, ethoxy-, trifluoromethyl- or trifluoromethoxy-substituted phenyl, furanyl, pyridyl, thienyl or benzyl,
or
A and D together represent optionally substituted C₃-C₅-alkanediyl in which optionally one carbon atom is replaced by sulphur or oxygen and which is optionally substituted by hydroxyl, C₁-C₆-alkyl or C₁₋C₄-alkoxy, or
A and D (in the case of the compounds of the formula (I-1)) together with the atoms to which they are attached represent one of the following groups AD: 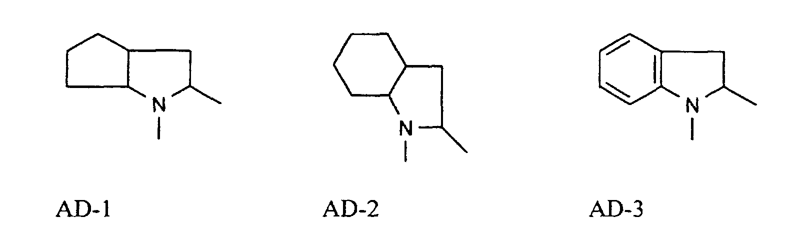 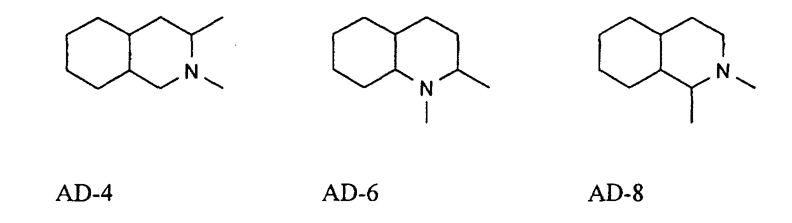 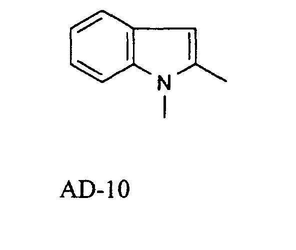
A and Q¹ together represent C₃-C₄-alkanediyl or butenediyl, which is optionally mono- or disubstituted by fluorine, hydroxyl, methyl or methoxy, or
Q¹ represents hydrogen,
Q² represents hydrogen,
Q⁴, Q⁵ and Q⁶ independently of one another each represent hydrogen, methyl or ethyl,
Q³ represents hydrogen, methyl, ethyl or C₃-C₆-cycloalkyl in which optionally one methylene group is replaced by oxygen or sulphur, or
Q³ and Q⁴ together with the carbon atom to which they are attached represent optionally methyl- or methoxy-substituted saturated C₅-C₆-cycloalkyl in which optionally one ring member is replaced by oxygen or sulphur,
G represents hydrogen (a) or represents one of the groups 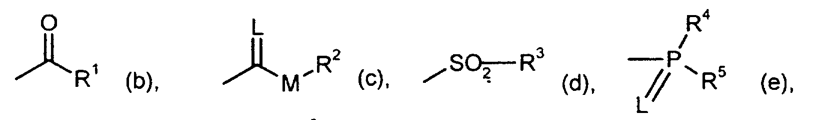 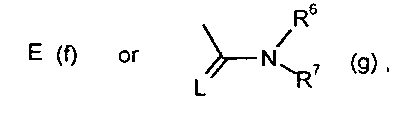 in which
E represents a metal ion or an ammonium ion,
L represents oxygen or sulphur and
M represents oxygen or sulphur,
R¹ represents in each case optionally fluorine- or chlorine-substituted C₁-C₁₄-alkyl, C₂-C₁₄-alkenyl, C₁-C₄-alkoxy-C₁-C₆-alkyl, C₁-C₄-alkylthio-C₁-C₆-alkyl, poly-C₁-C₄-alkoxy-C₁-C₄-alkyl or optionally fluorine-, chlorine-, methyl-, ethyl-, n-propyl-, i-propyl-, n-butyl-, i-butyl-, tert-butyl-, methoxy-, ethoxy-, n-propoxy- or iso-propoxy-substituted C₃-C₆-cycloalkyl in which optionally one or two not directly adjacent ring members are replaced by oxygen and/or sulphur,
represents optionally fluorine-, chlorine-, bromine-, cyano-, nitro-, methyl-, ethyl-, n-propyl-, 1-propyl-, methoxy-, ethoxy-, trifluoromethyl-, trifluoromethoxy-, methylthio-, ethylthio-, methylsulphonyl- or ethylsulphonyl-substituted phenyl,
represents optionally fluorine-, chlorine-, bromine-, methyl-, ethyl-, n-propyl-, i-propyl-, methoxy-, ethoxy-, trifluoromethyl- or trifluoromethoxy-substituted benzyl,
represents in each case optionally fluorine-, chlorine-, bromine-, methyl- or ethyl-substituted furanyl, thienyl, pyridyl, pyrimidyl, thiazolyl or pyrazolyl,
represents optionally fluorine-, chlorine-, methyl- or ethyl-substituted phenoxy-C₁-C₂-alkyl or
represents in each case optionally fluorine-, chlorine-, amino-, methyl- or ethyl-substituted pyridyloxy-C₁-C₂-alkyl, pyrimidyloxy-C₁-C₂-alkyl or thiazolyloxy-C₁-C₂-alkyl,
R² represents in each case optionally fluorine-substituted C₁-C₁₄-alkyl, C₂-C₁₄-alkenyl, C₁-C₄-alkoxy-C₂-C₆-alkyl or poly-C₁-C₄-alkoxy-C₂-C₆-alkyl,
represents optionally fluorine-, chlorine-, methyl-, ethyl-, n-propyl-, iso-propyl- or methoxy-substituted C₃-C₆-cycloalkyl,
or represents in each case optionally fluorine-, chlorine-, cyano-, nitro-, methyl-, ethyl-, n-propyl-, i-propyl-, methoxy-, ethoxy-, trifluoromethyl- or trifluoromethoxy-substituted phenyl or benzyl,
R³ represents optionally fluorine-substituted methyl, ethyl, n-propyl, isopropyl or in each case optionally fluorine-, chlorine-, bromine-, methyl-, tert-butyl-, methoxy-, trifluoromethyl-, trifluoromethoxy-, cyano- or nitro-substitued phenyl or benzyl,
R⁴ and R⁵ independently of one another each represent C₁-C₄-alkyl, C₁-C₄-alkoxy, C₁-C₄-alkylamino, di-(C₁-C₄-alkyl)amino, C₁-C₄-alkylthio or represent in each case optionally fluorine-, chlorine-, bromine-, nitro-, cyano-, C₁-C₂-alkoxy-, C₁-C₂-fluoroalkoxy-, C₁-C₂-alkylthio-, C₁-C₂-fluoroalkylthio- or C₁-C₃-alkyl-substituted phenyl, phenoxy or phenylthio, and
R⁶ and R⁷ independently of one another each represent hydrogen, represent C₁-C₄-alkyl, C₃-C₆-cycloalkyl, C₁-C₄-alkoxy, C₃-C₄-alkenyl, C₁-C₄-alkoxy-C₁-C₄-alkyl, represent optionally fluorine-, chlorine-, bromine-, trifluoromethyl-, methyl- or methoxy-substituted phenyl, represent optionally fluorine-, chlorine-, bromine-, methyl-, trifluoromethyl- or methoxy-substituted benzyl, or together represent a C₅-C₆-alkylene radical in which optionally one methylene group is replaced by oxygen or sulphur.

2. Process for preparing compounds of the formula (I) according to Claim 1, **characterized in that**
(A) compounds of the formula (I-1-a) 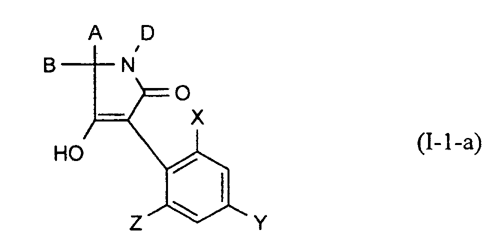 in which
A, B, D, X, Y and Z are each as defined in Claim 1
are obtained by the intramolecular condensation of
N-acylamino acid esters of the formula (II) 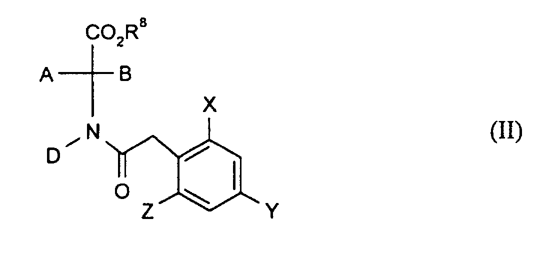 in which
A, B, D, X, Y and Z are each as defined above,
and
R⁸ represents alkyl,
in the presence of a diluent and in the presence of a base,
(B) compounds of the formula (I-2-a) 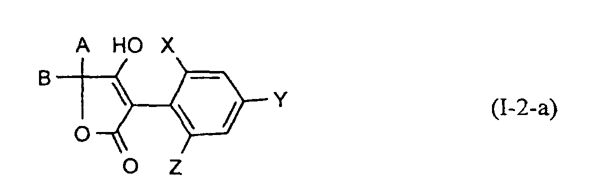 in which
A, B, X, Y and Z are each as defined above,
are obtained by the intramolecular condensation of
carboxylic esters of the formula (III) 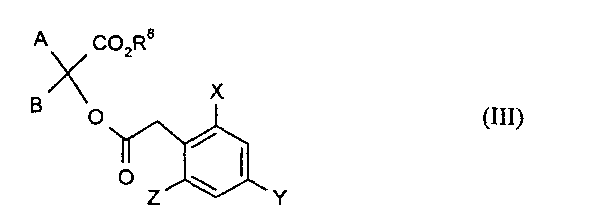 in which
A, B, X, Y, Z and R⁸ are each as defined above,
in the presence of a diluent and in the presence of a base,
(C) compounds of the formula (I-3-a) 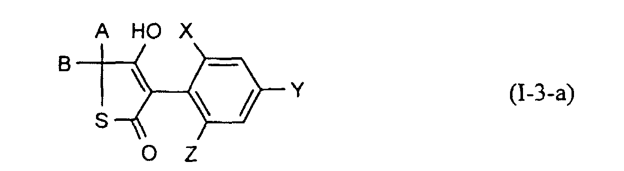 in which
A, B, X, Y and Z are each as defined above,
are obtained by the intramolecular cyclization of
β-ketocarboxylic esters of the formula (IV) 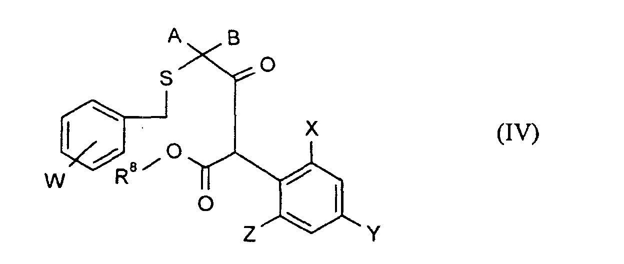 in which
A, B, X, Y, Z and R⁸ are each as defined above and
W represents hydrogen, halogen, alkyl or alkoxy,
if appropriate in the presence of a diluent and in the presence of an acid,
(D) compounds of the formula (I-4-a) 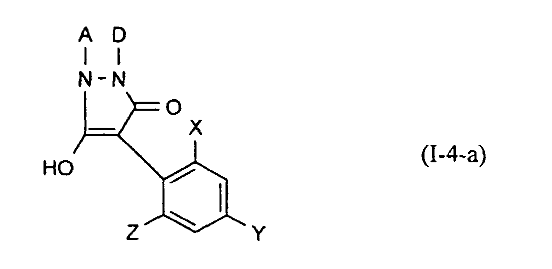 in which
A, D, X, Y and Z are each as defined above,
are obtained by reacting
(α) halogenocarbonyl ketenes of the formula (V) 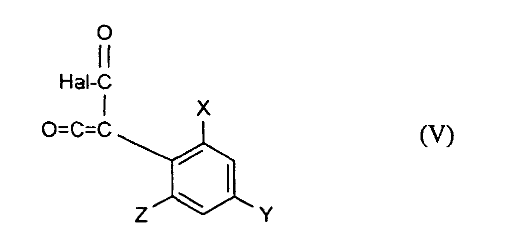 in which
X, Y and Z are each as defined above
and
Hal represents halogen,
or by reacting
(β) malonic acid derivatives of the formula (VI) 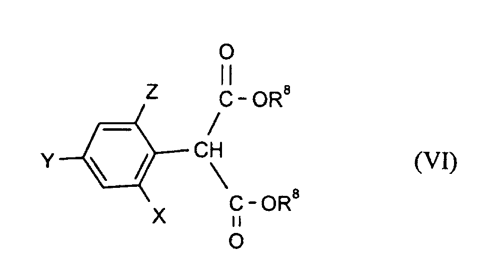 in which
R⁸, X, Y and Z are each as defined above,
with hydrazines of the formula (VII)
A-NH-NH-D (VII)
in which
A and D are each as defined above,
if appropriate in the presence of a diluent and if appropriate in the presence of a base,
(E) compounds of the formula (I-5-a) 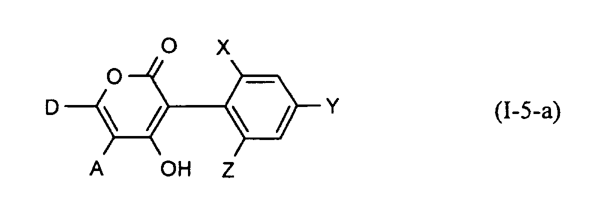 in which
A, D, X, Y and Z are each as defined above,
are obtained by reacting
carbonyl compounds of the formula (VIII) 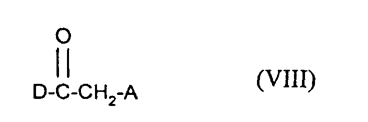 in which
A and D are each as defined above,
or their silyl enol ethers of the formula (VIIIa) 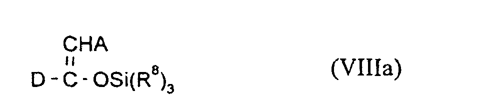 in which
A, D and R⁸ are each as defined above,
with ketene acid halides of the formula (V) 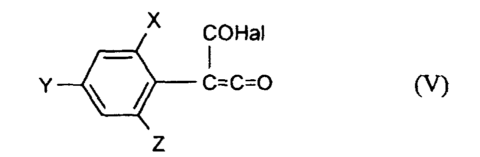 in which
X, Y and Z are each as defined above and
Hal represents halogen,
if appropriate in the presence of a diluent and if appropriate in the presence of an acid acceptor,
(F) compounds of the formula (I-6-a) 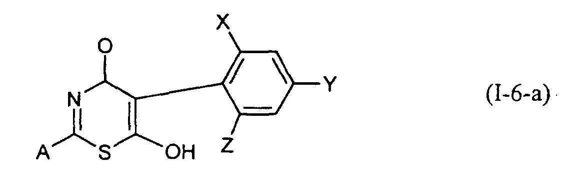 in which
A, X, Y and Z are each as defined above,
are obtained by reacting thioamides of the formula (IX) 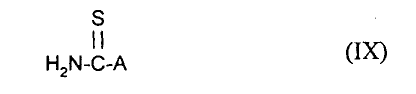 in which
A is as defined above,
with ketene acid halides of the formula (V) 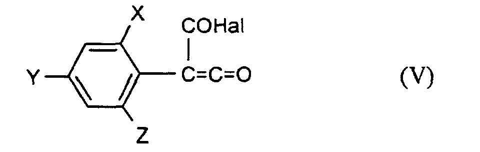 in which
Hal, X, Y and Z are each as defined above,
if appropriate in the presence of a diluent and if appropriate in the presence of an acid acceptor,
(G) compounds of the formula (I-7-a) 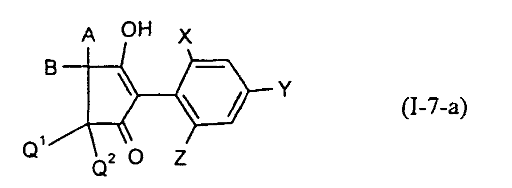 in which
A, B, Q¹, Q², W, X, Y and Z are each as defined in Claim 1
are obtained by intramolecular cyclization of
ketocarboxylic esters of the formula (X) 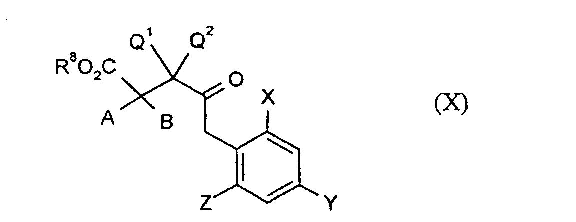 in which
A, B, Q¹, Q², X, Y and Z are each as defined above and
R⁸ represents alkyl,
if appropriate in the presence of a diluent and in the presence of a base,
(H) compounds of the formula (I-8-a) 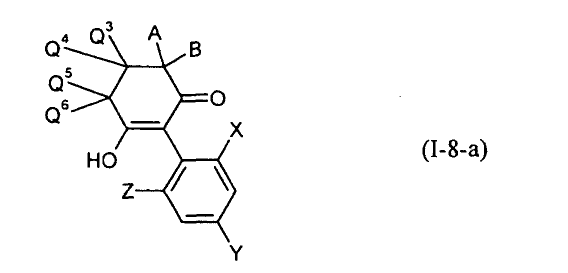 in which
A, B, Q³, Q⁴, Q⁵, Q⁶, X, Y and Z are each as defined in Claim 1
are obtained by intramolecular condensation of
6-aryl-5-keto-hexanoic esters of the formula (XI) 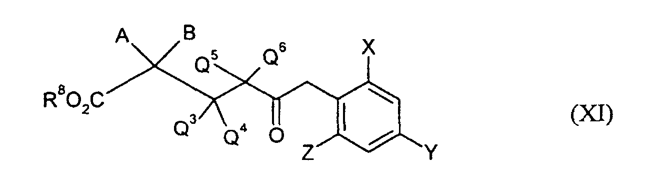 in which
A, B, Q³, Q⁴, Q⁵, Q⁶, X, Y and Z are each as defined above
and
R⁸ represents alkyl,
in the presence of a diluent and in the presence of a base,
(I) compounds of the formulae (I-8-a) shown above in which A, B, D, Q¹, Q², Q³, Q⁴, Q⁵, Q⁶, X, Y and Z are each as defined above are obtained by reacting compounds of the formula (I-1'-a) to (I-8'-a), 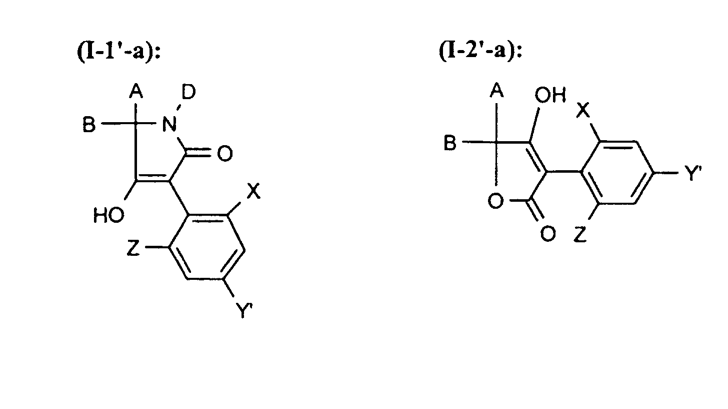 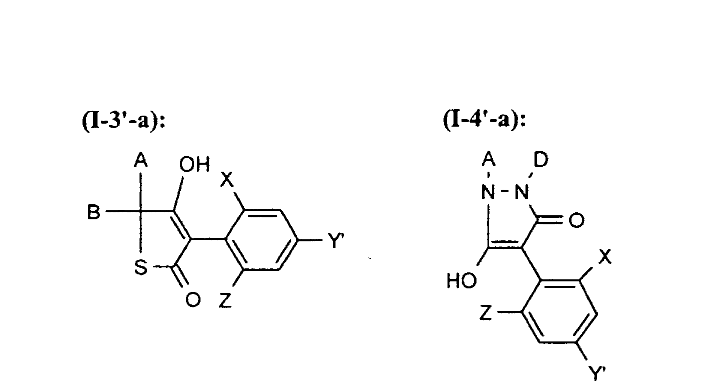 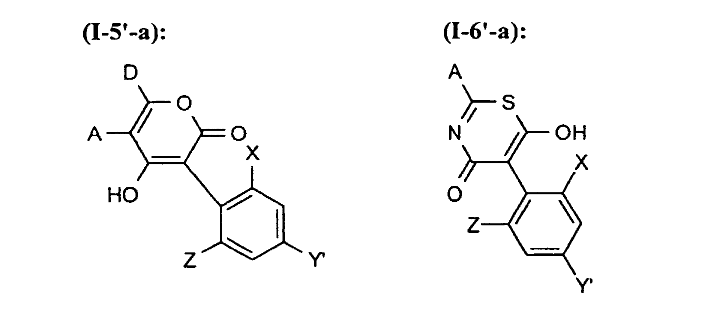 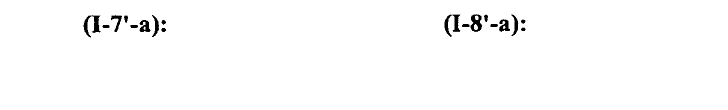 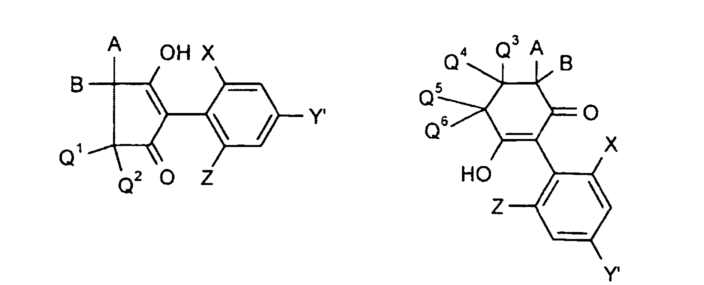 in which
A, B, D, Q¹, Q², Q³, Q⁴, Q⁵, Q⁶, X and Z are each as defined above and
Y' represents chlorine, bromine or iodine
with boronic acids of the formula (XII) 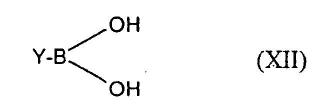 in which
Y is as defined above,
in the presence of a solvent, a base and a catalyst and subsequently reacting the resulting compounds of the formulae (I-1-a) to (I-8-a) in each case
(Jα) with acyl halides of the formula (XIII) 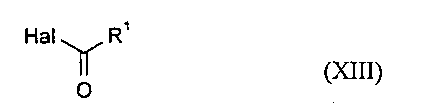 in which
R¹ is as defined in Claim 1 and
Hal represents halogen
or
(β) with carboxylic anhydrides of the formula (XIV)
R¹-CO-O-CO-R¹ (XIV)
in which
R¹ is as defined above,
if appropriate in the presence of a diluent and if appropriate in the presence of an acid binder, or in each case
(K) with chloroformic esters or chloroformic thioesters of the formula (XV)
R²-M-CO-Cl (XV)
in which
R² and M are each as defined in Claim 1,
if appropriate in the presence of a diluent and if appropriate in the presence of an acid binder, or in each case
(L) with chloromonothioformic esters or chlorodithioformic esters of the formula (XVI) 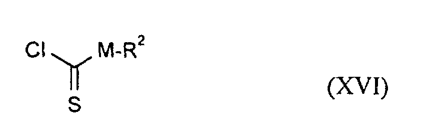 in which
M and R² are each as defined above,
if appropriate in the presence of a diluent and if appropriate in the presence of an acid binder, or in each case
(M) with sulphonyl chlorides of the formula (XVII)
R³-SO₂-Cl (XVII)
in which
R³ is as defined in Claim 1,
if appropriate in the presence of a diluent and if appropriate in the presence of an acid binder, or in each case
(N) with phosphorus compounds of the formula (XVIII) 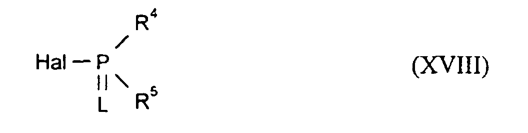 in which
L, R⁴ and R⁵ are each as defined in Claim 1 and
Hal represents halogen,
if appropriate in the presence of a diluent and if appropriate in the presence of an acid binder, or in each case
(O) with metal compounds or amines of the formulae (XIX) or (XX)
Me(OR¹⁰)ₜ (XIX) 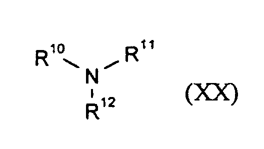
in which
Me represents a mono- or divalent metal,
t represents the number 1 or 2 and
R¹⁰, R¹¹, R¹² independently of one another each represent hydrogen or alkyl,
if appropriate in the presence of a diluent, or in each case
(Pα) with isocyanates or isothiocyanates of the formula (XXI)
R⁶-N=C=L (XXI)
in which
R⁶ and L are each as defined in Claim 1,
if appropriate in the presence of a diluent and if appropriate in the presence of a catalyst, or in each case
(β) with carbamoyl chlorides or thiocarbamoyl chlorides of the formula (XXII) 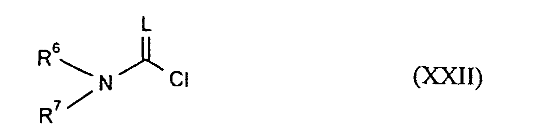 in which
L, R⁶ and R⁷ are each as defined in Claim 1,
if appropriate in the presence of a diluent and if appropriate in the presence of an acid binder.

3. Compounds of the formula (II) 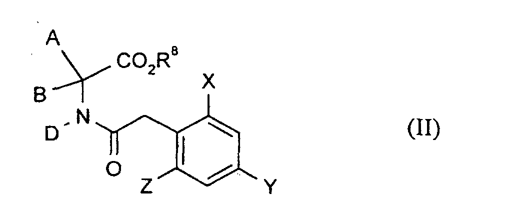 in which
A, B, D, X, Y and Z are each as defined in Claim 1 and
R⁸ represents alkyl.

4. Compounds of the formula (XXIV) 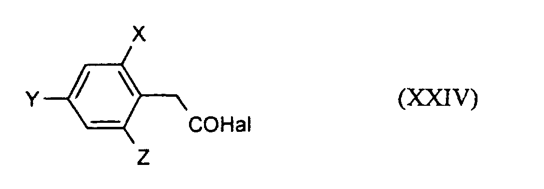 in which
X, Y and Z are each as defined in Claim 1 and
Hal represents chlorine or bromine.

5. Compounds of the formula (XXV) 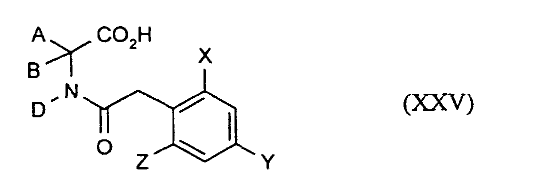 in which
A, B, D, X, Y and Z are each as defined in Claim 1.

6. Compounds of the formula (XXIX) 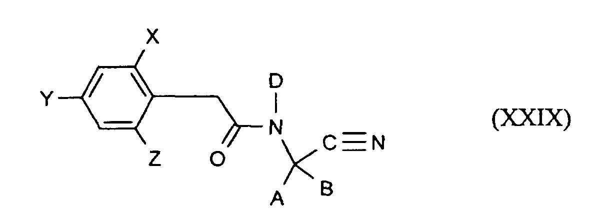 in which
A, B, D, X, Y and Z are each as defined in Claim 1.

7. Compounds of the formula (III) 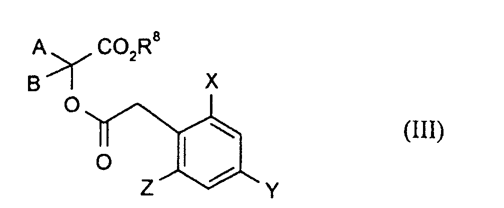 in which
A, B, X, Y and Z are each as defined above and
R⁸ represents alkyl.

8. Compounds of the formula (XXVII) 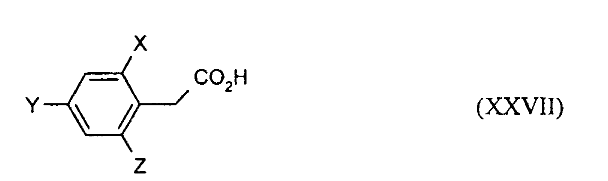 in which
X, Y and Z are each as defined in Claim 1.

9. Compounds of the formula (IV) 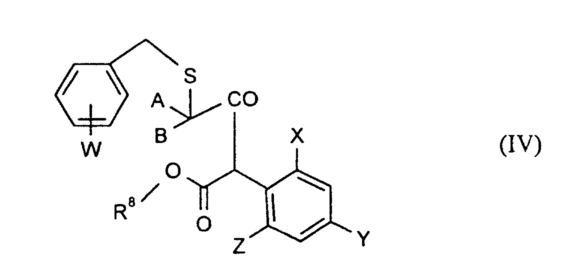 in which
A, B, W, X, Y and Z are each as defined in Claim 1 and
R⁸ represents alkyl.

10. Compounds of the formula (V) 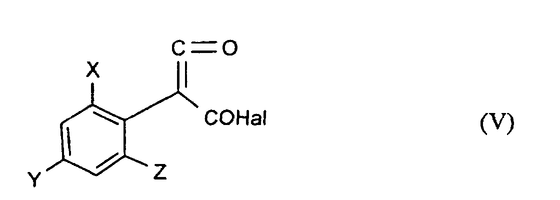 in which
X, Y and Z are each as defined in Claim 1 and
Hal represents chlorine or bromine.

11. Compounds of the formula (XXXVII) 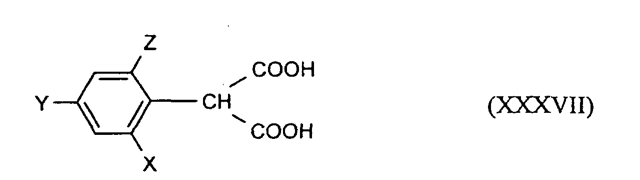 in which
X, Y and Z are each as defined in Claim 1.

12. Compounds of the formula (VI) 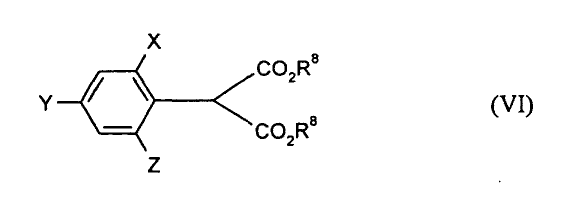 in which
X, Y and Z are each as defined in Claim 1 and
R⁸ represents alkyl.

13. Compounds of the formula (X) 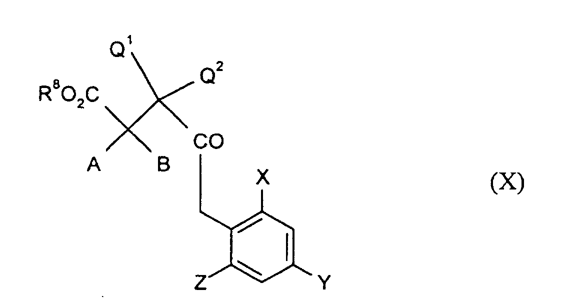 in which
A, B, Q¹, Q², X, Y and Z are each as defined in Claim 1 and
R⁸ represents alkyl.

14. Compounds of the formula (XXXVIII) 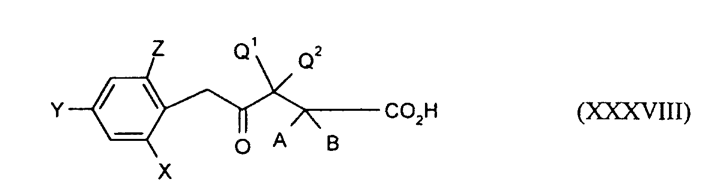 in which
X, Y, Z, A, B, Q¹ and Q² are each as defined in Claim 1.

15. Compounds of the formula (XXXIX) 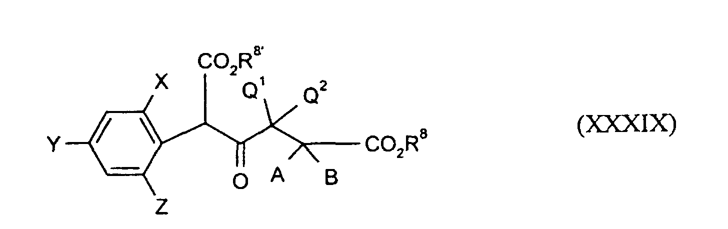 in which
A, B, D¹, D², X, Y and Z are each as defined in Claim 1 and
R⁸ and R^{8'} each represent alkyl.

16. Compounds of the formula (XI) 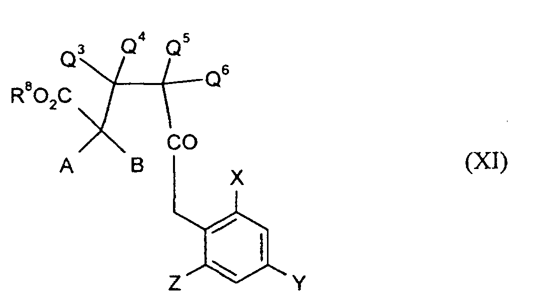 in which
A, B, Q³, Q⁴, Q⁵, Q⁶, X, Y and Z are each as defined in Claim 1 and
R⁸ represents alkyl.

17. Compounds of the formula (XLII) 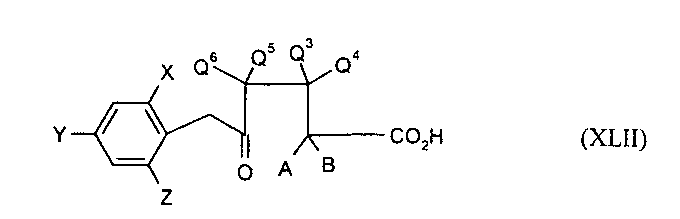 in which
A, B, Q³, Q⁴, Q⁵, Q⁶, X, Y and Z are each as defined in Claim 1.

18. Compounds of the formula (XLIII) 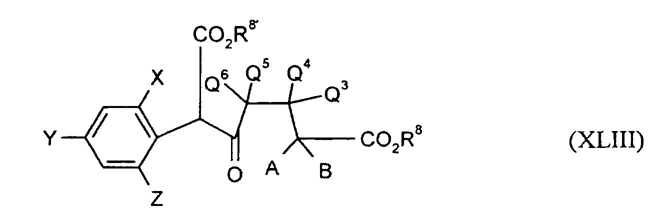 in which
A, B, Q³, Q⁴, Q⁵, Q⁶, X, Y and Z are each as defined in Claim 1 and
R⁸ and R^{8'} each represent alkyl.

19. Pesticides and/or herbicides, **characterized in that** they contain at least one compound of the formula (I) according to Claim 1.

20. The use of compounds of the formula (I) according to Claim 1 for controlling pests in crop protection, in the domestic sector, in the hygiene sector and in the protection of stored products.

21. Method for controlling pests in crop protection, in the domestic sector, in the hygiene sector and in the protection of stored products, **characterized in that** compounds of the formula (I) according to Claim 1 are allowed to act on the pests and/or their habitat.

22. Method for preparing pesticides and/or herbicides, **characterized in that** compounds of the formula (I) according to Claim 1 are mixed with extenders and/or surfactants.

## Revendications

1. Composés de formule (I) 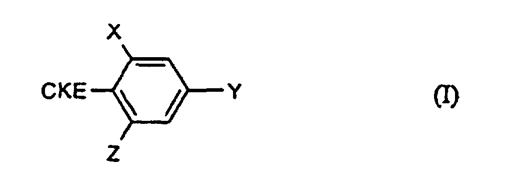 dans laquelle
X représente le fluor, le chlore, un reste méthyle, éthyle, propyle, iso-propyle,
Y représente le reste 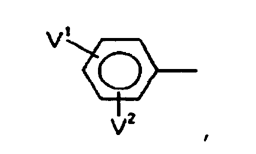
V¹ représente l'hydrogène, le fluor, le chlore, le brome, un reste méthyle, éthyle, n-propyle, isopropyle, n-butyle, iso-butyle, tertiobutyle, méthoxy, éthoxy, n-propoxy, iso-propoxy, trifluorométhyle, trifluorométhoxy, nitro, cyano ou phényle,
V² représente l'hydrogène, le fluor, le chlore, un reste méthyle, éthyle, n-propyle, iso-propyle, méthoxy, éthoxy, trifluorométhyle ou trifluorométhoxy,
Z représente l'hydrogène, le fluor, le chlore, un reste méthyle, éthyle ou n-propyle,
CKE désigne l'un des groupes 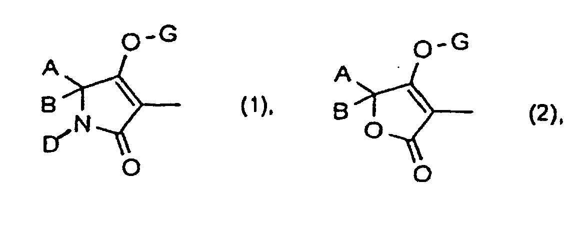 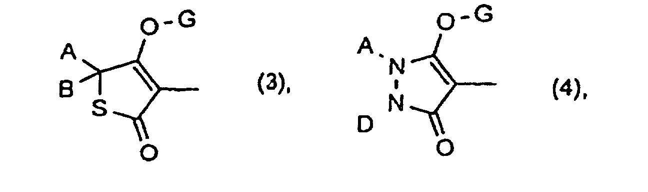 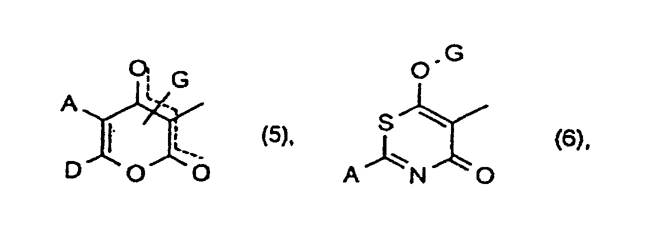 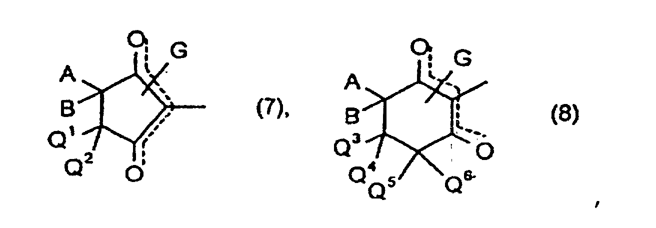
A représente l'hydrogène, un reste alkyle en C₁ à C₈ ou (alkoxy en C₁ à C₆)-(alkyle en C₁ à C₄), chacun éventuellement substitué par du fluor, un reste cycloalkyle en C₃ à C₆, éventuellement substitué par un radical fluor, méthyle, éthyle ou méthoxy, dans le noyau duquel un chaînon est éventuellement remplacé par de l'oxygène ou du soufre, ou bien (toutefois pas dans le cas des composés de formules (I-5), (I-7) et (I-8)) un reste phényle ou benzyle, chacun éventuellement substitué par un radical fluoro, chloro, bromo, méthyle, éthyle, n-propyle, isopropyle, méthoxy, éthoxy, trifluorométhyle, trifluorométhoxy, cyano ou nitro,
B est un reste alkyle en C₁ à C₄, ou bien
A, B et l'atome de carbone auquel ils sont liés forment un reste cycloalkyle saturé en C₅ ou C₆ dans le noyau duquel un chaînon est éventuellement remplacé par de l'oxygène ou du soufre et qui est éventuellement substitué une fois par un radical méthyle, éthyle, n-propyle, iso-propyle, butyle, iso-butyle, sec.-butyle, tertiobutyle, trifluorométhyle, méthoxy, éthoxy, n-propoxy, iso-propoxy, n-butoxy, iso-butoxy, sec.-butoxy, tertiobutoxy, fluoro, ou chloro, ou bien
A, B et l'atome de carbone auquel ils sont liés forment un reste cycloalkyle en C₅ ou C₆ dans lequel deux substituants forment, conjointement avec les atomes de carbone auxquels ils sont liés, un reste alcanediyle en C₂ à C₄ ou alcènediyle en C₂ à C₄, où dans chaque cas un groupe méthylène est éventuellement remplacé par de l'oxygène ou du soufre, ou un reste butadiènediyle,
D représente l'hydrogène, un reste alkyle en C₁ à C₈, alcényle en C₃ ou C₄, (alkoxy en C₁ à C₆) - (alkyle en C₂ à C₄), (alkylthio en C₁ à C₄) - (alkyle en C₂ à C₄) ou cycloalkyle en C₃ à C₆, dans lequel le cas échéant un groupe méthylène est remplacé par de l'oxygène ou du soufre, chacun éventuellement substitué par du fluor ou du chlore, ou bien (toutefois pas dans le cas des composés de formules (I-1) et (I-4)) un reste phényle, furannyle, pyridyle, thiényle ou benzyle, chacun éventuellement substitué par un radical fluoro, chloro, méthyle, éthyle, n-propyle, isopropyle, méthoxy, éthoxy, trifluorométhyle ou trifluorométhoxy,
ou bien
A et D forment ensemble un reste alcanediyle en C₃ à C₅ éventuellement substitué, dans lequel, le cas échéant, un atome de carbone est remplacé par du soufre ou de l'oxygène et qui est éventuellement substitué par un radical hydroxy, alkyle en C₁ à C₆ ou alkoxy en C₁ à C₄, ou bien
A et D (dans le cas des composés de formule (I-1)) forment conjointement avec les atomes auxquels ils sont liés l'un des groupes AD suivants : 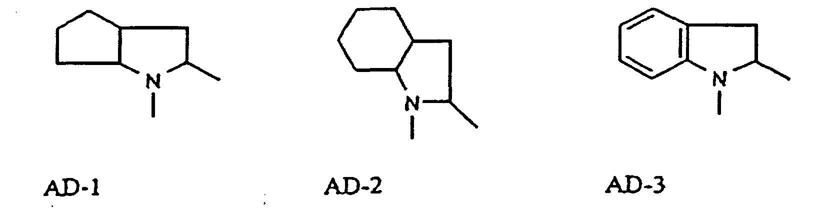 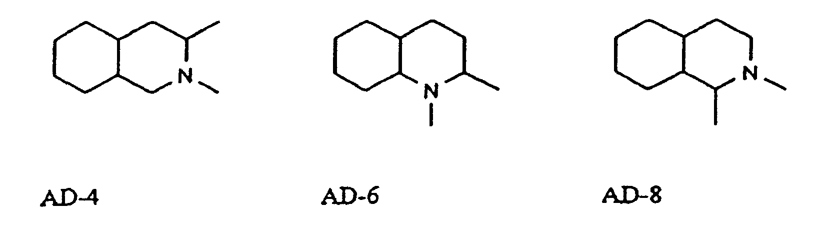 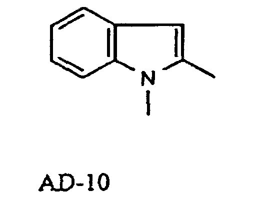
A et Q¹ forment ensemble un reste alcanediyle en C₃ ou C₄ éventuellement substitué une ou deux fois par un radical fluoro, hydroxy, méthyle ou méthoxy, ou un reste butènediyle, ou bien
Q¹ représente l'hydrogène,
Q² représente l'hydrogène,
Q⁴, Q⁵ et Q⁶ représentent indépendamment les uns des autres l'hydrogène, un reste méthyle ou éthyle,
Q³ représente l'hydrogène, un reste méthyle, éthyle ou cycloalkyle en C₃ à C₆, dans lequel, le cas échéant, un groupe méthylène est remplacé par de l'oxygène ou du soufre, ou bien
Q³ et Q⁴ forment, conjointement avec l'atome de carbone auquel ils sont liés, un reste cycloalkyle en C₅ ou C₆ saturé éventuellement substitué par un radical méthyle ou méthoxy, dans le noyau duquel, le cas échéant, un chaînon est remplacé par de l'oxygène ou du soufre,
G représente l'hydrogène (a) ou l'un des groupes 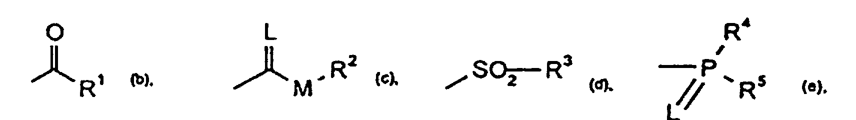 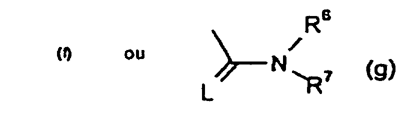 dans lesquels
E représente un ion métallique ou un ion ammonium,
L représente l'oxygène ou le soufre et
M représente l'oxygène ou le soufre,
R¹ est un reste alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly(alkoxy en C₁ à C₄)-(alkyle en C₁ à C₄), chacun éventuellement substitué par du fluor ou du chlore, ou un reste cycloalkyle en C₃ à C₆ éventuellement substitué par un radical fluoro, chloro, méthyle, éthyle, n-propyle, isopropyle, n-butyle, iso-butyle, tertiobutyle, méthoxy, éthoxy, n-propoxy ou iso-propoxy, dans le noyau duquel, le cas échéant, un ou deux chaînons non contigus sont remplacés par de l'oxygène et/ou du soufre,
un reste phényle éventuellement substitué par un radical fluoro, chloro, bromo, cyano, nitro, méthyle, éthyle, n-propyle, iso-propyle, méthoxy, éthoxy, trifluorométhyle, trifluorométhoxy, méthylthio, éthylthio, méthylsulfonyle ou éthylsulfonyle,
un reste benzyle éventuellement substitué par un radical fluoro, chloro, bromo, méthyle, éthyle, n-propyle, iso-propyle, méthoxy, éthoxy, trifluorométhyle ou trifluorométhoxy,
un reste furannyle, thiényle, pyridyle, pyrimidyle, thiazolyle ou pyrazolyle, chacun éventuellement substitué par un radical fluoro, chloro, bromo, méthyle ou éthyle,
un reste phénoxy-(alkyle en C₁ ou C₂) éventuellement substitué par un radical fluoro, chloro, méthyle ou éthyle, ou bien
un reste pyridyloxy-(alkyle en C₁ ou C₂), pyrimidyloxy-(alkyle en C₁ ou C₂) ou thiazolyloxy-(alkyle en C₁ ou C₂), chacun éventuellement substitué par un radical fluoro, chloro, amino, méthyle ou éthyle,
R² représente un reste alkyle en C₁ à C₁₄, alcényle en C₂ à C₁₄, (alkoxy en C₁ à C₄)-(alkyle en C₂ à C₆) ou poly(alkoxy en C₁ à C₄)-(alkyle en C₂ à C₆), chacun éventuellement substitué par du fluor,
un reste cycloalkyle en C₃ à C₆ éventuellement substitué par un radical fluoro, chloro, méthyle, éthyle, n-propyle, iso-propyle ou méthoxy,
ou bien un reste phényle ou benzyle, chacun éventuellement substitué par un radical fluoro, chloro, cyano, nitro, méthyle, éthyle, n-propyle, iso-propyle, méthoxy, éthoxy, trifluorométhyle ou trifluoro-méthoxy,
R³ représente un reste méthyle éventuellement substitué par du fluor, un reste éthyle, n-propyle, isopropyle ou un reste phényle ou benzyle, chacun éventuellement substitué par un radical fluoro, chloro, bromo, méthyle, tertiobutyle, méthoxy, trifluorométhyle, trifluorométhoxy, cyano ou nitro,
R⁴ et R⁵ représentent, indépendamment l'un de l'autre, un reste alkyle en C₁ à C₄, alkoxy en C₁ à C₄, alkylamino en C₁ à C₄, di (alkyle en C₁ à C₄) amino, alkylthio en C₁ à C₄ ou un reste phényle, phénoxy ou phénylthio, chacun éventuellement substitué par un radical fluoro, chloro, bromo, nitro, cyano, alkoxy en C₁ ou C₂, fluoralkoxy en C₁ ou C₂, alkylthio en C₁ ou C₂, fluoralkylthio en C₁ ou C₂ ou alkyle en C₁ à C₃, et
R⁶ et R⁷ représentent, indépendamment l'un de l'autre, l'hydrogène, un reste alkyle en C₁ à C₄, cycloalkyle en C₃ à C₆, alkoxy en C₁ à C₄, alcényle en C₃ ou C₄, (alkoxy en C₁ à C₄) - (alkyle en C₁ à C₄), un reste phényle éventuellement substitué par un radical fluoro, chloro, bromo, trifluorométhyle, méthyle ou méthoxy, un reste benzyle éventuellement substitué par un radical fluoro, chloro, bromo, méthyle, trifluorométhyle ou méthoxy, ou forment ensemble un reste alkylène en C₅ ou C₆ dans lequel, le cas échéant, un groupe méthylène est remplacé par de l'oxygène ou du soufre.

2. Procédé de production de composés de formule (I) suivant la revendication 1, **caractérisé en ce que**
(A) on obtient des composés de formule (I-1-a) 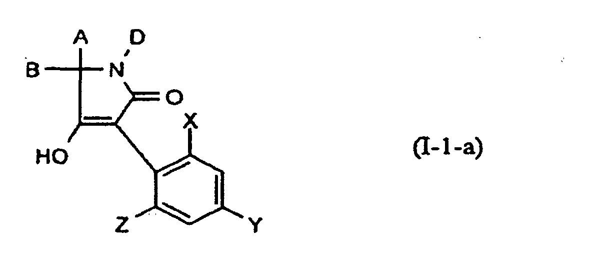 dans laquelle
A, B, D, X, Y et Z ont les définitions indiquées dans la revendication 1, lorsque
on effectue la condensation intramoléculaire d'esters de N-acylaminoacides de formule (II) 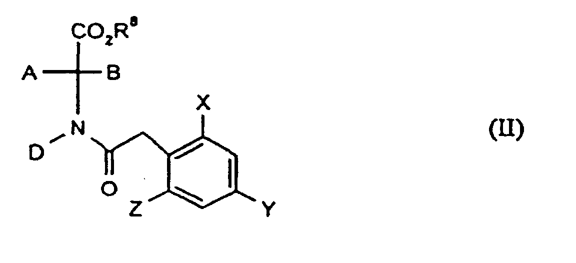 dans laquelle
A, B, D, X, Y et Z ont les définitions indiquées ci-dessus, et
R⁸ est un reste alkyle,
en présence d'un diluant et en présence d'une base,
(B) on obtient des composés de formule (I-2-a) 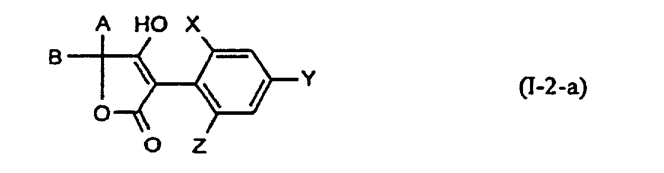 dans laquelle
A, B, X, Y et Z ont les définitions indiquées ci-dessus, lorsque
on effectue la condensation intramoléculaire d'esters d'acides carboxyliques de formule (III) 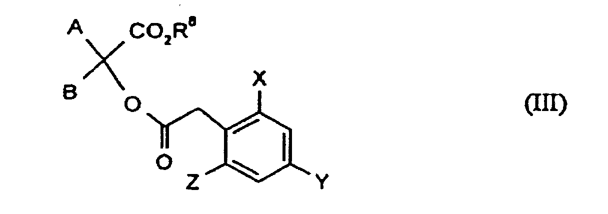 dans laquelle
A, B, X, Y et Z ont les définitions indiquées ci-dessus,
en présence d'un diluant et en présence d'une base,
(C) on obtient des composés de formule (I-3-a) 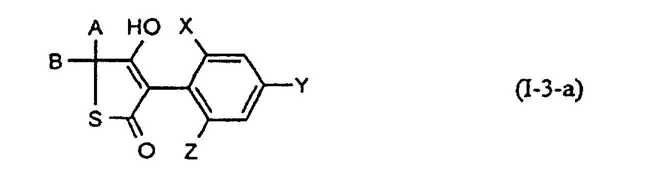 dans laquelle
A, B, X, Y et Z ont les définitions indiquées ci-dessus, lorsque
on effectue la cyclisation intramoléculaire d'esters d'acides β-céto-carboxyliques de formule (IV) 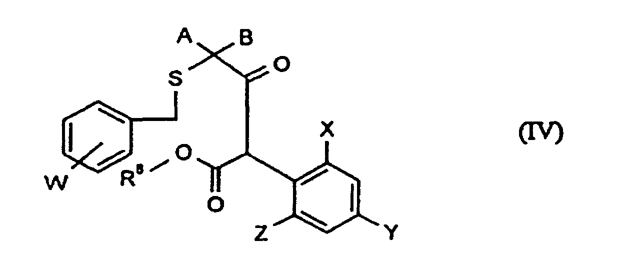 dans laquelle
A, B, X, Y, Z et R⁸ ont les définitions indiquées ci-dessus, et
W représente l'hydrogène, un halogène, un reste alkyle ou alkoxy,
éventuellement en présence d'un diluant et en présence d'un acide,
(D) on obtient des composés de formule (I-4-a) 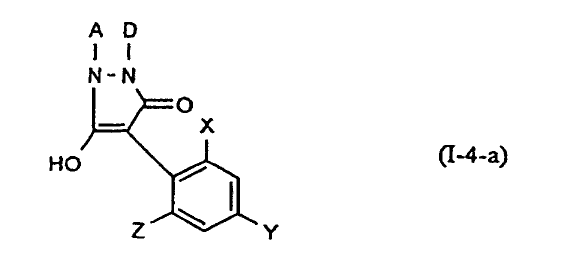 dans laquelle
A, D, X, Y et Z ont les définitions indiquées ci-dessus, lorsque l'on fait réagir
(α) des halogénocarbonylcétènes de formule (V) 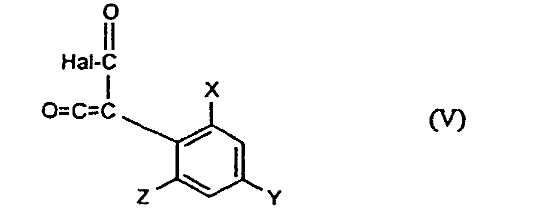 dans laquelle
X, Y et Z ont les définitions indiquées ci-dessus, et
Hal représente un halogène,
ou bien
(β) des dérivés d'acide malonique de formule (VI) 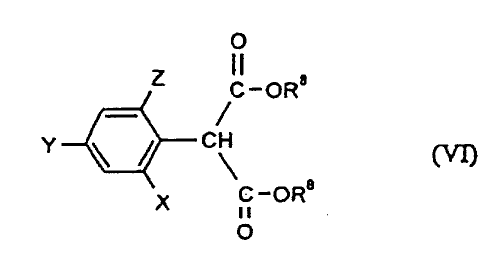 dans laquelle
R⁸, X, Y et Z ont les définitions indiquées ci-dessus,
avec des hydrazines de formule (VII)
A-NH-NH-D (VII)
dans laquelle
A et D ont les définitions indiquées ci-dessus,
éventuellement en présence d'un diluant et, le cas échéant, en présence d'une base,
(E) on obtient des composés de formule (I-5-a) 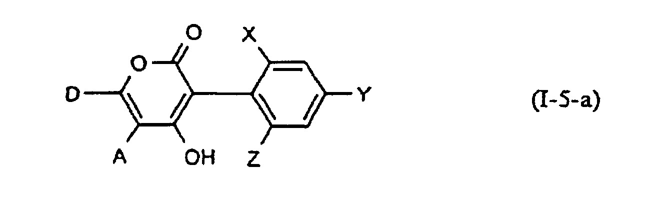 dans laquelle
A, D, X, Y et Z ont les définitions indiquées ci-dessus, lorsque
on fait réagir des composés carbonyliques de formule (VIII) 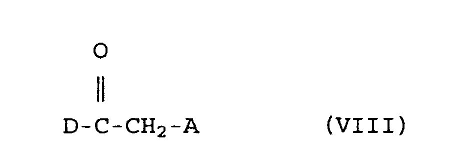 dans laquelle
A et D ont les définitions indiquées ci-dessus,
ou leurs éthers de silylénol de formule (VIIIa) 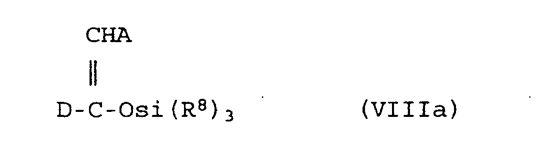 dans laquelle
A, D et R⁸ ont la définition indiquée ci-dessus,
avec des halogénures d'acides céténoïques de formule (V) 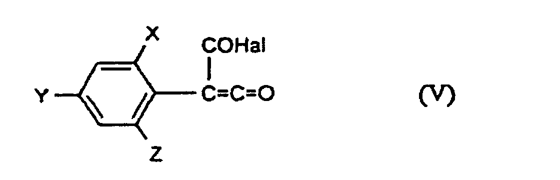 dans laquelle
X, Y et Z ont les définitions indiquées ci-dessus et
Hal représente un halogène,
le cas échéant en présence d'un diluant et en présence éventuelle d'un accepteur d'acide,
(F) on obtient des composés de formule (I-6-a) 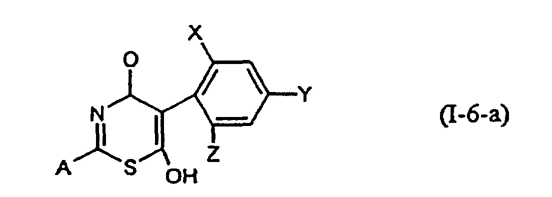 dans laquelle
A, X, Y et Z ont la définition indiquée ci-dessus,
lorsque l'on fait réagir des thioamides de formule (IX) 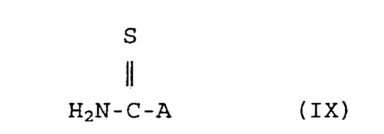 dans laquelle
A a la définition indiquée ci-dessus,
avec des halogénures d'acides céténoïques de formule (V) 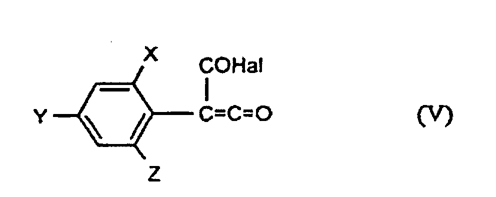 dans laquelle
Hal, X, Y et Z ont les définitions indiquées ci-dessus,
le cas échéant en présence d'un diluant et en présence éventuelle d'un accepteur d'acide,
(G) on obtient des composés de formule (I-7-a) 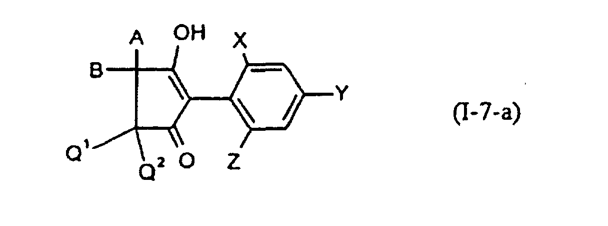 dans laquelle
A, B, Q¹, Q², W, X, Y et Z ont la définition indiquée dans la revendication 1, lorsque
on effectue la cyclisation intramoléculaire d'esters d'acides céténoïques de formule (X) 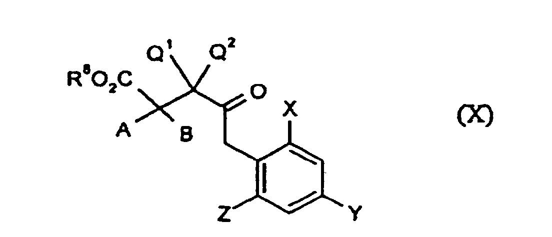 dans laquelle
A, B, Q¹, Q², X, Y et Z ont la définition indiquée ci-dessus, et
R⁸ est un reste alkyle,
éventuellement en présence d'un diluant et, le cas échéant, en présence d'une base,
(H) on obtient des composés de formule (I-8-a) 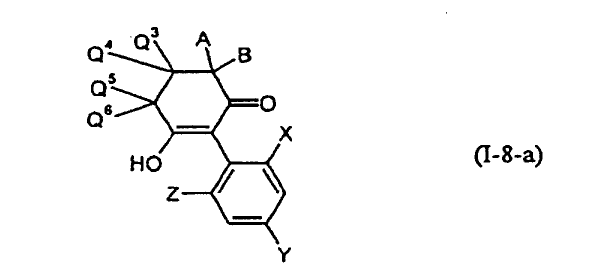 dans laquelle
A, B, Q³, Q⁴, Q⁵, Q⁶, X, Y et Z ont la définition indiquée dans la revendication 1, lorsque
on effectue la condensation intramoléculaire d'esters d'acides 6-aryl-5-céto-hexanoïques de formule (XI) 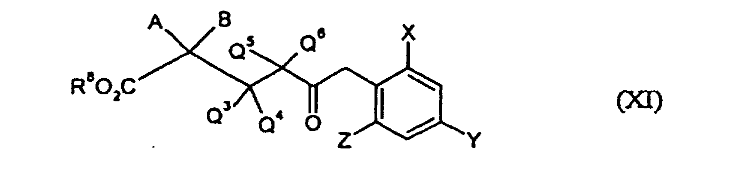 dans laquelle
A, B, Q³, Q⁴, Q⁵, Q⁶, X, Y et Z ont la définition indiquée ci-dessus,
et
R⁸ est un reste alkyle,
en présence d'un diluant et, le cas échéant, en présence d'une base,
(I) on obtient des composés des formules (I-8-a) indiquées ci-dessus, dans lesquelles A, B, D, Q¹, Q², Q³, Q⁴, Q⁵, Q⁶, X, Y et Z ont la définition indiquée ci-dessus, lorsque l'on fait réagir des composés de formules (I-1'-a) à (I-8'-a), 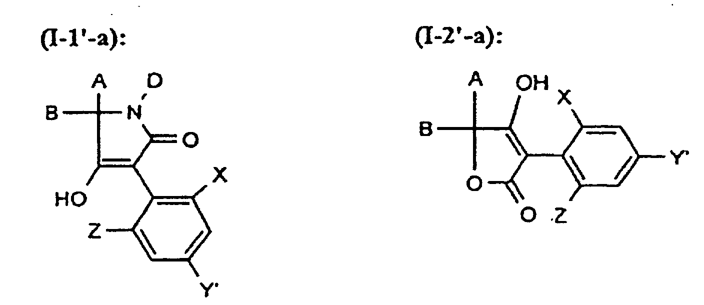 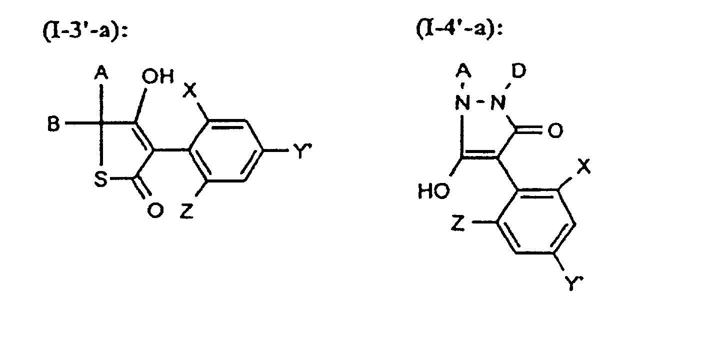 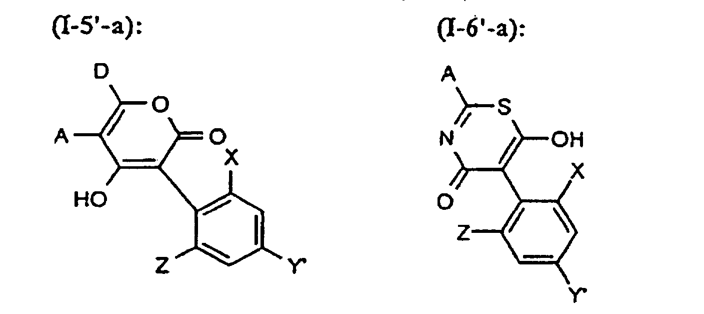 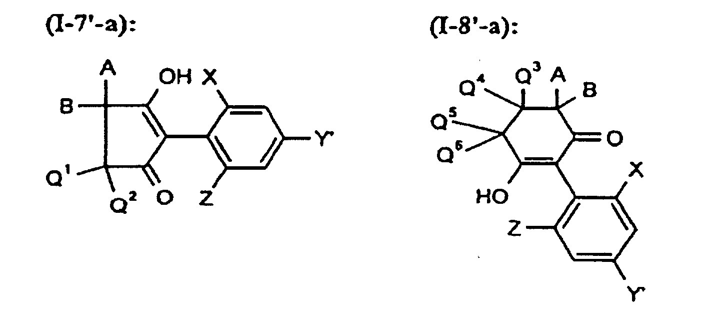 dans lesquelles
A, B, D, Q¹, Q², Q³, Q⁴, Q⁵, Q⁶, X et Z ont la définition indiquée ci-dessus, et
Y' représente le chlore, le brome ou l'iode,
avec des acides boroniques de formule (XII) 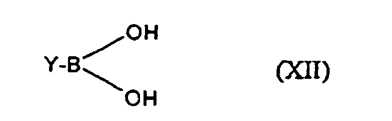 dans laquelle
Y a la définition indiquée ci-dessus,
en présence d'un solvant, d'une base et d'un catalyseur, puis on fait réagir éventuellement les composés ainsi obtenus de formules (I-1-a) à (I-8-a) dans chaque cas
(Jα) avec des halogénures d'acides de formule (XIII) 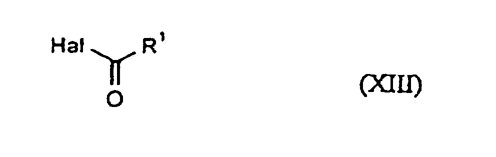 dans laquelle
R¹ a la définition indiquée dans la revendication 1, et
Hal représente un halogène,
ou bien
(β) avec des anhydrides d'acides carboxyliques de formule (XIV)
R¹-CO-O-CO-R¹ (XIV)
dans laquelle
R¹ a la définition indiquée ci-dessus,
éventuellement en présence d'un diluant et, le cas échéant, en présence d'un accepteur d'acide, ou bien dans chaque cas
(K) avec des esters d'acide chloroformique et des thioesters d'acide chloroformique de formule (XV)
R²-M-CO-Cl (XV)
dans laquelle
R² et M ont les définitions indiquées dans la revendication 1,
éventuellement en présence d'un diluant et, le cas échéant, en présence d'un accepteur d'acide, ou dans chaque cas
(L) avec des esters d'acide chloromonothioformique ou des esters d'acide chlorodithioformique de formule (XVI) 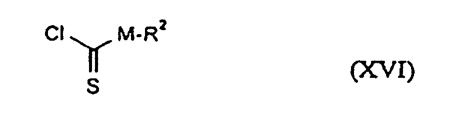 dans laquelle
M et R² ont les définitions indiquées ci-dessus,
le cas échéant en présence d'un diluant et en présence éventuellement d'un accepteur d'acide, ou bien dans chaque cas
(M) avec des chlorures d'acides sulfoniques de formule (XVII)
R³-SO₂-Cl (XVII)
dans laquelle
R³ a la définition indiquée dans la revendication 1,
éventuellement en présence d'un diluant et, le cas échéant, en présence d'un accepteur d'acide, ou bien dans chaque cas
(N) avec des composés phosphorés de formule (XVIII) 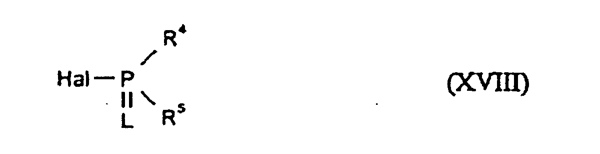 dans laquelle
L, R⁴ et R⁵ ont les définitions indiquées dans la revendication 1 et
Hal représente un halogène,
éventuellement en présence d'un diluant et, le cas échéant, en présence d'un accepteur d'acide, ou bien dans chaque cas
(O) avec des composés métalliques ou des amines de formule (XIX) ou (XX)
Me(OR¹⁰)ₜ (XIX) 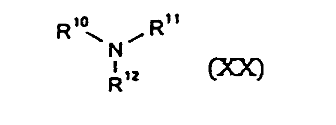
dans lesquelles
Me représente un métal monovalent ou divalent,
t représente le nombre 1 ou 2 et
R¹⁰, R¹¹, R¹² représentent, indépendamment les uns des autres, l'hydrogène ou un reste alkyle,
éventuellement en présence d'un diluant, ou bien dans chaque cas
(Pα) avec des isocyanates ou des isothiocyanates de formule (XXI)
R⁶-N=C=L (XXI)
dans laquelle
R⁶ et L ont les définitions indiquées dans la revendication 1,
éventuellement en présence d'un diluant et, le cas échéant, en présence d'un catalyseur, ou bien dans chaque cas
(β) avec des chlorures d'acides carbamiques ou des chlorures d'acides thiocarbamiques de formule (XXII) 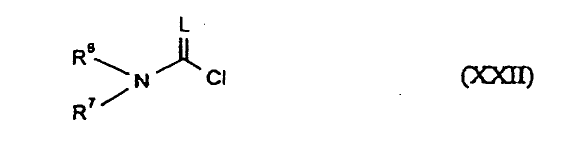 dans laquelle
L, R⁶ et R⁷ ont les définitions indiquées dans la revendication 1,
le cas échéant en présence d'un diluant et en présence éventuellement d'un accepteur d'acide.

3. Composés de formule (II) 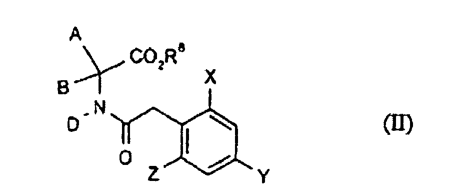 dans laquelle
A, B, D, X, Y et Z ont les définitions indiquées dans la revendication 1, et
R⁸ est un reste alkyle.

4. Composés de formule (XXIV) 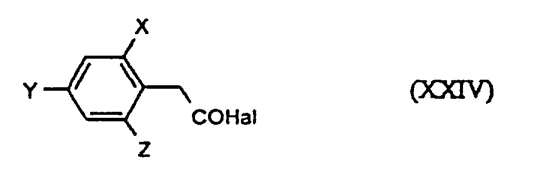 dans laquelle
X, Y et Z ont les définitions indiquées dans la revendication 1, et
Hal représente le chlore ou le brome.

5. Composés de formule (XXV) 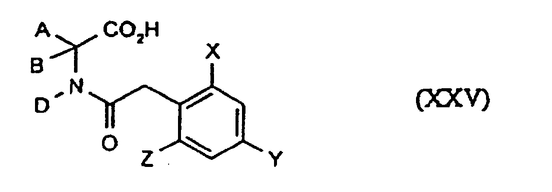 dans laquelle
A, B, D, X, Y et Z ont les définitions indiquées dans la revendication 1.

6. Composés de formule (XXIX) 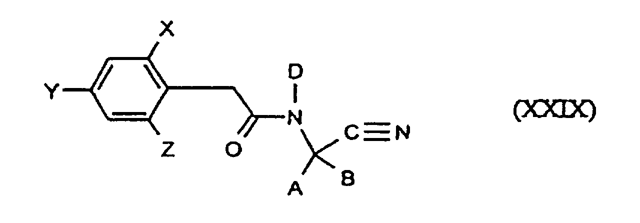 dans laquelle
A, B, D, X, Y et Z ont les définitions indiquées dans la revendication 1.

7. Composés de formule (III) 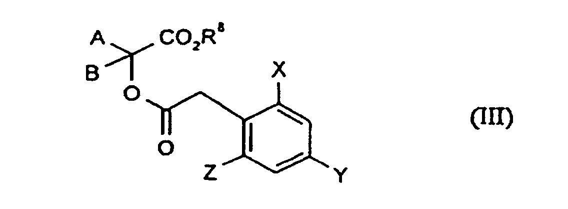 dans laquelle
A, B, X, Y et Z ont les définitions indiquées ci-dessus, et
R⁸ est un reste alkyle.

8. Composés de formule (XXVII) 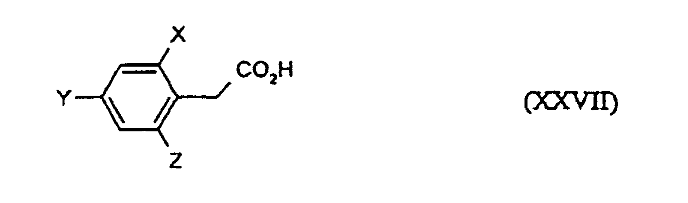 dans laquelle
X, Y et Z ont les définitions indiquées dans la revendication 1.

9. Composés de formule (IV) 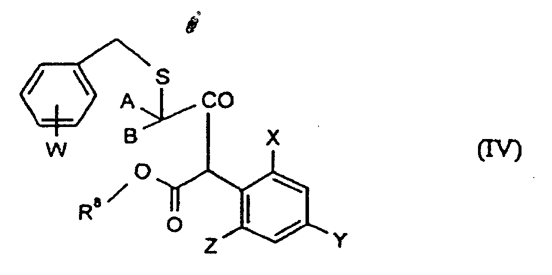 dans laquelle
A, B, W, X, Y et Z ont les définitions indiquées dans la revendication 1, et
R⁸ est un reste alkyle.

10. Composés de formule (V) 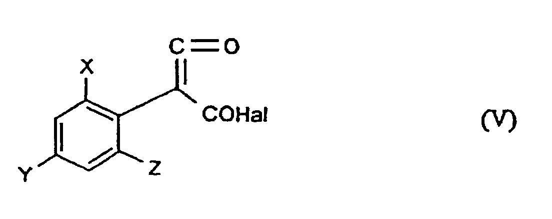 dans laquelle
X, Y et Z ont les définitions indiquées dans la revendication 1, et
Hal représente le chlore ou le brome.

11. Composés de formule (XXXVII) 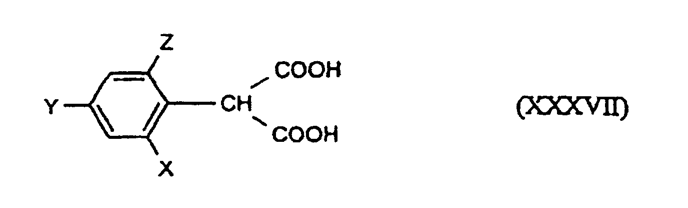 dans laquelle
X, Y et Z ont les définitions indiquées dans la revendication 1.

12. Composés de formule (VI) 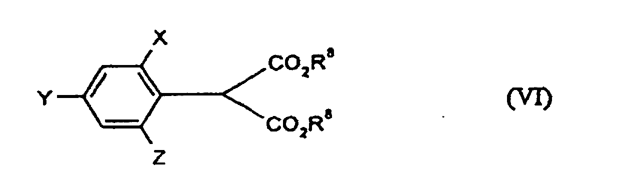 dans laquelle
X, Y et Z ont la définition indiquée dans la revendication 1, et
R⁸ est un reste alkyle.

13. Composés de formule (X) 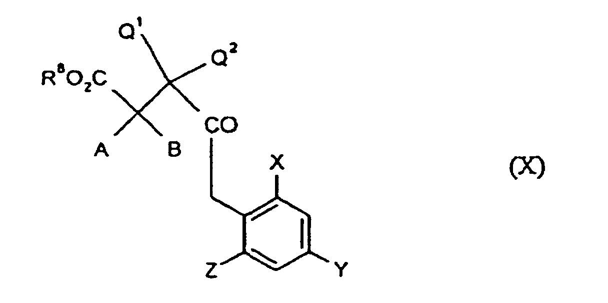 dans laquelle
A, B, Q¹, Q², X, Y et Z ont la définition indiquée dans la revendication 1, et
R⁸ est un reste alkyle.

14. Composés de formule (XXXVIII) 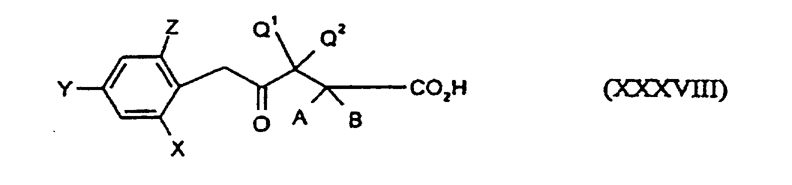 dans laquelle
X, Y, Z, A, B, Q¹ et Q² ont la définition indiquée dans la revendication 1.

15. Composés de formule (XXXIX) 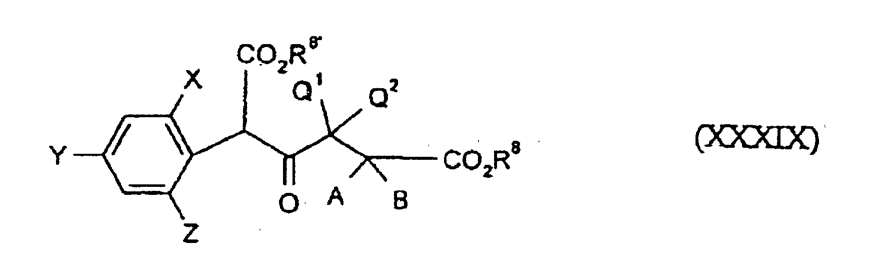 dans laquelle
A, B, D¹, D², X, Y et Z ont la définition indiquée dans la revendication 1, et
R⁸ et R^{8'} représentent un reste alkyle.

16. Composés de formule (XI) 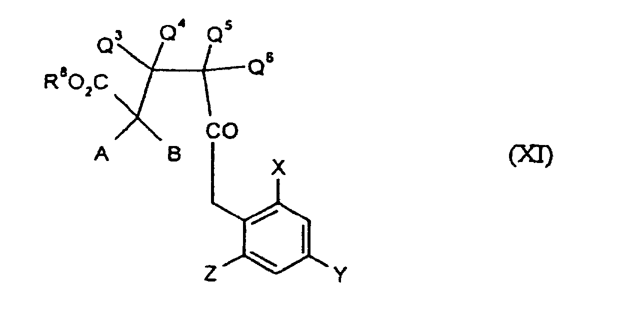 dans laquelle
A, B, Q³, Q⁴, Q⁵, Q⁶, X, Y et Z ont la définition indiquée dans la revendication 1, et
R⁸ est un reste alkyle.

17. Composés de formule (XLII) 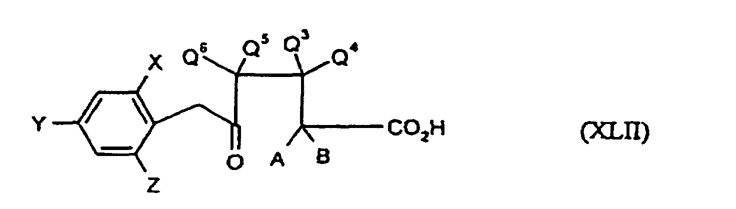 dans laquelle
A, B, Q³, Q⁴, Q⁵, Q⁶, X, Y et Z ont la définition indiquée dans la revendication 1.

18. Composés de formule (XLIII) 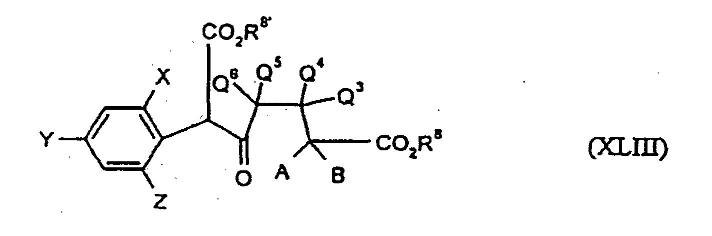 dans laquelle
A, B, Q³, Q⁴, Q⁵, Q⁶, X, Y et Z ont la définition indiquée dans la revendication 1 et
R⁸ et R^{8'} représentent un reste alkyle.

19. Compositions pesticides et/ou compositions herbicides, **caractérisées par** une teneur en au moins un composé de formule (I) suivant la revendication 1.

20. Utilisation de composés de formule (I) suivant la revendication 1 pour combattre des parasites dans la protection des plantes, dans le domaine de l'entretien domestique, dans le secteur de l'hygiène et dans la protection des denrées.

21. Procédé pour combattre des parasites dans la protection des plantes, dans le domaine de l'entretien domestique, dans le secteur de l'hygiène et dans la protection des denrées, **caractérisé en ce que** l'on fait agir des composés de formule (I) suivant la revendication 1 sur les parasites et/ou sur leur milieu.

22. Procédé de préparation de compositions pesticides et/ou de compositions herbicides, **caractérisé en ce que** l'on mélange des composés de formule (I) suivant la revendication 1 avec des diluants et/ou des agents tensioactifs.
